# EUROPEAN PATENT APPLICATION

(11) **EP 4 741 507 A1**
(43) Date of publication of application: **13.05.2026**
(21) Application number: 24838760.7
(22) Date of filing: 08.07.2024
(51) Int. Cl.: C12N 15/74, C12N 1/21, C12N 15/63, C12N 1/36, A61P 35/00

(54) **MODIFIED BACTERIUM HAVING ANTI-TUMOR ACTIVITY AND BETTER SAFETY**

(30) Priority: 07.07.2023 CN 202310836546
(71) Applicant: Shenzhen Synthetica Pioneering Co., Ltd., Shenzhen, Guangdong 518107 (CN)
(72) Inventor: LIU, Chenli, Shenzhen, Guangdong 518107 (CN); WANG, Zuowei, Shenzhen, Guangdong 518107 (CN); DONG, Yuxuan, Shenzhen, Guangdong 518107 (CN); SHENG, Fangqian, Shenzhen, Guangdong 518107 (CN); LU, Weiqi, Shenzhen, Guangdong 518107 (CN); LI, Yang, Shenzhen, Guangdong 518107 (CN); ZANG, Zhongsheng, Shenzhen, Guangdong 518107 (CN); LI, Wanlin, Shenzhen, Guangdong 518107 (CN); MALEEHA, Saifi, Shenzhen, Guangdong 518107 (CN)
(74) Representative: Michalski Hüttermann & Partner Patentanwälte mbB
(86) International application number: PCT/CN2024/104164
(87) International publication number: WO 2025/011507

(57) **Abstract**

The present invention relates to a modified bacterium with anti-tumor activity, wherein the bacterium comprises a hypoxia regulated lipopolysaccharide (LPS) gene expression cassette comprising a gene involved in the biosynthesis or transport of LPS or a gene encoding murein-lipoprotein under the control of a first strictly hypoxia inducible promoter. The bacterium further comprises an essential gene expression cassette under the control of a strictly hypoxia inducible promoter, and the bacteria is deficient in at least one gene or functional expression product thereof that participates in or regulates an endogenous anti-oxidative stress response pathway. The present invention also relates to a pharmaceutical composition comprising the modified bacterium and anti-tumor use thereof.

## Description

### Technical Field

The present invention relates to a modified bacterium with anti-tumor activity, a pharmaceutical composition comprising the modified bacterium and the anti-tumor use thereof.

### Background

Cancer has become one of the most important diseases threatening human life and health, but existing treatments (radiotherapy, chemotherapy, surgery and targeted drugs) all have shortcomings and there is an urgent need to develop new treatment approaches. Traditional bacterial tumor therapies, represented by Coley's toxin, have a history of 150 years, but are known for their unstable efficacy and safety concerns. The development of genetic engineering technology has made great progress in reducing the toxicity of bacterial strains, and a series of clinical trials have demonstrated that the attenuated engineered bacteria for human tumor therapy are safe but with limited efficacy, i.e., are unable to combine safety and therapeutic efficacy, thus failing to satisfy the needs of clinical tumor therapy.

In addition, bacteria carrying intact lipopolysaccharide (LPS) can activate the immune response in normal tissues and organs after the administration to a patient. On the one hand, such an immune response will lead to the loss of anti-tumor bacteria during the delivery, and on the other hand, it will cause side effects to the body, for example, resulting in the loss of body weight in patients. In 2016, researchers obtained attenuated Salmonella strains by the knockout of the *msbB* gene in the Salmonella LPS pathway and the murein-lipoprotein synthesis gene *lppAB,* or the knockout of both the two genes. However, previous studies showed that the therapeutic effect of attenuated Salmonella strain VNP20009 (*msbB-, purI-*) with *msbB* knockout on tumor is not significant.

Therefore, the further optimization for tumor therapy with bacteria is imperative.

### Summary of the Invention

In the present invention, a strictly anaerobic bacterium is constructed by means of synthetic biological genetic circuits, which shows therapeutic efficacy against tumors after being administered to an animal body or a human body, and can be cleared by normal tissues and organs in a short period of time, thereby reducing the toxic and side effects on the animal body or the human body due to the long-term retention of the bacterium in vivo. The bacterium of the present invention is deficient in the wild-type genes related to the biosynthesis and transport of LPS, and the deficient genes are conditionally expressed in the bacterium, thereby achieving site-specific production of complete LPS in tumors, enabling the induction of an immune response in tumors, meanwhile, avoiding the immune response of normal tissues against LPS, and thus, the bacterium has more reliable anti-tumor efficacy and safety.

The present invention provides a modified bacterium, wherein the bacterium comprises a hypoxia regulated lipopolysaccharide (LPS) gene expression cassette, and the LPS gene expression cassette comprises a gene involved in the biosynthesis or transport of LPS or a gene encoding murein-lipoprotein under the control of a first strictly hypoxia inducible promoter, as compared with an unmodified starting strain.

In one embodiment, the bacterium comprise a hypoxia regulated essential gene expression cassette comprising an essential gene of the bacterium under the control of a second strictly hypoxia inducible promoter, and the bacterium is deficient in at least one gene required for survival in macrophages or a functional expression product thereof, as compared to an unmodified starting strain.

In one embodiment, the bacterium comprise a hypoxia regulated essential gene expression cassette comprising an essential gene of the bacterium under the control of a second strictly hypoxia inducible promoter, and the bacterium is deficient in at least one gene involved in or regulating the endogenous anti-oxidative stress response pathway or a functional expression product thereof, as compared to an unmodified starting strain.

In one embodiment, the bacterium further comprise a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

In one embodiment, the bacterium comprise a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium expresses a wild-type lipopolysaccharide (LPS) and is deficient in at least one gene required for survival in macrophages or a functional expression product thereof, as compared to an unmodified starting strain.

The present invention provides a modified *Salmonella typhimurium* bacterium, wherein the bacterium comprises a hypoxia regulated lipopolysaccharide (LPS) gene expression cassette comprising a gene involved in the biosynthesis or transport of LPS or a gene encoding a murein-lipoprotein under the control of a first strictly hypoxia inducible promoter, as compared with an unmodified starting strain.

In one embodiment, the bacterium further comprises a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof, as compared to an unmodified starting strain.

In one embodiment, the bacterium further comprises a hypoxia regulated essential gene expression cassette comprising an essential gene of the bacterium under the control of a second strictly hypoxia inducible promoter, and a pH regulated expression cassette comprising a gene encoding a bacteria-derived hemolysin protein under the control of a promoter that is active under acidic pH condition, wherein the bacterium expresses wild-type LPS, and the bacterium is deficient in at least one gene required for survival in macrophages or a functional expression product thereof.

In one embodiment, the present invention provides a pharmaceutical composition comprising the modified bacterium of the present invention. In one embodiment, the pharmaceutical composition is used for treating a malignant tumor.

In one embodiment, the present invention provides a method of treating a malignant tumor comprising administering the pharmaceutical composition of the present invention to a subject having a malignant tumor.

In one embodiment, the present invention provides use of the modified bacterium of the present invention in the preparation of a medicament for treating a malignant tumor.

### Brief Description of Drawings

FIG.1 shows a schematic diagram of the construction scheme of DB-ZW1 strain.
FIG.2 shows changes in the distribution of DB-ZW1 in tumors and various organs over time. Among them, FIG.2A shows the changes of the density of DB-ZW1 in tumors and various organs over time; FIG.2B shows the changes of the density of DB-ZW1 in various normal organs over time.
FIG.3 shows the tumor inhibiting effect of DB- ZW1 on bladder cancer, melanoma and orthotopic colon cancer. Among them, FIGs.3A-C illustrate the effects of DB-ZW1 on the tumor volume of subcutaneous bladder cancer, orthotopic melanoma, and subcutaneous orthotopic colon cancer, respectively; and FIG. 3D illustrates the effect of DB-ZW1 on the tumor number of orthotopic colon cancer.

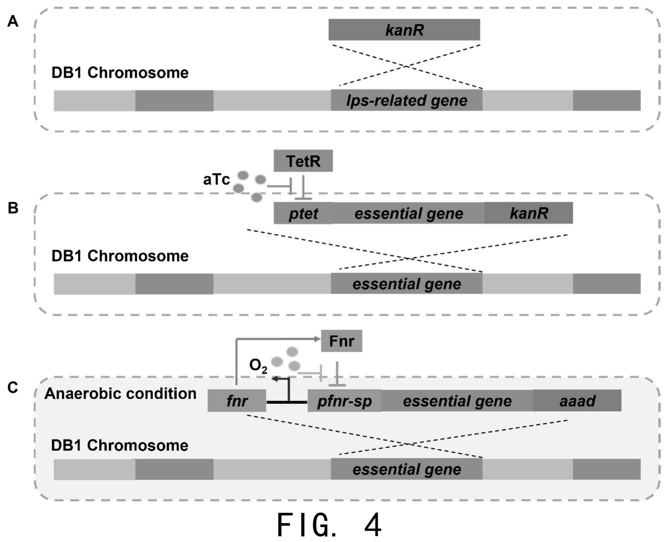

FIG.4 is the schematic diagram of the construction of DB-ZW1 variant strain. For non-essential genes, a homologous recombination method by replacing the target gene with a resistance gene was used the knockout of the endogenous gene (FIG. 4A); for essential genes, the endogenous gene was replaced with an essential gene regulated by an aTc inducer (FIG. 4B), or the endogenous gene was replaced with a gene, of which the expression is driven by a hypoxia activated promoter, so that the bacteria only expresses this series of genes in the hypoxia region (malignant solid tumor necrosis region) (FIG. 4C).
FIG.5 shows the PCR detection of the construction of strains expressing each LPS-associated gene or murein-lipoprotein gene under hypoxia induction, where the numbers of the lanes correspond to the numbers of primers corresponding to the genes in Table 5.
FIG.6 shows the therapeutic effect of strain ZW1(Δmsbb)-Fnr-SP-msbb-KnaR.
FIG.7 shows the therapeutic effect of strain ZW1(ΔlppAB)-Fnr-SP-lppAB-KnaR.

### Detailed Description of the Invention

### Definitions

In the present invention, the scientific and technical terms used herein have the meaning as commonly understood by a person skilled in the art unless otherwise specified. Also, the protein and nucleic acid chemistry, molecular biology, cell and tissue culture, microbiology, immunology related terms, and laboratory procedures used herein are terms and routine steps that are widely used in the corresponding field. At the same time, for a better understanding of the present invention, the definitions and explanations of the related terms are provided below.

As used herein, "lipopolysaccharide (LPS)" refers to a substance composed of a lipid and a polysaccharide, which is a component of the outer wall of the cell wall of Gram-negative bacteria, and is usually composed of O-antigen, polysaccharide core region and lipid A. The gene involved in the biosynthesis of LPS includes a gene involved in the synthesis of O-antigen, a gene involved in the synthesis of core region, and a gene involved in the synthesis of lipid A. The transport of lipopolysaccharides involves the assembly of lipid A with the core (msbA gene), the extension synthesis of O unit (wzy gene), the inner membrane inversion of O unit (wzxE gene), and the transport of LPS from inner membrane to outer membrane (lptA, lptB, lptC/yrbK, lptD, and lptG). Examples of the gene involved in the biosynthesis or transport of LPS and their DNA sequences are shown in Table 1. Those skilled in the art will understand that the genes also encompass the reverse complement of the listed sequences.

**Table 1**

| Classification | Name | Exemplary sequence in *Salmonella typhimurium* (SEQ ID NO:) |
|---|---|---|
| Synthesis of O antigen | rfbN* | 26 |
| | rfbU | 27 |
| | rfbV* | 28 |
| | wzzB | 29 |
| | gtrBb | 30 |
| | gtrBa | 31 |
| | rfbP/wbaP | 32 |
| | rfe | 33 |
| Synthesis of core region | rfaB | 34 |
| | rfaC/waaC* | 35 |
| | rfaF* | 36 |
| | rfaG* | 37 |
| | yibD | 38 |
| | rfaI | 39 |
| | rfaJ | 40 |
| | rfaK | 41 |
| | rfaL# | 42 |
| | rfaP | 43 |
| | rfaQ | 44 |
| | rfaY* | 45 |
| | rfaZ | 46 |
| | yijP | 47 |
| | gmhA | 48 |
| | yaeD | 49 |
| | rfaE | 50 |
| | rfaD | 51 |
| Synthesis of lipid A | lpxA | 52 |
| | lpxB* | 53 |
| | lpxC | 54 |
| | lpxD | 55 |
| | lpxH | 56 |
| | lpxK* | 57 |
| | htrB* | 58 |
| | msbB/lpxM | 59 |
| | lpxO | 60 |
| | lpxP | 61 |
| | yeiU* | 62 |
| | kdtA* | 63 |
| | arnT | 64 |
| | pagP* | 65 |
| | yjdB | 66 |
| | yhjw* | 67 |
| | kdsA* | 68 |
| | kdsB | 69 |
| | yrbI | 70 |
| | yrbH | 71 |
| Transport of LPS related | msbA | 72 |
| | Wzy | 73 |
| | wzxE* | 74 |
| | lptA | 75 |
| | lptB | 76 |
| | lptC/yrbK* | 77 |
| | lptD* | 78 |
| | lptG | 79 |

| | | |
|---|---|---|
| *Essential gene #Assmeble O antigen onto glucose | | |

"Hypoxia regulated essential gene expression cassette" herein means a stretch of DNA capable of promoting the expression of an essential gene under hypoxic conditions, which contains an essential gene under the control of a hypoxia inducible promoter and, if necessary, may further contain other regulatory elements required for the expression of the essential gene.

As used herein, "essential gene" refers to a gene that is decisive for the growth and / or survival of a bacterium, in the absence of which or its functional expression product the bacterium fails to survive, divide and / or grow normally. Typical examples of deletion of an essential gene or a functional expression product thereof are nutritionally deficient strains that fail to survive, divide and / or grow normally under in vitro culture conditions or in vivo environment in the absence of specific exogenous supplements. Essential genes usually appear as a single copy on the bacterial chromosome.

As used herein, an "inducible promoter" refers to a promoter that can greatly increase the transcription level of a gene under the stimulation of a specific physical or chemical signal.

As used herein, "strictly hypoxia inducible promoter" refers to a promoter that is capable of initiating the transcription of a specific gene under anaerobic conditions but is virtually impossible to activate and produce transcripts under aerobic conditions.

"Strictly hypoxia inducible promoters" useful in the present invention can be identified by the following experiments:
An expression strain containing an essential gene (e.g., dapA/dapE) controlled by a promoter to be determined is constructed, and subjected to Luria-Bertani (abbreviated as LB) plate streaking in an anaerobic incubator and anaerobic culture at 37°C for 24 h. Under anaerobic conditions, single clones are picked and resuspended in 20 µL of LB broth. The 20 µl resuspension of a single clone is equally added to the liquid in the following four tubes (A, B, C and D, 5 µl per tube):
A. 2 ml LB broth,
B. 2 ml LB broth,
C. 2 ml LB broth+DAP,
D. 2 ml LB broth+DAP.

The tubes A and C are incubated at 37°C in an anaerobic incubator for 24 h, and the tubes B and D are incubated at 37°C in an aerobic shaker for 24 h.

If the bacteria in the tubes A, C and D can grow, and the bacteria in the tube B cannot grow (the detected OD600 value is less than 0.05), the promoter is considered as a strictly hypoxia inducible promoter.

Exemplary strictly hypoxia inducible promoters that can be used in the present invention as identified by the experiments above are shown in Table 2.

**Table 2**

| Promoter name | Sequence | SEQ ID NO: |
|---|---|---|
| pepTp | GCAGGGGTAAAAGTGACCTGACGCAATATTTGTCT TTTCTTGCTTCTTAATAATGTTGTCACAAAAAGTG AGGGTGACTACATGG | 9 |
| fnrSp | CGCCGCAAAGTTTGAGCGAAGTCAATAAACTCTCT ACCCATTCAGGGCAATATCTCTCTTGCAGGTGAAT GCAACGTCAAGCGAT | 10 |
| ysgAp | TCAGAAGAAGCAAAAAGACACTTTACCGAAGGGTT TAACATTTTTTCGTGATACTCATCACCATGACGCA AATGCGTTGCATAAA | 11 |
| ssbp1 | AACCGAGGTCACAACATAGTAAAAGCGCTATTGGT AATGGTACAATCGCGCGTTTACACTTATTCAGAAC GATTTTTTTCAGGAG | 12 |
| Hip1 | GGATAAAAGTGACCTGACGCAATATTTGTCTTTTC TTGCTTAATAATGTTGTCA | 13 |
| BBa_I14018 | TGTAAGTTTATACATAGGCGAGTACTCTGTTATGG | 14 |
| BBa_R1074 | TTAAATTTCCTCTCGTCAGGCCGGAATAACTCCCT ATAATGCGCCACCACACTGATAGTGCTAGTGTAGA TCAC | 15 |
| Ptet-Fnr | AAAATTGATCTGAATCAATATTTTGACAAAAATTG | 16 |
| | ATCTGAATCAATATTTACTGAGC | |
| Ptet-arcA | GTTAATAAAATGTTATTGACAGTTAATAAAATGTT ATACTGAGC | 17 |
| sseA | TTAGCACGTTAATTATCTATCGTGTATATG | 18 |

As used herein, "gene involved in or regulating the endogenous anti-oxidative stress response pathway" refers to a gene involved in the resistance of bacteria (e.g., intracellular bacteria, such as Salmonella) against reactive oxygen species (ROS) in an in vivo or in vitro environment.

As used herein, "gene required for survival in macrophages" refers to a gene involved in maintaining or enhancing the viability of intracellular bacteria in macrophages. The function of the products of these genes is associated to resistance to the killing effect of macrophages on microorganisms, and thus deletion of these genes or their functional expression products can reduce the viability of bacteria (e.g., intracellular bacteria, such as Salmonella) in macrophages.

As used herein, the "functional expression product" includes RNA, proteins, and the like.

As used herein, "facultative anaerobic bacteria" refers to bacteria that can survive under both aerobic and anaerobic conditions.

As used herein, the "pH regulated expression cassette" refers to a group of gene expression elements in which the expression of the corresponding gene (s) is regulated by environmental pH.

As used herein, "a promoter that is active under acid pH condition" refers to a promoter that can be activated under acid pH condition and regulate the expression of the corresponding gene (s). In some embodiments, the promoter that is active under acid pH condition of the present invention may be activated or still have promoter activity at a pH less than 7.0, 6.9, 6.8, 6.7, 6.6, 6.5, 6.4, 6.3, 6.2, 6.1, 6.0, 5.9, 5.8, 5.7, 5.6, or 5.5. In some embodiments, the above promoter that is active under acid pH condition also has promoter activity at neutral pH (e.g., pH 7.0 to 7.4).

"Polypeptide", "peptide", and "protein" are used interchangeably in the present invention to refer to a polymer of amino acid residues. The terms apply to an amino acid polymer in which one or more amino acid residues is artificial chemical analogue of corresponding naturally occurring amino acid (s), as well as to a naturally occurring amino acid polymer. The terms "polypeptide", "peptide", "amino acid sequence", and "protein" may also include modified forms including, but not limited to, glycosylation, lipid ligation, sulfation, γ-carboxylation of glutamic acid residues, and ADP-ribosylation.

As used herein, "polynucleotide" refers to a macromolecule formed by linking multiple nucleotides via phosphodiester linkages, wherein the nucleotide comprises ribonucleotide and deoxyribonucleotide. The sequence of the polynucleotide of the present invention can be subjected to codon optimization for different host cells (such as *Escherichia coli*), thereby improving the expression of the polypeptide. Methods for codon optimization are known in the art.

"Sequence identity" between two polypeptides or two polynucleotide sequences refers to percentage of identical amino acids or nucleotides between the sequences. Methods for assessing the level of sequence identity between polypeptide or polynucleotide sequences are known in the art. Sequence identity can be assessed using various known sequence analysis software. For example, sequence identity can be assessed by an on-line alignment tool of EMBL-EBI (https://www.ebi.ac.uk/Tools/psa/). Sequence identity between two sequences can be assessed using default parameters through Needleman-Wunsch algorithm.

As used herein, the term "live bacterium" refers to a strain that is viable, and characterized by active nutrient metabolism, and is capable of performing its own biological functions. Live bacteria may include bacterial biomass produced in the metabolic process of the strain.

The term "Gram-negative bacteria" used herein refers to bacteria that do not retain the initial basic dye stain (e.g., crystal violet) that is part of the procedure known as the Gram stain. In an exemplary Gram stain, cells are first fixed to a slide by heat and stained with a basic dye (e.g., crystal violet), which is taken up by both Gram-negative and Gram-positive bacteria. The slides are then treated with a mordant (e.g., Gram's iodine), which binds to basic dye (e.g. crystal violet) and traps it in the cell. The cells are then washed with acetone or alcohol, and then counterstained with a second dye of different color (e.g., safranin). Gram-positive organisms retain the initial violet stain, while Gram-negative organisms are decolorized by the wash solvent organic and hence show the counterstain. Exemplary Gram-negative bacteria include, but are not limited to, Escherichia spp., Shigella spp., Salmonella spp., Campylobacter spp., Neisseria spp., Haemophilus spp., Aeromonas spp., Francisella spp., Yersinia spp., Klebsiella spp., Bordetella spp., Legionella spp., Corynebacteria spp., Citrobacter spp., Chlamydia spp., Brucella spp., Pseudomonas spp., Helicobacter spp. and Vibrio spp.

As used herein, the term "malignant solid tumor" refers to an abnormal mass of tissues, usually not comprising cysts or liquid areas. Solid tumors may be benign (not cancerous) or malignant (cancerous). Different types of malignant solid tumors are named for the type of cells that form them. Examples of malignant solid tumors are sarcomas, carcinomas, and lymphomas. Leukemia (blood cancer) does not usually form a malignant solid tumor (as defined by the NIH National Cancer Institute). Malignant solid tumors include, but are not limited to, clusters of abnormal cells that may originate from different tissue types, for example, the liver, colon, colorectum, skin, breast, pancreas, cervix, corpus uteri, bladder, gallbladder, kidney, larynx, lip, oral cavity, esophagus, ovary, prostate, stomach, testis, thyroid, or lungs, and thus malignant solid tumors include malignant solid liver tumors, colon tumors, colorectal tumors, skin tumors, breast tumors, pancreatic tumors, cervical tumors, uterine corpus tumors, bladder tumors, gallbladder tumors, renal tumors, laryngeal tumors, lip tumors, oral tumors, esophageal tumors, ovarian tumors, prostate tumors, stomach tumors, testicular tumors, thyroid tumors, or lung tumors.

For purposes of treatment herein, the term "subject" is preferably a subject in need of treatment for a target pathological condition, such as a tumor. For the purpose of prevention, the subject is preferably a subject at risk of or predisposed to developing the target pathological condition. The term "subject" includes living organisms such as prokaryotes and eukaryotes. Examples of subjects include mammals, such as humans, dogs, cows, horses, pigs, sheep, goats, cats, mice, rabbits, rats, and genetically modified non-human animals. In some preferred embodiments, the subject is a human.

As used herein, "treatment" is an approach for obtaining beneficial or desired clinical results. For purposes of this invention, beneficial or desired clinical results include, but are not limited to, one or more of the following: reducing the proliferation of (or destroying) neoplastic or cancerous cells, inhibiting metastasis of neoplastic cells, shrinking or decreasing the size of a tumor, remission of malignant tumor, decreasing symptoms resulting from malignant tumor, increasing the quality of life of those suffering from malignant tumor, decreasing the dose of other medications required to treat malignant tumor, delaying the progression of malignant tumor, curing a malignant tumor, and / or prolong survival of patients having malignant tumor.

As used herein, an "effective amount" or "effective dosage" of bacterium, medicament, or pharmaceutical composition is an amount sufficient to affect any one or more beneficial or desired results. For prophylactic use, beneficial or desired results include eliminating or reducing the risk, lessening the severity, or delaying the outset of the disease, including biochemical, histological and / or behavioral symptoms of the disease, its complications and intermediate pathological phenotypes presenting during development of the disease. For therapeutic use, beneficial or desired results include such as reducing one or more symptoms of a disease such as tumor, decreasing the dose of other medications required to treat the disease, enhancing the effect of another medication, and / or delaying the progression of the cancer in patients. For example, an "effective amount" preferably inhibits cell growth or tumor growth by at least about 10%, preferably at least about 20%, more preferably at least about 30%, more preferably at least about 40%, more preferably at least about 50%, more preferably at least about 60%, more preferably at least about 70%, more preferably at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition in vitro by assays known to the skilled practitioner. A therapeutically effective amount of a therapeutic compound can decrease tumor size, or otherwise ameliorate symptoms in a subject. One of ordinary skill in the art would be able to determine such amounts based on such factors as the subject's size, the severity of the subject's symptoms, and the particular composition or route of administration selected.

As used herein, "pharmaceutically acceptable carrier" includes any material which, when combined with an active ingredient, allows the ingredient to retain biological activity and is non-reactive with the subject's immune system, including but not limited to a disintegrating agent, an adhesive, a filler, a buffer, a tension agent, a stabilizer, an antioxidant, a surfactant or a lubricant. Preferably, the carrier is suitable for intravenous, intramuscular, subcutaneous, parenteral, spinal or epidermal administration (e.g., by injection or infusion). Depending on the route of administration, the bacterium of the present invention may be coated in a material to protect the compound from the action of acids and other natural conditions that may inactivate the bacterium. Pharmaceutically acceptable carriers include normal saline, PBS buffer, sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated.

### Modified bacterium

The present invention provides a modified bacterium having improved anti-tumor activity and/or improved safety.

In one embodiment, the modified bacterium of the invention comprise an inducible lipopolysaccharide (LPS) gene expression cassette comprising a gene involved in the biosynthesis or transport of LPS or a gene encoding a murein-lipoprotein under the control of an inducible promoter, as compared to the unmodified starting strain.

In one embodiment, the LPS gene expression cassette is a hypoxia regulated lipopolysaccharide (LPS) gene expression cassette comprising a gene involved in the biosynthesis or transport of LPS or a gene encoding a murein-lipoprotein under the control of a first strictly hypoxia inducible promoter.

In one embodiment, the gene involved in the biosynthesis of LPS is selected from a gene involved in the synthesis of O-antigen, a gene involved in the synthesis of core region, and a gene involved in the synthesis of lipid A. In one embodiment, the gene involved in the synthesis of O-antigen is selected from the group consisting of genes rfbU, rfbN, rfbV, wzzB, gtrBb, gtrBa, and rfbP/wbaP. In one embodiment, the gene involved in the synthesis of core regionis selected from the group consisting of genes rfaB, rfaC/waaC, rfaF, rfaG, yibD, rfaI, rfaJ, rfaK, rfaL, rfaP, rfaQ, rfaY, rfaZ, yijP, gmhA, yaeD, rfaE and rfaD. In one embodiment, the gene involved in the synthesis of lipid A is selected from the group consisting of genes lpxA, lpxB, lpxC, lpxD, lpxH, lpxK, htrB, msbB/lpxM, lpxO, lpxP, eiU, kdtA, arnT, pagP, yjdB, yhjw, kdsA, kdsB, yrbI, and yrbH. In one embodiment, the gene involved in the transport of LPS is selected from the group consisting of genes msbA, Wzy, wzxE, lptA, lptB, lptC/yrbK, lptD, and lptG.

In one embodiment, the gene involved in the biosynthesis or transport of LPS or the gene encoding a murein-lipoprotein is a naturally occurring gene in the chromosome of the bacterium, wherein the native promoter of the gene involved in the biosynthesis or transport of LPS or the gene encoding a murein-lipoprotein is functionally replaced by the inducible promoter, e.g., the first strictly hypoxia inducible promoter, whereby the expression of the gene involved in the biosynthesis or transport of LPS or the gene encoding a murein-lipoprotein in the bacterium is completely under the control of the inducible promoter, e.g., the first strictly hypoxia inducible promoter.

In one embodiment, the LPS gene expression cassette is exogenous, and the gene involved in the biosynthesis or transport of LPS or the gene encoding murein-lipoprotein naturally occurring in the chromosome of the bacterium is deleted or functionally inactivated, whereby the expression of the gene involved in the biosynthesis or transport of LPS or the gene encoding murein-lipoprotein in the bacterium is completely under the control of the inducible promoter, such as the first strictly hypoxia inducible promoter. In one embodiment, the exogenous LPS gene expression cassette is integrated into the bacterial chromosome. In one embodiment, the exogenous LPS gene expression cassette is outside of the bacterial chromosome. In one embodiment, the exogenous LPS gene expression cassette is present in a plasmid carried by the bacterium.

In one embodiment, the bacterium further comprises a hypoxia regulated essential gene expression cassette. In one embodiment, the essential gene expression cassette comprises an essential gene of the bacterium under the control of a second strictly hypoxia inducible promoter. In one embodiment, the bacterium is deficient in at least one gene involved in or regulating the endogenous anti-oxidative stress response pathway or a functional expression product thereof. In one embodiment, the bacterium is deficient in at least one gene required for survival in macrophages or a functional expression product thereof. In one embodiment, the bacterium expresses a wild-type lipopolysaccharide (LPS).

In one embodiment, the first and second strictly hypoxia inducible promoters are each independently selected from pepTp, fnrSp, ysgAp, ssbpl, Hip1, BBa_I14018, BBa_R1074, Ptet-arcA, and Ptet-Fnr. Preferably, the first and second strictly hypoxia inducible promoters are each independently selected from the group consisting of fnrSp and/or ssbp1 promoters.

In one embodiment, the modified bacterium of the invention comprises a hypoxia regulated essential gene expression cassette, and the bacterium is deficient in at least one non-essential gene involved in the biosynthesis or transport of LPS and/or a gene encoding murein-lipoprotein or an expression product thereof, as compared to the unmodified starting strain.

In one embodiment, the gene involved in the biosynthesis of LPS is selected from a gene involved in the synthesis of O-antigen, a gene involved in the synthesis of core region, and a gene involved in the synthesis of lipid A. In one embodiment, the gene involved in the synthesis of O-antigen is selected from the group consisting of genes wzzB, gtrBb, gtrBa, and rfbP/wbaP. In one embodiment, the gene involved in the synthesis of core regionis selected from the group consisting of genes rfaB, yibD, rfaI, rfaJ, rfaK, rfaL, rfaP, rfaQ, rfaZ, yijP, gmhA, yaeD, rfaE and rfaD. In one embodiment, the gene involved in the synthesis of lipid A is selected from the group consisting of genes lpxA, lpxC, lpxD, lpxH, msbB/lpxM, lpxO, lpxP, arnT, yjdB, kdsB, yrbI, and yrbH. In one embodiment, the gene involved in the transport of LPS is selected from the group consisting of genes msbA, Wzy, lptA, lptB, and lptG.

In one embodiment, the essential gene expression cassette comprises an essential gene of the bacterium under the control of a strictly hypoxia inducible promoter. In one embodiment, the bacterium is deficient in at least one gene involved in or regulating the endogenous anti-oxidative stress response pathway or a functional expression product thereof. In one embodiment, the bacterium is deficient in at least one gene required for survival in macrophages or a functional expression product thereof.

In one embodiment, the bacterium does not express a wild-type lipopolysaccharide (LPS). In one embodiment, the bacterium does not express LPS or express mutant or structurally incomplete LPS.

In one embodiment, the strictly hypoxia inducible promoter is selected from pepTp, fnrSp, ysgAp, ssbp1, Hipl, BBa_I14018, BBa_R1074, Ptet-arcA, and Ptet-Fnr. Preferably, the strictly hypoxia inducible promoter is ssbpl promoter.

In one embodiment, the expression product of the essential gene is responsible for the synthesis of 2,6-diaminopimelic acid (DAP) in the bacterium. In one embodiment, when cultured under aerobic conditions, the bacterial growth relies on the additional DAP or an analog thereof added to the medium. In one embodiment, the essential gene is selected from *dapA, dapB, dapD, dapE, argD, dapF,* and any combination thereof. Preferably, the essential gene is selected from *dapA* and *dapE.* Preferably, the dapE comprises a nucleotide sequence having a sequence identity of at least 81%, preferably at least 82%, more preferably at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or most preferably at least 99% to SEQ ID NO: 19. Preferably, the nucleotide sequence of dapE comprises SEQ ID NO: 19. Preferably, the nucleotide sequence of dapE consists of SEQ ID NO: 19.

In one embodiment, the gene involved in or regulating the endogenous anti-oxidative stress response pathway is an HtrA serine protease family gene. In one embodiment, the functional expression product of the gene involved in or regulating the endogenous anti-oxidative stress response pathway is an HtrA serine protease family related protein. In one embodiment, the bacterium is deficient in the activity of HtrA serine protease. In one embodiment, the gene involved in or regulating the endogenous anti-oxidative stress response pathway is *htrA.* In one embodiment, the bacterium is deficient in *htrA.* Preferably, the *htrA* comprises a nucleotide sequence having a sequence identity of at least 81%, preferably at least 82%, more preferably at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or most preferably at least 99% to SEQ ID NO: 20. Preferably, the nucleotide sequence of *htrA* comprises SEQ ID NO: 20. Preferably, the nucleotide sequence of *htrA* consists of SEQ ID NO: 20.

In one embodiment, the essential gene is a gene naturally occurring in the bacterial chromosome. In one embodiment, the native promoter of the essential gene is functionally replaced by the second strictly hypoxia inducible promoter. Thus, the expression of the essential gene in the bacterium is completely under the control of the second strictly hypoxia inducible promoter.

In one embodiment, the essential gene expression cassette is exogenous. In one embodiment, the essential gene naturally occurring in the bacterial chromosome is deleted or functionally inactivated. Thus, the expression of the essential gene in the bacterium is completely under the control of the second strictly hypoxia inducible promoter. In one embodiment, the exogenous essential gene expression cassette is integrated into the bacterial chromosome. In one embodiment, the exogenous essential gene expression cassette is outside of the bacterial chromosome. In one embodiment, the exogenous essential gene expression cassette is present in a plasmid carried by the bacterium.

In one embodiment, the modified bacterium further comprise a pH regulated expression cassette. In one embodiment, the modified bacterium comprises a hypoxia regulated essential gene expression cassette and a pH regulated expression cassette, as compared to an unmodified starting strain. In one embodiment, the pH regulated expression cassette comprises a gene encoding a bacteria-derived hemolysin protein under the control of a promoter that is active under acid pH condition. According to some embodiments of the present invention, the promoter that is active under acid pH condition is active in a pH range of about pH 4.0 to 7.0. In some embodiments, the promoter that is active under acid pH condition is also active at pH 6.0 ± 1. In some embodiments, the promoter that is active under acid pH condition is active at pH values below 7.0, 6.9, 6.8, 6.7, 6.6, 6.5, 6.4, 6.3, 6.2, 6.1, 6.0, 5.9, 5.8, 5.7, 5.6, or 5.5, or between any two of these values. In one embodiment, the promoter that is active under acid pH conditions is selected from the group consisting of sseA, ssrA, ssaB, ssaG, ssaM, and ssaR. In one embodiment, the promoter that is active under acid pH condition is sseA. In one embodiment, the bacteria-derived hemolysin protein is a Gram-negative bacterial hemolysin protein. In one embodiment, the gene encoding the gram-negative bacterial hemolysin protein is *hlyA* or *hlyE.* In one embodiment, the *hlyA* or *hlyE* is derived from *Listeria monocytogenes, Vibrio cholerae,* or *Escherichia coli.* Preferably, the *hlyA* comprises a nucleotide sequence having a sequence identity of at least 81%, preferably at least 82%, more preferably at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or most preferably at least 99% to SEQ ID NO: 21. Preferably, the nucleotide sequence of *hlyA* comprises SEQ ID NO: 21. Preferably, the nucleotide sequence of *hlyA* consists of SEQ ID NO: 21.

In one embodiment, the unmodified starting strain is a facultative anaerobic bacterium. In one embodiment, the bacterium is of *Enterobacteriaceae.* Preferably, the bacterium is a bacteirum from *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.* Preferably, the bacterium is selected from the group consisting of *Escherichia coli, Enterobacter blattae (E. blattae), Enterobacter fergusonii (E. fergusonii), Enterobacter hermannii (E. hermannii), Enterobacter vulneris (E. vulneris), Salmonella enterica (S. enterica), Salmonella bongori (S. bongori), Salmonella typhi (S. typhi), Salmonella choleraesuis (S. choleraesuis), Salmonella typhimurium (S. typhimurium), Shigella dysenteriae (S. dysenteriae), Shigella flexneri (S. flexneri), Shigella boydii (S. boydii), Shigella sonnei (S. sonnei), Klebsiella pneumoniae (K. peumoniae), Klebsiella oxytoca (K. oxytoca), Yersinia pestis (Y. pestis), Yersinia enterocolitica (Y. enterocolitica), Yersinia pseudotuberculosis (Y. pseudotuberculosis), Yersinia aldouae (Y. aldouae), Yersinia bercovieri (Y. bercovieri), Yersinia frederiksenii (Y. frederiksenii), Yersinia intermedia (Y. intermedia), Yersinia kristersenii (Y. kristersenii), Yersinia mollaretti (Y. mollaretti), Yersinia rohdei (Y. rohdei), Yersinia ruckeri (Y. ruckeri), Citrobacter freundii (C. freundii), Citrobacter koseri (C. kaseri), Citrobacter braakii (C. braakii), Enterobacter aerogenes (E. aerogenes), Enterobacter cloacae (E. cloacae), Enterobacter gergoviae (E. gergoviac), Enterobacter sakazakii (E. sakazakii), Enterobacter taylorae (E. tavlorae), Enterobacter amnigenus (E. aminigenus), Enterobacter intermedius (E. intermedius), Enterobacter asburiae (E. asburiac), Enterobacter cancerogenus (E. cancerogenus), Enterobacter dissolvens (E. dissolvens), Enterobacter nimipressuralis (E. nimipressuralis), Serratia marcescens (S. marcescens), Serratia entomophila (S. entomophila), Serratia ficaria (S. ficaria), Serratia fonticola (S. fonticola), Serratia grimesii (S. grimesii), Serratia liquefaciens (S. liquefaciens), Serratia odorifera (S. odorifera), Serratia plymuthica (S. plymuthica), Serratia proteamaculans (S. proteamaculans), Serratia rubidaea (S. rubidaea), Serratia ureilytica (S. ureilytica), Proteus mirabilis (P. mirabilis), Proteus vulgaris (P. vulgaris), Proteus myxofaciens (P. myxofaciens), Proteus penneri (P. penneri), Proteus hauseri (P. hauseri)), Morganella morganii (M. morganii), Providencia alcalifaciens (P. alcalifaciens), Providencia rustigianii (P. rustigianii), Providencia stuartii (P. stuartii), Providencia rettgeri (P. rettgeri), Providencia heimbachae (P. heimbochae), Hafnia alvei (H. alvei),* and *Pantoea agglomerans (P. agglomerans).* Preferably, the bacterium is of *Salmonella typhimurium.* Preferably, the starting strain is *Salmonella typhimurium* SL7207.

In one embodiment, when administrated to a subject with a tumor, the bacterium can survive and proliferate in the tumor tissue but is rapidly cleared in normal tissue. In one embodiment, when administrated to a subject with a malignant tumor, the bacterium can induce anti-tumor specific immune response. In one embodiment, when administrated to a subject with a malignant tumor, the bacterium can induce anti-tumor immune memory.

In one embodiment, the bacterium does not express a wild-type flagellin. In one embodiment, the modified bacterium is deficient in *the fliC* gene.

In one embodiment, the survival level of the bacterium in macrophages ranges from about 50% to about 30% of the survival level of the unmodified starting strain. In one embodiment, the survival level of the bacterium in macrophages ranges from about 30% to about 10% of the survival level of the unmodified starting strain. Preferably, the survival level of the bacterium in macrophages ranges from about 10% to about 1% of the survival level of the unmodified starting strain.

### Pharmaceutical composition

The present invention provides a pharmaceutical composition comprising an effective amount of the modified bacterium of the present invention. In one embodiment, the modified bacterium is a live bacterium.

In one embodiment, the pharmaceutical composition is used for treating a malignant tumor. In one embodiment, the pharmaceutical composition is used for inducing an anti-tumor specific immune response in a subject with a malignant tumor. In one embodiment, the pharmaceutical composition is used for inducing anti-tumor immune memory in a subject with a malignant tumor. In one embodiment, the pharmaceutical composition is used for preventing or treating metastasis or recurrence of a malignant tumor. In one embodiment, the pharmaceutical composition is used for treating a malignant tumor that is resistant to, or has failed to, a prior anti-tumor therapy.

In one embodiment, the malignant tumor is a malignant tumor from the nervous system, respiratory system, digestive system, urinary system, reproductive system, hematopoietic system, lymphatic system, endocrine system, or skin mucosa. In some embodiments, the malignant tumor is a sarcoma or cancer. In one embodiment, the malignant tumor is a solid tumor. In one embodiment, the malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

In one embodiment, the pharmaceutical composition further comprises a pharmaceutically acceptable carrier.

### Treatment Methods and Uses

The present invention provides a method for treating a malignant tumor, comprising administering an effective amount of the modified bacterium of the present invention or the pharmaceutical composition of the present invention to a subject with the malignant tumor.

The present invention provides a method for inducing an anti-tumor specific immune response in a subject with a malignant tumor, comprising administering an effective amount of the modified bacterium of the present invention or the pharmaceutical composition of the present invention to a subject with the malignant tumor.

The present invention provides a method for inducing anti-tumor immune memory in a subject with a malignant tumor, comprising administering an effective amount of the modified bacterium of the present invention or the pharmaceutical composition of the present invention to a subject with the malignant tumor.

The present invention provides a method for preventing or treating metastasis or recurrence of a malignant tumor, comprising administering an effective amount of the modified bacterium of the present invention or the pharmaceutical composition of the present invention to a subject with the malignant tumor.

The present invention provides a method for preventing or treating metastasis or recurrence of a malignant tumor, comprising administering an effective amount of the modified bacterium of the present invention or the pharmaceutical composition of the present invention to a subject with the malignant tumor or at a high risk of metastasis or recurrence of the malignant tumor.

The present invention provides a method for treating a malignant tumor that is resistant to, or has failed to, a prior anti-tumor therapy, comprising administering an effective amount of the modified bacterium of the present invention or the pharmaceutical composition of the present invention to a subject with the malignant tumor that is resistant to, or has failed to, a prior anti-tumor therapy.

The present invention provides use of the modified bacterium of the present invention in the manufacture of a medicament in the treatment of a malignant tumor.

The present invention provides use of the modified bacterium of the present invention in the manufacture of a medicament for inducing an anti-tumor specific immune response in a subject with a malignant tumor.

The present invention provides use of the modified bacterium of the present invention in the manufacture of a medicament for inducing anti-tumor immune memory in a subject with a malignant tumor.

The present invention provides use of the modified bacterium of the present invention in the manufacture of a medicament for preventing or treating metastasis or recurrence of a malignant tumor.

The present invention provides use of the modified bacterium of the present invention in the manufacture of a medicament for treating a malignant tumor that is resistant to, or has failed to, a prior anti-tumor therapy.

In one embodiment, the modified bacterium is a live bacterium.

In one embodiment, the malignant tumor is a malignant tumor from the nervous system, respiratory system, digestive system, urinary system, reproductive system, hematopoietic system, lymphatic system, endocrine system, or skin mucosa. In some embodiments, the malignant tumor is a sarcoma or cancer. In one embodiment, the malignant tumor is a solid tumor. In one embodiment, the malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

In one embodiment, the bacterium, medicament, or pharmaceutical composition of the present invention is administered by intravenous, intratumoral, intramuscular, subcutaneous, intraperitoneal, transcerebral, gastrointestinal, body surface, oral mucosal, transnasal, transrectal, or transvaginal routes. In one embodiment, the bacterium, medicament, or pharmaceutical composition of the present invention may be formulated in a form that is administered by intravenous, intratumoral, intramuscular, subcutaneous, intraperitoneal, transcerebral, gastrointestinal, body surface, oral mucosal, transnasal, transrectal, or transvaginal routes.

In one embodiment, the dosage form of the medicament or pharmaceutical composition of the present invention may, for example, be in the form of a solution, an emulsion, a freeze-dried preparation or suspension; for oral administration, the dosage form may be in the form of tablets or capsules; for intranasal dosage, the dosage form may be in the form of powder, drop or aerosol; for body surface administration, the dosage form may be in the form of an aqueous solution, a suspension, an ointment, a cream or a gel; and for rectal or vaginal administration, the dosage form may be a suppository, an enema, or delivered as part of an endoscopy or colonoscopy procedure. In one embodiment, the medicament or pharmaceutical composition is formulated in the form of a solution, an emulsion, a freeze-dried preparation or suspension; for oral administration, it may be formulated in the form of a tablet or capsule; for intranasal dosage form, it may be formulated in the form of powder, nasal drops, or aerosol; for body surface administration, it may be formulated in the form of an aqueous solution, a suspension, an ointment, a cream, or a gel; and for rectal or vaginal administration, it can be formulated as a suppository, an enema or delivered as part of an endoscopy or colonoscopy procedure. For methods known in the art for preparing dosage forms see, for example, "Remington's Pharmaceutical Sciences" (20th ed.), edited by A. Gennaro, 2000, Mack Publishing Company, Inc. Easton, PA.

In some embodiments, the bacterium, medicament, or pharmaceutical composition of the present invention may be administered at various intervals, including but not limited to: 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, 35 minutes, 40 minutes, 45 minutes, 50 minutes, 55 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 2 days, 3 days, 4 days, 5 days, 6 days, 7 days, 8 days, 9 days, or 10 days, including the range between any two of the listed values, for example: 1 minute to 10 minutes, 1 minute to 30 minutes, 1 minute to 1 hour, 1 minute to 2 hours, 1 minute to 4 hours, 1 minute to 12 hours, 1 minute to 18 hours, 1 minute to 1 day, 10 minutes to 30 minutes, 10 minutes to 1 hour, 10 minutes to 2 hours, 10 minutes to 4 hours, 10 minutes to 12 hours, 10 minutes to 18 hours, 10 minutes to 1 day, 30 minutes to 1 hour, 30 minutes to 2 hours, 30 minutes to 4 hours, 30 minutes to 12 hours, 30 minutes to 18 hours, 30 minutes to 1 day, 30 minutes to 2 days, 1 hour to 2 hours, 1 hour to 4 hours, 1 hour to 12 hours, 1 hour to 18 hours, 1 hour to 1 day, 4 hours to 12 hours, 4 hours to 18 hours, 4 hours to 1 day, 1 hour to 2 days, 1 hour to 3 days, 1 hour to 4 days, 1 hour to 5 days, 1 hour to 7 days, 1 hour to 10 days, 2 hours to 3 days, 2 hours to 4 days, 2 hours to 5 days, 2 hours to 7 days, 2 hours to 10 days, or 5 days to 10 days. In some embodiments, the bacterium, medicament, or pharmaceutical composition is administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. In some embodiments, the bacterium, medicament, or pharmaceutical composition of the present invention may be formulated to be administered at various intervals, including but not limited to: 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, 35 minutes, 40 minutes, 45 minutes, 50 minutes, 55 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 2 days, 3 days, 4 days, 5 days, 6 days, 7 days, 8 days, 9 days, or 10 days, including the range between any two of the listed values, for example: 1 minute to 10 minutes, 1 minute to 30 minutes, 1 minute to 1 hour, 1 minute to 2 hours, 1 minute to 4 hours, 1 minute to 12 hours, 1 minute to 18 hours, 1 minute to 1 day, 10 minutes to 30 minutes, 10 minutes to 1 hour, 10 minutes to 2 hours, 10 minutes to 4 hours, 10 minutes to 12 hours, 10 minutes to 18 hours, 10 minutes to 1 day, 30 minutes to 1 hour, 30 minutes to 2 hours, 30 minutes to 4 hours, 30 minutes to 12 hours, 30 minutes to 18 hours, 30 minutes to 1 day, 30 minutes to 2 days, 1 hour to 2 hours, 1 hour to 4 hours, 1 hour to 12 hours, 1 hour to 18 hours, 1 hour to 1 day, 4 hours to 12 hours, 4 hours to 18 hours, 4 hours to 1 day, 1 hour to 2 days, 1 hour to 3 days, 1 hour to 4 days, 1 hour to 5 days, 1 hour to 7 days, 1 hour to 10 days, 2 hours to 3 days, 2 hours to 4 days, 2 hours to 5 days, 2 hours to 7 days, 2 hours to 10 days, or 5 days to 10 days. In some embodiments, the bacterium, medicament, or pharmaceutical composition is formulated to be administered once a week, once every two weeks, once every three weeks, once every four weeks, once every five weeks, once every six weeks, once every seven weeks, once every eight weeks, once every nine weeks, once every ten weeks, once every fifteen weeks, once every twenty weeks, once every twenty-five weeks, or once every twenty-six weeks. The dosage regimen may depend on the pharmacokinetic decay pattern expected to be achieved by the practitioner. Progress in therapy can be monitored by routine techniques and assays. The dosing regimen can change over time.

In some embodiments, the effective amount of bacteria in the medicament or pharmaceutical composition comprises at least about 10⁴ colony forming units (cfu), e.g., at least about 10⁴, 10⁵, 10⁶, 10⁷, 10⁸, 10⁹, 10¹⁰, 10¹¹, 10¹², or 10¹³ cfu, including ranges between any of the listed values, e.g., 10⁴-10⁸cfu, 10⁴-10⁹cfu, 10⁴-10¹⁰cfu, 10⁴-10¹¹cfu, 10⁴-10¹²cfu, 10⁴-10¹²cfu, 10⁵-10⁸cfu, 10⁵-10⁹cfu, 10⁵-10¹⁰cfu, 10⁵-10¹¹cfu, 10⁵-10¹²cfu, 10⁵-10¹²cfu, 10⁶-10⁸cfu, 10⁶-10⁹cfu, 10⁶-10¹⁰cfu , 10⁶-10¹¹cfu, 10⁶-10¹²cfu, 10⁶-10¹²cfu, 10⁷-10⁸cfu, 10⁷-10⁹cfu, 10⁷-10¹⁰cfu , 10⁷-10¹¹cfu, 10⁷-10¹²cfu, 10⁷-10¹²cfu, 10⁸-10⁹cfu, 10⁸-10¹⁰cfu, 10⁸-10¹¹cfu, 10⁸-10¹²cfu or 10⁸-10¹²cfu.

In some embodiments, the volume of an effective dose of the bacterium, medicament, or pharmaceutical composition of the present invention is less than or equal to about 3 mL, about 2.5 mL, about 2 mL, about 1.5 mL, about 1 mL, about 0.75 mL, about 0.5 mL, about 0.25 mL, or about 0.1 mL. In some embodiments, the volume of an effective dose of the bacterium, medicament, or pharmaceutical composition is about 20 mL, about 19 mL, about 18 mL, about 17 mL, about 16 mL, about 15 mL, about 14 mL, about 13 mL, about 12 mL, about 11 mL, about 10 mL, about 9 mL, about 8 mL, about 7 mL, about 6 mL, about 5 mL, about 4 mL, about 3 mL, about 2 mL, or about 1 mL. Alternatively, the volume of an effective dose of the bacterium, medicament, or pharmaceutical composition is about 20.5 mL, about 19.5 mL, about 18.5 mL, about 17.5 mL, about 16.5 mL, about 15.5 mL, about 14.5 mL, about 13.5 mL, about 12.5 mL, about 11.5 mL, about 10.5 mL, about 9.5 mL, about 8.5 mL, about 7.5 mL, about 6.5 mL, about 5.5 mL, about 4.5 mL, about 3.5 mL, about 2.5 mL, about 1.5 mL, or about 0.5 mL. In some embodiments, the volume of an effective dose of the bacterial, medicament, or pharmaceutical composition is about 200 mL, about 190 mL, about 180 mL, about 170 mL, about 160 mL, about 150 mL, about 140 mL, about 130 mL, about 120 mL, about 110 mL, about 100 mL, about 90 mL, about 80 mL, about 70 mL, about 60 mL, about 50 mL, about 40 mL, or about 30 mL, as well as the range between any two of the listed values, for example, 100 mL-110 mL, 100 mL-120 mL, 90 mL-120 mL, 90 mL-130 mL. Alternatively, the volume of an effective dose of the bacterium, medicament, or pharmaceutical composition is about 205 mL, about 195 mL, about 185 mL, about 175 mL, about 165 mL, about 155 mL, about 145 mL, about 135 mL, about 125 mL, about 115 mL, about 105 mL, about 95 mL, about 85 mL, about 75 mL, about 65 mL, about 55 mL, about 45 mL, about 35 mL, or about 25 mL, as well as the range between any two of the listed values, for example, 105 mL-115 mL, 105 mL-125 mL, 95 mL-125 mL, 95 mL-135 mL. Alternatively, the volume of an effective dose of the bacterium, medicament or pharmaceutical composition is about 900 microlitres, about 800 microlitres, about 700 microlitres, about 600 microlitres, about 500 microlitres, about 400 microlitres, about 300 microlitres, about 200 microlitres, or about 100 microlitres, optionally about 950 microlitres, about 850 microlitres, about 750 microlitres, about 650 microlitres, about 550 microlitres, about 450 microlitres, about 350 microlitres, about 250 microlitres, about 150 microlitres, or about 50 microlitres. In some embodiments, the volume of an effective dose of the bacterium, medicament, or pharmaceutical composition is less than or equal to about 2.0 mL.

In the medicament or pharmaceutical composition, for example, the concentration of bacterium of the present invention may be at least about 10 ³, 10 ⁴, 10 ⁵, 10 ⁶, 10 ⁷, 10 ⁸, 10⁹, 10 ¹⁰, 10 ¹¹, 10 ¹² or 10 ¹³cfu/ml, including a range between any of the listed values, for example, 10 ³-10 ⁸cfu/ml, 10 ³-10 ⁹cfu/ml, 10 ³-10 ¹⁰cfu/ml, 10 ³-10 ¹¹cfu/ml, 10 ³-10 ¹²cfu/ml, 10 ³-10 ¹³cfu/ml, 10 ⁴-10 ⁸cfu/ml, 10 ⁴-10 ⁹cfu/ml, 10 ⁴-10 ¹⁰cfu/ml, 10⁴-10 ¹¹cfu/ml, 10 ⁴-10 ¹²cfu/ml, 10 ⁴-10 ¹³cfu/ml, 10 ⁵-10 ⁸cfu/ml, 10 ⁵-10 ⁹cfu/ml, 10 ⁵-10 ¹⁰cfu/ml, 10 ⁵-10 ¹¹cfu/ml, 10⁵-10 ¹²cfu/ml, 10 ⁵-10 ¹³cfu/ml, 10 ⁶-10 ³cfu/ml, 10 ⁶-10 ⁹cfu/ml, 10 ⁶-10 ¹⁰cfu/ml, 10 ⁶-10 ¹¹cfu/ml, 10 ⁶-10 ¹²cfu/ml, 10 ⁶-10 ¹³cfu/ml, 10 ⁷-10 ⁸cfu/ml, 10 ⁷-10 ⁹cfu/ml, 10 ⁷-10 ¹⁰cfu/ml, 10 ⁷-10 ¹¹cfu/ml, 10 ⁷-10 ¹²cfu/ml, 10 ⁷-10 ¹³cfu/ml, 10 ⁸-10 ⁹cfu/ml, 10 ⁸-10 ¹⁰cfu/ml, 10 ⁸-10 ¹¹cfu/ml, 10 ⁸-10 ¹²cfu/ml or 10 ⁸-10 ¹³cfu/ml. In the medicament or pharmaceutical composition, for example, the concentration of bacterium of the present invention may be at least about 10³, 10⁴, 10⁵, 10⁶, 10⁷, 10⁸, 10⁹, 10¹⁰, 10¹¹, 10¹² or 10¹³cfu/g, including a range between any of the listed values, for example, 10³-10⁸cfu/g, 10³-10⁹cfu/g, 10³-10¹⁰cfu/g, 10³-10¹¹cfu/g, 10³-10¹²cfu/g, 10³-10¹³cfu/g, 10⁴-10⁸cfu/g, 10⁴-10⁹cfu/g, 10⁴-10¹⁰cfu/g, 10⁴-10¹¹cfu/g, 10⁴-10¹²cfu/g, 10⁴-10¹³cfu/g, 10⁵-10⁸cfu/g, 10⁵-10⁹cfu/g, 10⁵-10¹⁰cfu/g, 10⁵-10¹¹cfu/g, 10⁵-10¹²cfu/g, 10⁵-10¹³cfu/g, 10⁶-10⁸cfu/g, 10⁶-10⁹cfu/g, 10⁶-10¹⁰cfu/g, 10⁶-10¹¹cfu/g, 10⁶-10¹²cfu/g, 10⁶-10¹³cfu/g, 10⁷-10⁸cfu/g, 10⁷-10⁹cfu/g, 10⁷-10¹⁰cfu/g, 10⁷-10¹¹cfu/g, 10⁷-10¹²cfu/g, 10⁷-10¹³cfu/g, 10⁸-10⁹cfu/g, 10⁸-10¹⁰cfu/g, 10⁸-10¹¹cfu/g, 10⁸-10¹²cfu/g or 10⁸-10¹³cfu/g.

Dosage ranges set forth herein are exemplary only and do not limit the dosage ranges that may be selected by medical practitioners. The amount of active compound (e.g., the bacterium of the invention) in the pharmaceutical composition of the present invention may vary according to factors such as the disease state, age, sex, and weight of the individual, including the presence or absence of the malignant tumor, the type and severity of the malignant tumor to be treated, the activity or viability of the bacterium, medicament or pharmaceutical composition, the route of administration, the duration of treatment, the medicament, if any, used in combination with the bacterium, medicament or pharmaceutical composition, the dietary and general health status of the subject, and similar factors well known in the art. Dosage regimens may be adjusted to provide the optimum therapeutic response. For example, a single dose may be administered, several divided doses may be administered over time or the dose may be proportionally reduced or increased as indicated by the exigencies of the therapeutic situation.

The bacterium, medicament or pharmaceutical composition of the present invention may be manufactured by methods well known in the art such as microbial growth in fermenters, followed by concentration and washing by centrifugation, filtration or dialysis, conventional granulating, mixing, dissolving, encapsulating, lyophilizing, or emulsifying processes, among others. The bacterium, medicament or pharmaceutical composition of the present invention may be produced in various forms, including granules, precipitates, or particulates, powders, including freeze dried, rotary dried or spray dried powders, amorphous powders, injections, emulsions, elixirs, suspensions or solutions. Formulations may optionally contain stabilizers, pH modifiers, surfactants, bioavailability modifiers and combinations of these.

In one embodiment, the bacterium, medicament or pharmaceutical composition of the present invention may be administered alone or in combination with other compounds or compositions in the presence of a carrier. In one embodiment, the bacterium, medicament or pharmaceutical composition may be administered in combination with other malignancy therapies, including, but not limited to, radiotherapy, chemotherapy, and surgery. The bacterium, medicament or pharmaceutical composition may be used as an adjuvant in therapies in such cases. Therefore, another aspect of the embodiment relates to a bacterium, medicament, or pharmaceutical composition for use as an adjuvant in a malignancy therapy selected from radiotherapy and chemotherapy.

### Exemplary Embodiments

Embodiment 1: A modified bacterium, wherein the bacterium comprises an inducible lipopolysaccharide (LPS) gene expression cassette comprising a gene involved in the biosynthesis or transport of LPS under the control of an inducible promoter or a gene encoding a mureinlipoprotein, as compared to an unmodified starting strain, for example, the LPS gene expression cassette is a hypoxia regulated LPS gene expression cassette comprising a gene involved in the biosynthesis or transport of LPS or a gene encoding a murein-lipoprotein under the control of a first strictly hypoxia inducible promoter.

Embodiment 2. The modified bacterium of embodiment 1, wherein the gene involved in the biosynthesis of LPS is selected from the group consisting of a gene involved in the synthesis of O-antigen, a gene involved in the synthesis of core region, and a gene involved in the synthesis of lipid A.

Embodiment 3. The modified bacterium of embodiment 2, wherein the gene involved in the synthesis of O-antigen is selected from the group consisting of genes rfbN, rfbV, wzzB, gtrBb, gtrBa, and rfbP/wbaP.

Embodiment 4. The modified bacterium of embodiment 2, wherein the gene involved in the synthesis of core region is selected from the group consisting of genes rfaB, rfaC/waaC, rfaF, rfaG, yibD, rfaI, rfaJ, rfaK, rfaL, rfaP, rfaQ, rfaY, rfaZ, yijP, gmhA, yaeD, rfaE, and rfaD.

Embodiment 5. The modified bacterium of embodiment 2, wherein the gene involved in the synthesis of lipid A is selected from the group consisting of genes lpxA, lpxB, lpxC, lpxD, lpxH, lpxK, htrB, msbB/lpxM, lpxO, lpxP, yeU, kdtA, arnT, pagP, yjdB, yhjw, kdsA, kdsB, yrbI, and yrbH.

Embodiment 6. The modified bacterium of embodiment 1, wherein the gene involved in the transport of LPS is selected from the group consisting of genes msbA, Wzy, wzxE, lptA, lptB, lptC/yrbK, lptD, and lptG.

Embodiment 7. The modified bacterium of any one of embodiments 1-6, wherein the gene involved in the biosynthesis or transport of LPS or the gene encoding murein-lipoprotein is a gene naturally occurring in the bacterial chromosome, wherein the native promoter of the gene involved in the biosynthesis or transport of LPS or the gene encoding murein-lipoprotein is functionally replaced by the inducible promoter, e.g., the first strictly hypoxia inducible promoter, whereby the expression of the gene involved in the biosynthesis or transport of LPS or the gene encoding murein-lipoprotein in the bacterium is completely under the control of the inducible promoter, e.g., the first strictly hypoxia inducible promoter.

Embodiment 8. The modified bacterium of any one of embodiments 1-6, wherein the LPS gene expression cassette is exogenous, and the gene involved in the biosynthesis or transport of LPS or the gene encoding murein-lipoprotein naturally occurring in the bacterial chromosome is deleted or functionally inactivated, whereby the expression of the gene involved in the biosynthesis or transport of LPS or the gene encoding murein-lipoprotein in the bacterium is completely under the control of the inducible promoter, such as the first strictly hypoxia inducible promoter.

Embodiment 9. The modified bacterium of embodiment 8, wherein the exogenous LPS gene expression cassette is integrated into the bacterial chromosome.

Embodiment 10. The modified bacterium of embodiment 8, wherein the exogenous LPS gene expression cassette is located outside of the bacterial chromosome.

Embodiment 11. The modified bacterium of embodiment 10, wherein the exogenous LPS gene expression cassette is present in a plasmid carried by the bacterium.

Embodiment 12. The modified bacterium of any of embodiments 1-11, wherein the first strictly hypoxia inducible promoter is selected from pepTp, fnrSp, ysgAp, ssbp1, Hipl, BBa_I14018, BBa_R1074, Ptet-arcA, and Ptet-Fnr.

Embodiment 13. The modified bacterium of any one of embodiments 1-12, wherein the first strictly hypoxia inducible promoter is a fnrSp promoter.

### Embodiments A

Embodiment A1: The modified bacterium of any one of embodiments 1-13, wherein the bacterium comprise a hypoxia regulated essential gene expression cassette comprising an essential gene of the bacterium under the control of a second strictly hypoxia inducible promoter, and the bacterium is deficient in at least one gene involved in or regulating the endogenous anti-oxidative stress response pathway or a functional expression product thereof.

Embodiment A2: The modified bacterium of embodiment A1, wherein the second strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment A3: The modified bacterium of embodiment A1, wherein the second strictly hypoxia inducible promoter is ssbpl promoter.

Embodiment A4: The modified bacterium of any one of the preceding embodiments A, wherein the expression product of the essential gene is responsible for synthesis of 2,6-diaminopimelic acid (DAP) in the bacterium, and when cultured under aerobic conditions, the bacterial growth is dependent on the additional DAP or an analog thereof added to the medium.

Embodiment A5: The modified bacterium of embodiment A4, wherein the essential gene is selected from *dapA, dapB, dapD, dapE, argD, dapF,* and any combination thereof.

Embodiment A6: The modified bacterium of embodiment A4, wherein the essential gene is selected from *dapA* and *dapE.*

Embodiment A7: The modified bacterium of any one of the preceding embodiments A, wherein the gene involved in or regulating the endogenous anti-oxidative stress response pathway is an HtrA serine protease family gene.

Embodiment A8: The modified bacterium of any one of the preceding embodiments A, wherein the functional expression product of the gene involved in or regulating the endogenous anti-oxidative stress response pathway is an HtrA serine protease family related protein.

Embodiment A9: The modified bacterium of any one of the preceding embodiments A, wherein the bacterium is deficient in the activity of HtrA serine protease.

Embodiment A10: The modified bacterium of any one of the preceding embodiments A, wherein the gene involved in or regulating the endogenous anti-oxidative stress response pathway is *htrA.*

Embodiment A11: The modified bacterium of any one of the preceding embodiments A, wherein the bacterium is deficient in *htrA.*

Embodiment A12: The modified bacterium of any one of embodiments A1-A11, wherein the essential gene is a gene naturally occurring in the bacterial chromosome, and wherein the native promoter of the essential gene is functionally replaced by the second strictly hypoxia inducible promoter, whereby the expression of the essential gene in the bacterium is completely under the control of the strictly hypoxia inducible promoter.

Embodiment A13: The modified bacterium of any one of embodiments A1-A11, wherein the essential gene expression cassette is exogenous, and the essential gene naturally occurring in the bacterial chromosome is deleted or functionally inactivated, whereby the expression of the essential gene in the bacterium is completely under the control of the second strictly hypoxia inducible promoter.

Embodiment A14: The modified bacterium of embodiments A13, wherein the exogenous essential gene expression cassette is integrated into the bacterial chromosome.

Embodiment A15: The modified bacterium of embodiments A13, wherein the exogenous essential gene expression cassette is outside of the bacterial chromosome.

Embodiment A16: The modified bacterium of embodiments A15, wherein the exogenous essential gene expression cassette is present in the plasmid carried by the bacterium.

Embodiment A17: The modified bacterium of any one of the preceding embodiments A, which further comprises a pH regulated expression cassette, wherein the pH regulated expression cassette comprises a gene encoding a bacteria-derived hemolysin protein under the control of a promoter that is active under acid pH condition.

Embodiment A18: The modified bacterium of embodiments A17, wherein the bacteria-derived hemolysin protein is a gram-negative bacterial hemolysin protein.

Embodiment A19: The modified bacterium of embodiments A18, wherein the gene encoding the gram-negative bacterial hemolysin protein is *hlyA* or *hlyE.*

Embodiment A20: The modified bacterium of embodiments A19, wherein the *hlyA* or *hlyE* is derived from *Listeria monocytogenes, Vibrio cholerae,* or *Escherichia coli.*

Embodiment A21: The modified bacterium of any one of embodiments A17-A20, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment A22: The modified bacterium of any one of embodiments A17-A21, wherein the promoter that is active under acid pH condition is selected from the group consisting of sseA, ssrA, ssaB, ssaG, ssaM, and ssaR.

Embodiment A23: The modified bacterium of any one of embodiments A17-A21, wherein the promoter that is active under acid pH condition is sseA.

Embodiment A24: The modified bacterium of any one of the preceding embodiments A, wherein the unmodified starting strain is a facultative anaerobic bacterium.

Embodiment A25: The modified bacterium of any one of the preceding embodiments A, wherein the bacterium is a bacterium of *Enterobacteriaceae.*

Embodiment A26: The modified bacterium of any one of the preceding embodiments A, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment A27: The modified bacterium of any one of the preceding embodiments A, wherein the bacterium is selected from the group consisting of *Escherichia coli, Enterobacter blattae (E. blattae), Enterobacter fergusonii (E. fergusonii), Enterobacter hermannii (E. hermannii), Enterobacter vulneris (E. vulneris), Salmonella enterica (S. enterica), Salmonella bongori (S. bongori), Salmonella typhi (S. typhi), Salmonella choleraesuis (S. choleraesuis), Salmonella typhimurium (S. typhimurium), Shigella dysenteriae (S. dysenteriae), Shigella flexneri (S. flexneri), Shigella boydii (S. boydii), Shigella sonnei (S. sonnei), Klebsiella pneumoniae (K. peumoniae)*, *Klebsiella oxytoca (K. oxytoca), Yersinia pestis (Y. pestos), Yersinia enterocolitica (Y. enterocolitica), Yersinia pseudotuberculosis (Y. pseudotuberculosis), Yersinia aldouae (Y. aldouae), Yersinia bercovieri (Y. bercovieri), Yersinia frederiksenii (Y. frederiksenii), Yersinia intermedia (Y. intermedia), Yersinia kristersenii (Y. kristersenii), Yersinia mollaretti (Y. mollaretti), Yersinia rohdei (Y. rohdei), Yersinia ruckeri (Y. ruckeri), Citrobacter freundii (C. freundii), Citrobacter koseri (C. kaseri), Citrobacter braakii (C. braakii), Enterobacter aerogenes (E. aerogenes), Enterobacter cloacae (E. cloacae), Enterobacter gergoviae (E. gergoviac), Enterobacter sakazakii (E. sakazakii), Enterobacter taylorae (E. tavlorae), Enterobacter amnigenus (E. aminigenus), Enterobacter intermedius (E. intermedius), Enterobacter asburiae (E. asburiac), Enterobacter cancerogenus (E. cancerogenus), Enterobacter dissolvens (E. dissolvens), Enterobacter nimipressuralis (E. nimipressuralis), Serratia marcescens (S. marcescens), Serratia entomophila (S. entomophila), Serratia ficaria (S. ficaria), Serratia fonticola (S. fonticola), Serratia grimesii (S. grimesii), Serratia liquefaciens (S. liquefaciens), Serratia odorifera (S. odorifera), Serratia plymuthica (S. plymuthica), Serratia proteamaculans (S. proteamaculans), Serratia rubidaea (S. rubidaea), Serratia ureilytica (S. ureilytica), Proteus mirabilis (P. mirabilis), Proteus vulgaris (P. vulgaris), Proteus myxofaciens (P. myxofaciens), Proteus penneri (P. penneri), Proteus hauseri (P. hauseri)), Morganella morganii (M. morganii), Providencia alcalifaciens (P. alcalifaciens), Providencia rustigianii (P. rustigianii), Providencia stuartii (P. stuartii), Providencia rettgeri (P. rettgeri), Providencia heimbachae (P. heimbochae), Hafnia alvei (H. alvei),* and *Pantoea agglomerans (P. agglomerans).*

Embodiment A28: The modified bacterium of any one of the preceding embodiments A, wherein the bacterium is of *Salmonella typhimurium.*

Embodiment A29: The modified bacterium of embodiment A28, wherein the starting strain is *Salmonella typhimurium* SL7207.

Embodiment A30: The modified bacterium of any one of the preceding embodiments A, wherein the bacterium expresses a wild-type lipopolysaccharide (LPS).

Embodiment A31: The modified bacterium of any one of the preceding embodiments A, wherein, when administrated to a subject with a tumor, the bacterium is capable of surviving and proliferating in the tumor tissue but is rapidly cleared in normal tissue.

Embodiment A32: The modified bacterium of any one of the preceding embodiments A, wherein, when administrated to a subject with a malignant tumor, the bacterium is capable of inhibiting the growth of the malignant tumor.

Embodiment A33: The modified bacterium of any one of the preceding embodiments A, wherein, when administrated to a subject with a malignant tumor, the bacterium is capable of inducing an anti-tumor specific immune response.

Embodiment A34: The modified bacterium of any one of the preceding embodiments A, wherein, when administrated to a subject with a malignant tumor, the bacterium is capable of inducing anti-tumor immune memory.

Embodiment A35: The modified bacterium of any one of the preceding embodiments A, which does not express a wild-type flagellin.

Embodiment A36: The modified bacterium of any one of the preceding embodiments A, which is deficient in the *fliC* gene.

Embodiment A37: The modified bacterium of any one of the preceding embodiments A, wherein the survival level of the bacterium in macrophages ranges from about 50% to about 30% of the survival level of the unmodified starting strain.

Embodiment A38: The modified bacterium of any one of embodiments A1-A36, wherein the survival level of the bacterium in macrophages ranges from about 30% to about 10% of the survival level of the unmodified starting strain.

Embodiment A39: The modified bacterium of any one of embodiments A1-A36, wherein the survival level of the bacterium in macrophages ranges from about 10% to about 1% of the survival level of the unmodified starting strain.

### Embodiments B

Embodiment B1: The modified bacterium of any one of embodiments 1-13, wherein the bacterium comprise a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof.

Embodiment B2: The modified bacterium of embodiment B1, wherein the second strictly hypoxia inducible promoter is selected from pepTp, fnrSp, ysgAp, ssbpl, Hipl, BBa_I14018, BBa_R1074, Ptet-arcA, and Ptet-Fnr.

Embodiment B3: The modified bacterium of embodiment B1, wherein the second strictly hypoxia inducible promoter is ssbpl promoter.

Embodiment B4: The modified bacterium of any one of the preceding embodiments B, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment B5: The modified bacterium of embodiment B4, wherein the essential gene is selected from *dapA, dapB, dapD, dapE, argD, dapF,* and any combination thereof.

Embodiment B6: The modified bacterium of embodiment B4, wherein the essential gene is selected from *dapA* and *dapE.*

Embodiment B7: The modified bacterium of any one of the preceding embodiments B, wherein the gene involved in or regulating the endogenous anti-oxidative stress response pathway is an HtrA serine protease family gene.

Embodiment B8: The modified bacterium of any one of the preceding embodiments B, wherein the functional expression product of the gene involved in or regulating the endogenous anti-oxidative stress response pathway is an HtrA serine protease family related protein.

Embodiment B9: The modified bacterium of any one of the preceding embodiments B, wherein the bacterium is deficient in the activity of HtrA serine protease.

Embodiment B10: The modified bacterium of any one of the preceding embodiments B, wherein the gene involved in or regulating the endogenous anti-oxidative stress response pathway is *htrA.*

Embodiment B11: The modified bacterium of any one of the preceding embodiments B, wherein the bacterium is deficient in *htrA.*

Embodiment B12: The modified bacterium of any one of embodiments B1-B11, wherein the essential gene is a gene naturally occurring in the bacterial chromosome, and the native promoter of the essential gene is functionally replaced by the second strictly hypoxia inducible promoter, whereby the expression of the essential gene in the bacterium is completely under the control of the second strictly hypoxia inducible promoter.

Embodiment B13: The modified bacterium of any one of embodiments B1-B11, wherein the essential gene expression cassette is exogenous, and the essential gene naturally occurring in the bacterial chromosome is deleted or functionally inactivated, whereby the expression of the essential gene in the bacterium is completely under the control of the second strictly hypoxia inducible promoter.

Embodiment B14: The modified bacterium of embodiments B13, wherein the exogenous essential gene expression cassette is integrated into the bacterial chromosome.

Embodiment B15: The modified bacterium of embodiments B13, wherein the exogenous essential gene expression cassette is outside of the bacterial chromosome.

Embodiment B16: The modified bacterium of embodiments B15, wherein the exogenous essential gene expression cassette is present in the plasmid carried by the bacterium.

Embodiment B17: The modified bacterium of embodiments any one of the preceding embodiments B, wherein the bacteria-derived hemolysin protein is a gram-negative bacterial hemolysin protein.

Embodiment B18: The modified bacterium of embodiment B17, wherein the gene encoding the gram-negative bacterial hemolysin protein is *hlyA* or *hlyE.*

Embodiment B19: The modified bacterium of embodiment B18, wherein the *hlyA* or *hlyE* is derived from *Listeria monocytogenes, Vibrio cholerae,* or *Escherichia coli.*

Embodiment B20: The modified bacterium of any one of the preceding embodiments B, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment B21: The modified bacterium of any one of the preceding embodiments B, wherein the promoter that is active under acid pH condition is selected from the group consisting of sseA, ssrA, ssaB, ssaG, ssaM, and ssaR.

Embodiment B22: The modified bacterium of any one of the preceding embodiments B, wherein the promoter that is active under acid pH condition is sseA.

Embodiment B23: The modified bacterium of any one of the preceding embodiments B, wherein the unmodified starting strain is a facultative anaerobic bacterium.

Embodiment B24: The modified bacterium of any one of the preceding embodiments B, wherein the bacterium is a bacterium of Enterobacteriaceae.

Embodiment B25: The modified bacterium of any one of embodiments B, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment B26: The modified bacterium of any one of the preceding embodiments B, wherein the bacterium is selected from the group consisting of *Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei,* and *Pantoea agglomerans.*

Embodiment B27: The modified bacterium of any one of the preceding embodiments B, wherein the bacterium is of *Salmonella typhimurium.*

Embodiment B28: The modified bacterium of embodiment B27, wherein the starting strain is *Salmonella typhimurium* SL7207.

Embodiment B29: The modified bacterium of any one of the preceding embodiments B, wherein the bacterium expresses a wild-type lipopolysaccharide (LPS).

Embodiment B30: The modified bacterium of any one of the preceding embodiments B, wherein, when administrated to a subject with a tumor, the bacterium is capable of surviving and proliferating in the tumor tissue but is rapidly cleared in normal tissue.

Embodiment B31: The modified bacterium of any one of the preceding embodiments B, wherein, when administrated to a subject with a malignant tumor, the bacterium is capable of inhibiting the growth of the malignant tumor.

Embodiment B32: The modified bacterium of any one of the preceding embodiments B, wherein, when administrated to a subject with a malignant tumor, the bacterium is capable of inducing an anti-tumor specific immune response.

Embodiment B33: The modified bacterium of any one of the preceding embodiments B, wherein, when administrated to a subject with a malignant tumor, the bacterium is capable of inducing anti-tumor immune memory.

Embodiment B34: The modified bacterium of any one of the preceding embodiments B, which does not express a wild-type flagellin.

Embodiment B35: The modified bacterium of any one of the preceding embodiments B, which is deficient in the *fliC* gene.

Embodiment B36: The modified bacterium of any one of the preceding embodiments B, wherein the survival level of the bacterium in macrophages ranges from about 50% to about 30% of the survival level of the unmodified starting strain.

Embodiment B37: The modified bacterium of any one of embodiments B1-B35, wherein the survival level of the bacterium in macrophages ranges from about 30% to about 10% of the survival level of the unmodified starting strain.

Embodiment B38: The modified bacterium of any one of embodiments B1-B35, wherein the survival level of the bacterium in macrophages ranges from about 10% to about 1% of the survival level of the unmodified starting strain.

### Embodiments C

Embodiment B1: The modified bacterium of any one of embodiments 1-13, wherein the bacterium comprise a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium is deficient in at least one gene involved in or regulating the endogenous anti-oxidative stress response pathway or a functional expression product thereof.

Embodiment C2: The modified bacterium of embodiment C1, wherein the second strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment C3: The modified bacterium of embodiment C1, wherein the second strictly hypoxia inducible promoter is ssbpl promoter.

Embodiment C4: The modified bacterium of any one of the preceding embodiments C, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment C5: The modified bacterium of embodiment C4, wherein the essential gene is selected from *dapA, dapB, dapD, dapE, argD, dapF,* and any combination thereof.

Embodiment C6: The modified bacterium of embodiment C4, wherein the essential gene is selected from *dapA* and *dapE.*

Embodiment C7: The modified bacterium of any one of the preceding embodiments C, wherein the gene involved in or regulating the endogenous anti-oxidative stress response pathway is an HtrA serine protease family gene.

Embodiment C8: The modified bacterium of any one of the preceding embodiments C, wherein the functional expression product of the gene involved in or regulating the endogenous anti-oxidative stress response pathway is an HtrA serine protease family related protein.

Embodiment C9: The modified bacterium of any one of the preceding embodiments C, wherein the bacterium is deficient in the activity of HtrA serine protease.

Embodiment C10: The modified bacterium of any one of the preceding embodiments C, wherein the gene involved in or regulating the endogenous anti-oxidative stress response pathway is *htrA.*

Embodiment C11: The modified bacterium of any one of the preceding embodiments C, wherein the bacterium is deficient in *htrA.*

Embodiment C12: The modified bacterium of any one of embodiments C1-C11, wherein the essential gene is a gene naturally occurring in the bacterial chromosome, and the native promoter of the essential gene is functionally replaced by the second strictly hypoxia inducible promoter, whereby the expression of the essential gene in the bacterium is completely under the control of the second strictly hypoxia inducible promoter.

Embodiment C13: The modified bacterium of any one of embodiments C1-C11, wherein the essential gene expression cassette is exogenous, and the essential gene naturally occurring in the bacterial chromosome is deleted or functionally inactivated, whereby the expression of the essential gene in the bacterium is completely under the control of the second strictly hypoxia inducible promoter.

Embodiment C14: The modified bacterium of embodiment C13, wherein the exogenous essential gene expression cassette is integrated into the bacterial chromosome.

Embodiment C15: The modified bacterium of embodiment C13, wherein the exogenous essential gene expression cassette is outside of the bacterial chromosome.

Embodiment C16: The modified bacterium of embodiment C15, wherein the exogenous essential gene expression cassette is present in the plasmid carried by the bacterium.

Embodiment C17: The modified bacterium of embodiments any one of the preceding embodiments C, wherein the bacteria-derived hemolysin protein is a gram-negative bacterial hemolysin protein.

Embodiment C18: The modified bacterium of embodiment C17, wherein the gene encoding the gram-negative bacterial hemolysin protein is *hlyA* or *hlyE.*

Embodiment C19: The modified bacterium of embodiment C18, wherein the *hlyA* or *hlyE* is derived from *Listeria monocytogenes, Vibrio cholerae,* or *Escherichia coli.*

Embodiment C20: The modified bacterium of any one of the preceding embodiments C, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment C21: The modified bacterium of any one of the preceding embodiments C, wherein the promoter that is active under acid pH condition is selected from the group consisting of sseA, ssrA, ssaB, ssaG, ssaM, and ssaR.

Embodiment C22: The modified bacterium of any one of the preceding embodiments C, wherein the promoter that is active under acid pH condition is sseA.

Embodiment C23: The modified bacterium of any one of the preceding embodiments C, wherein the unmodified starting strain is a facultative anaerobic bacterium.

Embodiment C24: The modified bacterium of any one of the preceding embodiments C, wherein the starting strain is *Salmonella typhimurium* SL7207.

Embodiment C25: The modified bacterium of any one of embodiments C1-C24, wherein the bacterium expresses a wild-type lipopolysaccharide (LPS).

Embodiment C26: The modified bacterium of any one of the preceding embodiments C, wherein, when administrated to a subject with a tumor, the bacterium is capable of surviving and proliferating in the tumor tissue but is rapidly cleared in normal tissue.

Embodiment C27: The modified bacterium of any one of the preceding embodiments C, wherein, when administrated to a subject with a malignant tumor, the bacterium is capable of inhibiting the growth of the malignant tumor.

Embodiment C28: The modified bacterium of any one of the preceding embodiments C, wherein, when administrated to a subject with a malignant tumor, the bacterium is capable of inducing an anti-tumor specific immune response.

Embodiment C29: The modified bacterium of any one of the preceding embodiments C, wherein, when administrated to a subject with a malignant tumor, the bacterium is capable of inducing anti-tumor immune memory.

Embodiment C30: The modified bacterium of any one of the preceding embodiments C, which does not express a wild-type flagellin.

Embodiment C31: The modified bacterium of any one of the preceding embodiments C, which is deficient in the *fliC* gene.

Embodiment C32: The modified bacterium of any one of the preceding embodiments C, wherein the survival level of the bacterium in macrophages ranges from about 50% to about 30% of the survival level of the unmodified starting strain.

Embodiment C33: The modified bacterium of any one of embodiments C1-C31, wherein the survival level of the bacterium in macrophages ranges from about 30% to about 10% of the survival level of the unmodified starting strain.

Embodiment C34: The modified bacterium of any one of embodiments C1-C31, wherein the survival level of the bacterium in macrophages ranges from about 10% to about 1% of the survival level of the unmodified starting strain.

### Embodiments D

Embodiment D1: The modified bacterium of any one of embodiments 1-13, wherein the bacterium comprise a hypoxia regulated essential gene expression cassette comprising an essential gene of the bacterium under the control of a second strictly hypoxia inducible promoter, and the bacterium is deficient in at least one gene required for survival in macrophages or a functional expression product thereof.

Embodiment D2: The modified bacterium of embodiment D1, wherein the second strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment D3: The modified bacterium of embodiment D1, wherein the second strictly hypoxia inducible promoter is ssbpl promoter.

Embodiment D4: The modified bacterium of any one of the preceding embodiments D, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment D5: The modified bacterium of embodiment D4, wherein the essential gene is selected from *dapA, dapB, dapD, dapE, argD, dapF,* and any combination thereof.

Embodiment D6: The modified bacterium of embodiment D4, wherein the essential gene is selected from *dapA* and *dapE.*

Embodiment D7: The modified bacterium of any one of the preceding embodiments D, wherein the gene required for survival in macrophages is gene STM3120, STM3119, slyA, sifA, SPI-2, phoP, or htrA.

Embodiment D8: The modified bacterium of any one of the preceding embodiments D, wherein the functional expression product of the gene required for survival in macrophages is an HtrA serine protease family related protein.

Embodiment D9: The modified bacterium of any one of the preceding embodiments D, wherein the bacterium is deficient in the activity of HtrA serine protease.

Embodiment D10: The modified bacterium of any one of the preceding embodiments D, wherein the gene required for survival in macrophages is *htrA.*

Embodiment D11: The modified bacterium of any one of the preceding embodiments D, wherein the bacterium is deficient in *htrA.*

Embodiment D12: The modified bacterium of any one of embodiments D1-D11, wherein the essential gene is a gene naturally occurring in the bacterial chromosome, and the native promoter of the essential gene is functionally replaced by the second strictly hypoxia inducible promoter, whereby the expression of the essential gene in the bacterium is completely under the control of the second strictly hypoxia inducible promoter.

Embodiment D13: The modified bacterium of any one of embodiments D1-D11, wherein the essential gene expression cassette is exogenous, and the essential gene naturally occurring in the bacterial chromosome is deleted or functionally inactivated, whereby the expression of the essential gene in the bacterium is completely under the control of the second strictly hypoxia inducible promoter.

Embodiment D14: The modified bacterium of embodiment D13, wherein the exogenous essential gene expression cassette is integrated into the bacterial chromosome.

Embodiment D15: The modified bacterium of embodiment D13, wherein the exogenous essential gene expression cassette is outside of the bacterial chromosome.

Embodiment D16: The modified bacterium of embodiments D15, wherein the exogenous essential gene expression cassette is present in the plasmid carried by the bacterium.

Embodiment D17: The modified bacterium of any one of the preceding embodiments D, which further comprises a pH regulated expression cassette, wherein the pH regulated expression cassette comprises a gene encoding a bacteria-derived hemolysin protein under the control of a promoter that is active under acid pH condition.

Embodiment D18: The modified bacterium of embodiments D17, wherein the bacteria-derived hemolysin protein is a gram-negative bacterial hemolysin protein.

Embodiment D19: The modified bacterium of embodiments D18, wherein the gene encoding the gram-negative bacterial hemolysin protein is *hlyA* or *hlyE.*

Embodiment D20: The modified bacterium of embodiments D19, wherein the *hlyA* or *hlyE* is derived from *Listeria monocytogenes, Vibrio cholerae,* or *Escherichia coli.*

Embodiment D21: The modified bacterium of any one of embodiments D17-D20, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment D22: The modified bacterium of any one of embodiments D17-D21, wherein the promoter that is active under acid pH condition is selected from the group consisting of sseA, ssrA, ssaB, ssaG, ssaM, and ssaR.

Embodiment D23: The modified bacterium of any one of embodiments D17-D21, wherein the promoter that is active under acid pH condition is sseA.

Embodiment D24: The modified bacterium of any one of the preceding embodiments D, wherein the unmodified starting strain is a facultative anaerobic bacterium.

Embodiment D25: The modified bacterium of any one of the preceding embodiments D, wherein the bacterium is a bacterium of *Enterobacteriaceae.*

Embodiment D26: The modified bacterium of any one of the preceding embodiments D, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment D27: The modified bacterium of any one of the preceding embodiments D, wherein the bacterium is selected from the group consisting of *Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei,* and *Pantoea agglomerans.*

Embodiment D28: The modified bacterium of any one of the preceding embodiments D, wherein the bacterium is of *Salmonella typhimurium.*

Embodiment D29: The modified bacterium of embodiment D28, wherein the starting strain is *Salmonella typhimurium* SL7207.

Embodiment D30: The modified bacterium of any one of the preceding embodiments D, wherein the bacterium expresses a wild-type lipopolysaccharide (LPS).

Embodiment D31: The modified bacterium of any one of the preceding embodiments D, wherein, when administrated to a subject with a tumor, the bacterium is capable of surviving and proliferating in the tumor tissue but is rapidly cleared in normal tissue.

Embodiment D32: The modified bacterium of any one of the preceding embodiments D, wherein, when administrated to a subject with a malignant tumor, the bacterium is capable of inhibiting the growth of the malignant tumor.

Embodiment D33: The modified bacterium of any one of the preceding embodiments D, wherein, when administrated to a subject with a malignant tumor, the bacterium is capable of inducing an anti-tumor specific immune response.

Embodiment D34: The modified bacterium of any one of the preceding embodiments D, wherein, when administrated to a subject with a malignant tumor, the bacterium is capable of inducing anti-tumor immune memory.

Embodiment D35: The modified bacterium of any one of the preceding embodiments D, which does not express a wild-type flagellin.

Embodiment D36: The modified bacterium of any one of the preceding embodiments D, which is deficient in *the fliC* gene.

Embodiment D37: The modified bacterium of any one of the preceding embodiments D, wherein the survival level of the bacterium in macrophages ranges from about 50% to about 30% of the survival level of the unmodified starting strain.

Embodiment D38: The modified bacterium of any one of embodiments D1-D36, wherein the survival level of the bacterium in macrophages ranges from about 30% to about 10% of the survival level of the unmodified starting strain.

Embodiment D39: The modified bacterium of any one of embodiments D1-D36, wherein the survival level of the bacterium in macrophages ranges from about 10% to about 1% of the survival level of the unmodified starting strain.

### Embodiments E

Embodiment E1: The modified bacterium of any one of embodiments 1-13, wherein the bacterium comprise a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of a promoter that is active under acid pH condition, and the bacterium expresses a wild-type lipopolysaccharide (LPS) and is deficient in at least one gene required for survival in macrophages or a functional expression product thereof.

Embodiment E2: The modified bacterium of embodiment E1, wherein the second strictly hypoxia inducible promoter is selected from pepTp, fnrSp, ysgAp, ssbpl, Hipl, BBa_I14018, BBa_R1074, Ptet-arcA, and Ptet-Fnr.

Embodiment E3: The modified bacterium of embodiment E1, wherein the second strictly hypoxia inducible promoter is ssbpl promoter.

Embodiment E4: The modified bacterium of any one of the preceding embodiments E, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment E5: The modified bacterium of embodiment E4, wherein the essential gene is selected from *dapA, dapB, dapD, dapE, argD, dapF,* and any combination thereof.

Embodiment E6: The modified bacterium of embodiment E4, wherein the essential gene is selected from *dapA* and *dapE.*

Embodiment E7: The modified bacterium of any one of the preceding embodiments E, wherein the gene required for survival in macrophages is gene STM3120, STM3119, slyA, sifA, SPI-2, phoP, or htrA.

Embodiment E8: The modified bacterium of any one of the preceding embodiments E, wherein the functional expression product of the gene required for survival in macrophages is an HtrA serine protease family related protein.

Embodiment E9: The modified bacterium of any one of the preceding embodiments E, wherein the bacterium is deficient in the activity of HtrA serine protease.

Embodiment E10: The modified bacterium of any one of the preceding embodiments E, wherein the gene required for survival in macrophages is *htrA.*

Embodiment E11: The modified bacterium of any one of the preceding embodiments E, wherein the bacterium is deficient in *htrA.*

Embodiment E12: The modified bacterium of any one of embodiments E1-E11, wherein the essential gene is a gene naturally occurring in the bacterial chromosome, and the native promoter of the essential gene is functionally replaced by the second strictly hypoxia inducible promoter, whereby the expression of the essential gene in the bacterium is completely under the control of the second strictly hypoxia inducible promoter.

Embodiment E13: The modified bacterium of any one of embodiments E1-E11, wherein the essential gene expression cassette is exogenous, and the essential gene naturally occurring in the bacterial chromosome is deleted or functionally inactivated, whereby the expression of the essential gene in the bacterium is completely under the control of the second strictly hypoxia inducible promoter.

Embodiment E14: The modified bacterium of embodiment E13, wherein the exogenous essential gene expression cassette is integrated into the bacterial chromosome.

Embodiment E15: The modified bacterium of embodiment E13, wherein the exogenous essential gene expression cassette is outside of the bacterial chromosome.

Embodiment E16: The modified bacterium of embodiments E15, wherein the exogenous essential gene expression cassette is present in the plasmid carried by the bacterium.

Embodiment E17: The modified bacterium of any one of the preceding embodiments E, wherein the bacteria-derived hemolysin protein is a gram-negative bacterial hemolysin protein.

Embodiment E18: The modified bacterium of embodiment E17, wherein the gene encoding the gram-negative bacterial hemolysin protein is *hlyA* or *hlyE.*

Embodiment E19: The modified bacterium of embodiment E18, wherein the *hlyA* or *hlyE* is derived from *Listeria monocytogenes, Vibrio cholerae,* or *Escherichia coli.*

Embodiment E20: The modified bacterium of any one of preceding embodiments E, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment E21: The modified bacterium of any one of embodiments E, wherein the promoter that is active under acid pH condition is selected from the group consisting of sseA, ssrA, ssaB, ssaG, ssaM, and ssaR.

Embodiment E22: The modified bacterium of any one of embodiments E, wherein the promoter that is active under acid pH condition is sseA.

Embodiment E23: The modified bacterium of any one of embodiments E, wherein the unmodified starting strain is a facultative anaerobic bacterium.

Embodiment E24: The modified bacterium of any one of embodiments E, wherein the bacterium is a bacterium of *Enterobacteriaceae.*

Embodiment E25: The modified bacterium of any one of embodiments E, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment E26: The modified bacterium of any one of the preceding embodiments E, wherein the bacterium is selected from the group consisting of *Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei,* and *Pantoea agglomerans.*

Embodiment E27: The modified bacterium of any one of the preceding embodiments E, wherein the bacterium is of *Salmonella typhimurium.*

Embodiment E28: The modified bacterium of embodiment E27, wherein the starting strain is *Salmonella typhimurium* SL7207.

Embodiment E29: The modified bacterium of any one of the preceding embodiments E, wherein, when administrated to a subject with a tumor, the bacterium is capable of surviving and proliferating in the tumor tissue but is rapidly cleared in normal tissue.

Embodiment E30: The modified bacterium of any one of the preceding embodiments E, wherein, when administrated to a subject with a malignant tumor, the bacterium is capable of inhibiting the growth of the malignant tumor.

Embodiment E31: The modified bacterium of any one of the preceding embodiments E, wherein, when administrated to a subject with a malignant tumor, the bacterium is capable of inducing an anti-tumor specific immune response.

Embodiment E32: The modified bacterium of any one of the preceding embodiments E, wherein, when administrated to a subject with a malignant tumor, the bacterium is capable of inducing anti-tumor immune memory.

Embodiment E33: The modified bacterium of any one of the preceding embodiments E, which does not express a wild-type flagellin.

Embodiment E34: The modified bacterium of any one of the preceding embodiments E, which is deficient in the *fliC* gene.

Embodiment E35: The modified bacterium of any one of the preceding embodiments E, wherein the survival level of the bacterium in macrophages ranges from about 50% to about 30% of the survival level of the unmodified starting strain.

Embodiment E36: The modified bacterium of any one of embodiments E1-E34, wherein the survival level of the bacterium in macrophages ranges from about 30% to about 10% of the survival level of the unmodified starting strain.

Embodiment E37: The modified bacterium of any one of embodiments E1-E34, wherein the survival level of the bacterium in macrophages ranges from about 10% to about 1% of the survival level of the unmodified starting strain.

### Embodiments F

Embodiment F1: The modified bacterium of any one of embodiments 1-13, which is *Salmonella typhimurium,* wherein the bacterium comprise a hypoxia regulated essential gene expression cassette comprising an essential gene of the bacterium under the control of a second strictly hypoxia inducible promoter and a pH regulated expression cassette comprising a gene encoding a bacteria-derived hemolysin protein under the control of an promoter that is active under acid pH condition, and the bacterium expresses a wild-type lipopolysaccharide (LPS) and is deficient in at least one gene required for survival in macrophages or a functional expression product.

Embodiment F2: The modified bacterium of embodiment F1, wherein the second strictly hypoxia inducible promoter is selected from the group consisting of pepTp, fnrSp, ysgAp, ssbp1, Hip1, BBa_I14018, BBa_R1074, Ptet-arcA, and Ptet-Fnr.

Embodiment F3: The modified bacterium of embodiment F1, wherein the second strictly hypoxia inducible promoter is ssbp1 promoter.

Embodiment F4: The modified bacterium of any one of the preceding embodiments F, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment F5: The modified bacterium of embodiment F4, wherein the essential gene is selected from *dapA, dapB, dapD, dapE, argD, dapF,* and any combination thereof.

Embodiment F6: The modified bacterium of embodiment F4, wherein the essential gene is selected from *dapA* and *dapE.*

Embodiment F7: The modified bacterium of any one of the preceding embodiments F, wherein the gene required for survival in macrophages is gene STM3120, STM3119, slyA, sifA, SPI-2, phoP, or htrA.

Embodiment F8: The modified bacterium of any one of the preceding embodiments F, wherein the gene required for survival in macrophages is the gene involved in or regulating the endogenous anti-oxidative stress response pathway.

Embodiment F9: The modified bacterium of embodiment F8, wherein the gene involved in or regulating the endogenous anti-oxidative stress response pathway is an HtrA serine protease family gene.

Embodiment F10: The modified bacterium of embodiment F9, wherein the functional expression product of the gene involved in or regulating the endogenous anti-oxidative stress response pathway is an HtrA serine protease family related protein.

Embodiment F11: The modified bacterium of embodiment F9, wherein the bacterium is deficient in the activity of HtrA serine protease.

Embodiment F12: The modified bacterium of embodiment F9, wherein the gene involved in or regulating the endogenous anti-oxidative stress response pathway is *htrA.*

Embodiment F13: The modified bacterium of embodiment F9, wherein the bacterium is deficient in *htrA.*

Embodiment F14: The modified bacterium of any one of embodiments F1-F13, wherein the essential gene is a gene naturally occurring in the bacterial chromosome, and the native promoter of the essential gene is functionally replaced by the second strictly hypoxia inducible promoter, whereby the expression of the essential gene in the bacterium is completely under the control of the second strictly hypoxia inducible promoter.

Embodiment F15: The modified bacterium of any one of embodiments F1-F13, wherein the essential gene expression cassette is exogenous, and the essential gene naturally occurring in the bacterial chromosome is deleted or functionally inactivated, whereby the expression of the essential gene in the bacterium is completely under the control of the second strictly hypoxia inducible promoter.

Embodiment F16: The modified bacterium of embodiment F15, wherein the exogenous essential gene expression cassette is integrated into the bacterial chromosome.

Embodiment F17: The modified bacterium of embodiment F15, wherein the exogenous essential gene expression cassette is outside of the bacterial chromosome.

Embodiment F18: The modified bacterium of embodiments F17, wherein the exogenous essential gene expression cassette is present in the plasmid carried by the bacterium.

Embodiment F19: The modified bacterium of any one of the preceding embodiments F, wherein the bacteria-derived hemolysin protein is a gram-negative bacterial hemolysin protein.

Embodiment F20: The modified bacterium of embodiment F19, wherein the gene encoding the gram-negative bacterial hemolysin protein is *hlyA* or *hlyE.*

Embodiment F21: The modified bacterium of embodiment F20, wherein the *hlyA* or *hlyE* is derived from *Listeria monocytogenes, Vibrio cholerae,* or *Escherichia coli.*

Embodiment F22: The modified bacterium of any one of the preceding embodiments F, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment F23: The modified bacterium of any one of embodiments F, wherein the promoter that is active under acid pH condition is selected from the group consisting of sseA, ssrA, ssaB, ssaG, ssaM, and ssaR.

Embodiment F24: The modified bacterium of any one of the preceding embodiments F, wherein the promoter that is active under acid pH condition is sseA.

Embodiment F25: The modified bacterium of any one of the preceding embodiments F, wherein the starting strain is *Salmonella typhimurium* SL7207.

Embodiment F26: The modified bacterium of any one of the preceding embodiments F, wherein, when administrated to a subject with a tumor, the bacterium is capable of surviving and proliferating in the tumor tissue but is rapidly cleared in normal tissue.

Embodiment F27: The modified bacterium of any one of the preceding embodiments F, wherein, when administrated to a subject with a malignant tumor, the bacterium is capable of inhibiting the growth of the malignant tumor.

Embodiment F28: The modified bacterium of any one of the preceding embodiments F, wherein, when administrated to a subject with a malignant tumor, the bacterium is capable of inducing an anti-tumor specific immune response.

Embodiment F29: The modified bacterium of any one of the preceding embodiments F, wherein, when administrated to a subject with a malignant tumor, the bacterium is capable of inducing anti-tumor immune memory.

Embodiment F30: The modified bacterium of any one of the preceding embodiments F, which does not express a wild-type flagellin.

Embodiment F31: The modified bacterium of any one of the preceding embodiments F, which is deficient in *the fliC* gene.

Embodiment F32: The modified bacterium of any one of the preceding embodiments F, wherein the survival level of the bacterium in macrophages ranges from about 50% to about 30% of the survival level of the unmodified starting strain.

Embodiment F33: The modified bacterium of any one of embodiments F1-F31, wherein the survival level of the bacterium in macrophages ranges from about 30% to about 10% of the survival level of the unmodified starting strain.

Embodiment F34: The modified bacterium of any one of embodiments F1-F31, wherein the survival level of the bacterium in macrophages ranges from about 10% to about 1% of the survival level of the unmodified starting strain.

### Embodiments AA

Embodiment AA1: A modified bacterium, wherein the bacterium comprises a hypoxia regulated essential gene expression cassette comprising an essential gene of the bacterium under the control of a strictly hypoxia inducible promoter, the bacterium is deficient in at least one non-essential gene involved in the biosynthesis or transport of LPS and/or a gene encoding a mureinlipoprotein or an expression product thereof, and the bacterium is deficient in at least one gene involved in or regulating an endogenous anti-oxidative stress response pathway or a functional expression product thereof, as compared to an unmodified starting strain, wherein the deficient gene involved in the biosynthesis or transport of LPS is a non-essential gene.

Embodiment AA2: The modified bacterium of embodiment AA1, wherein the gene involved in the biosynthesis of LPS is selected from a gene involved in the synthesis of O-antigen, a gene involved in the synthesis of core region, and a gene involved in the synthesis of lipid A.

Embodiment AA3: The modified bacterium of embodiment AA2, wherein the gene involved in the synthesis of O-antigen is selected from the group consisting of genes wzzB, gtrBb, gtrBa, and rfbP/wbaP, the gene involved in the synthesis of core regionis selected from the group consisting of genes rfaB, yibD, rfaI, rfaJ, rfaK, rfaL, rfaP, rfaQ, rfaZ, yijP, gmhA, yaeD, rfaE, and rfaD, and the gene involved in the synthesis of lipid A is selected from the group consisting of genes lpxA, lpxC, lpxD, lpxH, msbB/lpxM, lpxO, lpxP, arnT, yjdB, kdsB, yrbI, and yrbH.

Embodiment AA4: The modified bacterium of embodiment AA1, wherein the gene involved in the transport of LPS is selected from the group consisting of genes msbA, Wzy, lptA, lptB, and lptG.

Embodiment AA5: The modified bacterium of embodiment AA1, wherein the bacterium does not express wild-type lipopolysaccharide (LPS). In one embodiment, the bacterium does not express LPS or express mutant or structurally incomplete LPS.

Embodiment AA6: The modified bacterium of any of the preceding embodiments AA, wherein the strictly hypoxia inducible promoter is selected from the group consisting of pepTp, fnrSp, ysgAp, ssbpl, Hipl, BBa_I14018, BBa_R1074, Ptet-arcA, and Ptet-Fnr.

Embodiment AA7: The modified bacterium of any of the preceding embodiments AA, wherein the strictly hypoxia inducible promoter is ssbpl promoter.

Embodiment AA8: The modified bacterium of any one of the preceding embodiments AA, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment AA9: The modified bacterium of embodiment AA8, wherein the essential gene is selected from *dapA, dapB, dapD, dapE, argD, dapF,* and any combination thereof.

Embodiment AA10: The modified bacterium of embodiment AA8, wherein the essential gene is selected from *dapA* and *dapE.*

Embodiment AA11: The modified bacterium of any one of the preceding embodiments AA, wherein the gene involved in or regulating the endogenous anti-oxidative stress response pathway is an HtrA serine protease family gene.

Embodiment AA12: The modified bacterium of any one of the preceding embodiments AA, wherein the functional expression product of the gene involved in or regulating the endogenous anti-oxidative stress response pathway is an HtrA serine protease family related protein.

Embodiment AA13: The modified bacterium of any one of the preceding embodiments AA, wherein the bacterium is deficient in the activity of HtrA serine protease.

Embodiment AA14: The modified bacterium of any one of the preceding embodiments AA, wherein the gene involved in or regulating the endogenous anti-oxidative stress response pathway is *htrA.*

Embodiment AA15: The modified bacterium of any one of the preceding embodiments AA, wherein the bacterium is deficient in *htrA.*

Embodiment AA16: The modified bacterium of any one of embodiments AA1-AA15, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment AA17: The modified bacterium of any one of embodiments AA1-AA15, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment AA18: The modified bacterium of embodiment AA17, wherein the exogenous essential gene expression cassette is integrated into the bacterial chromosome.

Embodiment AA19: The modified bacterium of embodiment AA17, wherein the exogenous essential gene expression cassette is outside of the bacterial chromosome.

Embodiment AA20: The modified bacterium of embodiments AA19, wherein the exogenous essential gene expression cassette is present in the plasmid carried by the bacterium.

Embodiment AA21: The modified bacterium of any one of the preceding embodiments AA, which further comprises a pH regulated expression cassette, wherein the pH regulated expression cassette comprises a gene encoding a bacteria-derived hemolysin protein under the control of a promoter that is active under acid pH condition.

Embodiment AA22: The modified bacterium of embodiments AA21, wherein the bacteria-derived hemolysin protein is a gram-negative bacterial hemolysin protein.

Embodiment AA23: The modified bacterium of embodiment AA22, wherein the gene encoding the gram-negative bacterial hemolysin protein is *hlyA* or *hlyE.*

Embodiment AA24: The modified bacterium of embodiment AA23, wherein the *hlyA* or *hlyE* is derived from Listeria monocytogenes, Vibrio cholerae, or Escherichia coli.

Embodiment AA25: The modified bacterium of any one of embodiments AA21-AA24, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment AA26: The modified bacterium of any one of embodiments AA21-AA25, wherein the promoter that is active under acid pH condition is selected from the group consisting of sseA, ssrA, ssaB, ssaG, ssaM, and ssaR.

Embodiment AA27: The modified bacterium of any one of embodiments AA21-AA25, wherein the promoter active under acidic pH condition is sseA.

Embodiment AA28: The modified bacterium of any one of embodiments AA, wherein the unmodified starting strain is a facultative anaerobic bacterium.

Embodiment AA29: The modified bacterium of any one of the preceding embodiments AA, wherein the bacterium is a bacterium of *Enterobacteriaceae.*

Embodiment AA30: The modified bacterium of any one of the preceding embodiments AA, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment AA31: The modified bacterium of any one of the preceding embodiments AA, wherein the bacterium is selected from the group consisting of *Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei,* and *Pantoea agglomerans.*

Embodiment AA32: The modified bacterium of any one of the preceding embodiments AA, wherein the bacterium is of *Salmonella typhimurium.*

Embodiment AA33: The modified bacterium of embodiment AA32, wherein the starting strain is *Salmonella typhimurium* SL7207.

Embodiment AA34: The modified bacterium of any one of the preceding embodiments AA, wherein, when administrated to a subject with a tumor, the bacterium is capable of surviving and proliferating in the tumor tissue but is rapidly cleared in normal tissue.

Embodiment AA35: The modified bacterium of any one of the preceding embodiments AA, wherein, when administrated to a subject with a malignant tumor, the bacterium is capable of inhibiting the growth of the malignant tumor.

Embodiment AA36: The modified bacterium of any one of the preceding embodiments AA, wherein, when administrated to a subject with a malignant tumor, the bacterium is capable of inducing an anti-tumor specific immune response.

Embodiment AA37: The modified bacterium of any one of the preceding embodiments AA, wherein, when administrated to a subject with a malignant tumor, the bacterium is capable of inducing anti-tumor immune memory.

Embodiment AA38: The modified bacterium of any one of the preceding embodiments AA, which does not express a wild-type flagellin.

Embodiment AA39: The modified bacterium of any of the preceding embodiments AA, which is deficient in *the fliC* gene.

Embodiment AA40: The modified bacterium of any one of the preceding embodiments AA, wherein the survival level of the bacterium in macrophages ranges from about 50% to about 30% of the survival level of the unmodified starting strain.

Embodiment AA41: The modified bacterium of any one of embodiments AA1-AA39, wherein the survival level of the bacterium in macrophages ranges from about 30% to about 10% of the survival level of the unmodified starting strain.

Embodiment AA42: The modified bacterium of any one of embodiments AA1-AA39, wherein the survival level of the bacterium in macrophages ranges from about 10% to about 1% of the survival level of the unmodified starting strain.

### Embodiments BB

Embodiment BB1: A modified bacterium, wherein the bacterium comprises a hypoxia regulated essential gene expression cassette comprising an essential gene of the bacterium under the control of a strictly hypoxia inducible promoter and a pH regulated expression cassette comprising a gene encoding a bacteria-derived hemolysin protein under the control of a promoter that is active under acid pH condition, the bacterium is deficient in at least one non-essential gene involved in the biosynthesis or transport of LPS and/or a gene encoding a mureinlipoprotein or an expression product thereof, and the bacterium is deficient in at least one gene involved in or regulating an endogenous anti-oxidative stress response pathway or a functional expression product thereof, as compared to an unmodified starting strain.

Embodiment BB2: The modified bacterium of embodiment BB1, wherein the gene involved in the biosynthesis of LPS is selected from a gene involved in the synthesis of O-antigen, a gene involved in the synthesis of core region, and a gene involved in the synthesis of lipid A.

Embodiment BB3: The modified bacterium of embodiment BB2, wherein the gene involved in the synthesis of O-antigen is selected from the group consisting of genes wzzB, gtrBb, gtrBa, and rfbP/wbaP, the gene involved in the synthesis of core regionis selected from the group consisting of genes rfaB, yibD, rfaI, rfaJ, rfaK, rfaL, rfaP, rfaQ, rfaZ, yijP, gmhA, yaeD, rfaE, and rfaD, and the gene involved in the synthesis of lipid A is selected from the group consisting of genes lpxA, lpxC, lpxD, lpxH, msbB/lpxM, lpxO, lpxP, arnT, yjdB, kdsB, yrbI, and yrbH.

Embodiment BB4: The modified bacterium of embodiment BB1, wherein the gene involved in the transport of LPS is selected from the group consisting of genes msbA, Wzy, lptA, lptB, and lptG.

Embodiment BB5: The modified bacterium of embodiment BB1, wherein the bacterium does not express wild-type lipopolysaccharide (LPS). In one embodiment, the bacteria do not express LPS or express mutant or structurally incomplete LPS.

Embodiment BB6: The modified bacterium of any of the preceding embodiments BB, wherein the strictly hypoxia inducible promoter is selected from the group consisting of pepTp, fnrSp, ysgAp, ssbpl, Hipl, BBa_I14018, BBa_R1074, Ptet-arcA, and Ptet-Fnr.

Embodiment BB7: The modified bacterium of any of the preceding embodiments BB, wherein the strictly hypoxia inducible promoter is the ssbp1 promoter.

Embodiment BB8: The modified bacterium of any one of the preceding embodiments BB, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment BB9: The modified bacterium of embodiment BB8, wherein the essential gene is selected from *dapA, dapB, dapD, dapE, argD, dapF,* and any combination thereof.

Embodiment BB10: The modified bacterium of embodiment BB8, wherein the essential gene is selected from *dapA* and *dapE.*

Embodiment BB11: The modified bacterium of any one of the preceding embodiments BB, wherein the gene involved in or regulating the endogenous anti-oxidative stress response pathway is an HtrA serine protease family gene.

Embodiment BB12: The modified bacterium of any one of the preceding embodiments BB, wherein the functional expression product of the gene involved in or regulating the endogenous anti-oxidative stress response pathway is an HtrA serine protease family related protein.

Embodiment BB13: The modified bacterium of any one of the preceding embodiments BB, wherein the bacterium is deficient in the activity of HtrA serine protease.

Embodiment BB14: The modified bacterium of any one of the preceding embodiments BB, wherein the gene involved in or regulating the endogenous anti-oxidative stress response pathway is *htrA.*

Embodiment BB15: The modified bacterium of any one of the preceding embodiments BB, wherein the bacterium is deficient in *htrA.*

Embodiment BB16: The modified bacterium of any one of embodiments BB1-BB15, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment BB17: The modified bacterium of any one of embodiments BB1-BB15, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment BB18: The modified bacterium of embodiment BB13, wherein the exogenous essential gene expression cassette is integrated into the bacterial chromosome.

Embodiment BB19: The modified bacterium of embodiment BB13, wherein the exogenous essential gene expression cassette is outside of the bacterial chromosome.

Embodiment BB20: The modified bacterium of embodiments BB15, wherein the exogenous essential gene expression cassette is present in the plasmid carried by the bacterium.

Embodiment BB21: The modified bacterium of any one of the preceding embodiments BB, wherein the bacteria-derived hemolysin protein is a gram-negative bacterial hemolysin protein.

Embodiment BB22: The modified bacterium of embodiment BB21, wherein the gene encoding the gram-negative bacterial hemolysin protein is *hlyA* or *hlyE.*

Embodiment BB23: The modified bacterium of embodiment BB22, wherein the *hlyA* or *hlyE* is derived from *Listeria monocytogenes, Vibrio cholerae,* or *Escherichia coli.*

Embodiment BB24: The modified bacterium of any one of preceding embodiments BB, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment BB25: The modified bacterium of any one of embodiments BB, wherein the promoter that is active under acid pH condition is selected from the group consisting of sseA, ssrA, ssaB, ssaG, ssaM, and ssaR.

Embodiment BB26: The modified bacterium of any one of embodiments BB, wherein the promoter that is active under acid pH condition is sseA.

Embodiment BB27: The modified bacterium of any one of embodiments BB, wherein the unmodified starting strain is a facultative anaerobic bacterium.

Embodiment BB28: The modified bacterium of any one of the preceding embodiments BB, wherein the bacterium is a bacterium of *Enterobacteriaceae.*

Embodiment BB29: The modified bacterium of any one of the preceding embodiments BB, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment BB20: The modified bacterium of any one of the preceding embodiments BB, wherein the bacterium is selected from the group consisting of *Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei,* and *Pantoea agglomerans.*

Embodiment BB31: The modified bacterium of any one of the preceding embodiments BB, wherein the bacterium is of *Salmonella typhimurium.*

Embodiment BB32: The modified bacterium of embodiment BB31, wherein the starting strain is *Salmonella typhimurium* SL7207.

Embodiment BB33: The modified bacterium of any one of the preceding embodiments BB, wherein, when administrated to a subject with a tumor, the bacterium is capable of surviving and proliferating in the tumor tissue but is rapidly cleared in normal tissue.

Embodiment BB34: The modified bacterium of any one of the preceding embodiments BB, wherein, when administrated to a subject with a malignant tumor, the bacterium is capable of inhibiting the growth of the malignant tumor.

Embodiment BB35: The modified bacterium of any one of the preceding embodiments BB, wherein, when administrated to a subject with a malignant tumor, the bacterium is capable of inducing an anti-tumor specific immune response.

Embodiment BB36: The modified bacterium of any one of the preceding embodiments BB, wherein, when administrated to a subject with a malignant tumor, the bacterium is capable of inducing anti-tumor immune memory.

Embodiment BB37: The modified bacterium of any one of the preceding embodiments BB, which does not express a wild-type flagellin.

Embodiment BB38: The modified bacterium of any of the preceding embodiments BB, which is deficient in the *fliC* gene.

Embodiment BB39: The modified bacterium of any one of the preceding embodiments BB, wherein the survival level of the bacterium in macrophages ranges from about 50% to about 30% of the survival level of the unmodified starting strain.

Embodiment BB40: The modified bacterium of any one of embodiments BB1-BB38, wherein the survival level of the bacterium in macrophages ranges from about 30% to about 10% of the survival level of the unmodified starting strain.

Embodiment BB41: The modified bacterium of any one of embodiments BB1-BB38, wherein the survival level of the bacterium in macrophages ranges from about 10% to about 1% of the survival level of the unmodified starting strain.

### Embodiments CC

Embodiment CC1: A modified *Salmonella typhimurium* bacterium, wherein the bacterium comprises a hypoxia regulated essential gene expression cassette comprising an essential gene of the bacterium under the control of a strictly hypoxia inducible promoter and a pH regulated expression cassette comprising a bacteria-derived hemolysin protein-encoding gene under the control of a promoter that is active under acid pH condition, the bacterium is deficient in at least one non-essential gene involved in the biosynthesis or transport of LPS and/or a gene encoding mureinlipoprotein or an expression product thereof, and the bacterium is deficient in at least one gene involved in or regulating an endogenous anti-oxidative stress response pathway or a functional expression product thereof, as compared to an unmodified starting strain.

Embodiment CC2: The modified bacterium of embodiment CC1, wherein the gene involved in the biosynthesis of LPS are selected from a gene involved in the synthesis of O-antigen, a gene involved in the synthesis of core region, and a gene involved in the synthesis of lipid A.

Embodiment CC3: The modified bacterium of embodiment CC2, wherein the gene involved in the synthesis of O-antigen is selected from the group consisting of genes wzzB, gtrBb, gtrBa, and rfbP/wbaP, the gene involved in the synthesis of core regionis selected from the group consisting of genes rfaB, yibD, rfaI, rfaJ, rfaK, rfaL, rfaP, rfaQ, rfaZ, yijP, gmhA, yaeD, rfaE, and rfaD, and the gene involved in the synthesis of lipid A is selected from the group consisting of genes lpxA, lpxC, lpxD, lpxH, msbB/lpxM, lpxO, lpxP, arnT, yjdB, kdsB, yrbI, and yrbH.

Embodiment CC4: The modified bacterium of embodiment CC1, wherein the gene involved in the transport of LPS is selected from the group consisting of genes msbA, Wzy, lptA, lptB, and lptG.

Embodiment CC5: The modified bacterium of embodiment CC1, wherein the bacterium does not express wild-type lipopolysaccharide (LPS). In one embodiment, the bacteria do not express LPS or express mutant or structurally incomplete LPS

Embodiment CC6: The modified bacterium of any of the preceding embodiments CC, wherein the strictly hypoxia inducible promoter is selected from the group consisting of pepTp, fnrSp, ysgAp, ssbpl, Hip1, BBa _ I14018, BBa _ R1074, Ptet-arcA, and Ptet-Fnr.

Embodiment CC7: The modified bacterium of any one of the preceding embodiments CC, wherein the strictly hypoxia inducible promoter is the ssbpl promoter.

Embodiment CC8: The modified bacterium of any one of the preceding embodiments CC, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment CC9: The modified bacterium of embodiment CC8, wherein the essential gene is selected from *dapA, dapB, dapD, dapE, argD, dapF,* and any combination thereof.

Embodiment CC10: The modified bacterium of embodiment CC8, wherein the essential gene is selected from *dapA* and *dapE.*

Embodiment CC11: The modified bacterium of any one of the preceding embodiments CC, wherein the gene involved in or regulating the endogenous anti-oxidative stress response pathway is an HtrA serine protease family gene.

Embodiment CC12: The modified bacterium of any one of the preceding embodiments CC, wherein the functional expression product of the gene involved in or regulating the endogenous anti-oxidative stress response pathway is an HtrA serine protease family related protein.

Embodiment CC13: The modified bacterium of any one of the preceding embodiments CC, wherein the bacterium is deficient in the activity of HtrA serine protease.

Embodiment CC14: The modified bacterium of any one of the preceding embodiments CC, wherein the gene involved in or regulating the endogenous anti-oxidative stress response pathway is *htrA.*

Embodiment CC15: The modified bacterium of any one of the preceding embodiments CC, wherein the bacterium is deficient in *htrA.*

Embodiment CC16: The modified bacterium of any one of embodiments CC1-CC15, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment CC17: The modified bacterium of any one of embodiments CC1-CC15, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment CC18: The modified bacterium of embodiment CC17, wherein the exogenous essential gene expression cassette is integrated into the bacterial chromosome.

Embodiment CC19: The modified bacterium of embodiment CC17, wherein the exogenous essential gene expression cassette is outside of the bacterial chromosome.

Embodiment CC20: The modified bacterium of embodiments CC19, wherein the exogenous essential gene expression cassette is present in the plasmid carried by the bacterium.

Embodiment CC21: The modified bacterium of any one of the preceding embodiments CC, wherein the bacteria-derived hemolysin protein is a gram-negative bacterial hemolysin protein.

Embodiment CC22: The modified bacterium of embodiment CC21, wherein the gene encoding the gram-negative bacterial hemolysin protein is *hlyA* or *hlyE.*

Embodiment CC23: The modified bacterium of embodiment CC22, wherein the *hlyA* or *hlyE* is derived from *Listeria monocytogenes, Vibrio cholerae,* or *Escherichia coli.*

Embodiment CC24: The modified bacterium of any one of preceding embodiments CC, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment CC25: The modified bacterium of any one of embodiments CC wherein the promoter that is active under acid pH condition is selected from the group consisting of sseA, ssrA, ssaB, ssaG, ssaM, and ssaR.

Embodiment CC26: The modified bacterium of any one of embodiments CC, wherein the promoter that is active under acid pH condition is sseA.

Embodiment CC27: The modified bacterium of any one of the preceding embodiments CC, wherein the unmodified starting strain is a facultative anaerobic bacterium.

Embodiment CC28: The modified bacterium of any one of the preceding embodiments CC, wherein the starting strain is *Salmonella typhimurium* SL7207.

Embodiment CC29: The modified bacterium of any one of the preceding embodiments CC, wherein, when administrated to a subject with a tumor, the bacterium is capable of surviving and proliferating in the tumor tissue but is rapidly cleared in normal tissue.

Embodiment CC30: The modified bacterium of any one of the preceding embodiments CC, wherein, when administrated to a subject with a malignant tumor, the bacterium is capable of inhibiting the growth of the malignant tumor.

Embodiment CC31: The modified bacterium of any one of the preceding embodiments CC, wherein, when administrated to a subject with a malignant tumor, the bacterium is capable of inducing an anti-tumor specific immune response.

Embodiment CC32: The modified bacterium of any one of the preceding embodiments CC, wherein, when administrated to a subject with a malignant tumor, the bacterium is capable of inducing anti-tumor immune memory.

Embodiment CC33: The modified bacterium of any one of the preceding embodiments CC, which does not express a wild-type flagellin.

Embodiment CC34: The modified bacterium of any one of the preceding embodiments CC, which is deficient in the *fliC* gene.

Embodiment CC35: The modified bacterium of any one of the preceding embodiments CC, wherein the survival level of the bacterium in macrophages ranges from about 50% to about 30% of the survival level of the unmodified starting strain.

Embodiment CC36: The modified bacterium of any one of embodiments CC1-CC34, wherein the survival level of the bacterium in macrophages ranges from about 30% to about 10% of the survival level of the unmodified starting strain.

Embodiment CC37: The modified bacterium of any one of embodiments CC1-CC34, wherein the survival level of the bacterium in macrophages ranges from about 10% to about 1% of the survival level of the unmodified starting strain.

### Embodiments DD

Embodiment DD1: A modified bacterium, wherein the bacterium comprises a hypoxia regulated essential gene expression cassette comprising essential genes of the bacterium under the control of a strictly hypoxia inducible promoter, the bacterium is deficient in at least one non-essential gene involved in the biosynthesis or transport of LPS and/or a gene encoding mureinlipoprotein or an expression product thereof, and the bacterium is deficient in at least one gene required for survival in macrophages or a functional expression product thereof, as compared to an unmodified starting strain.

Embodiment DD2: The modified bacterium of embodiment DD1, wherein the gene involved in the biosynthesis of LPS is selected from the group consisting of a gene involved in the synthesis of O-antigen, a gene involved in the synthesis of core region, and a gene involved in the synthesis of lipid A.

Embodiment DD3: The modified bacterium of embodiment DD2, wherein the gene involved in the synthesis of O-antigen is selected from the group consisting of genes wzzB, gtrBb, gtrBa, and rfbP/wbaP, the gene involved in the synthesis of core regionis selected from the group consisting of genes rfaB, yibD, rfaI, rfaJ, rfaK, rfaL, rfaP, rfaQ, rfaZ, yijP, gmhA, yaeD, rfaE, and rfaD, and the gene involved in the synthesis of lipid A is selected from the group consisting of genes lpxA, lpxC, lpxD, lpxH, msbB/lpxM, lpxO, lpxP, arnT, yjdB, kdsB, yrbI, and yrbH.

Embodiment DD4: The modified bacterium of embodiment DD1, wherein the gene involved in the transport of LPS is selected from the group consisting of genes msbA, Wzy, lptA, lptB, and lptG.

Embodiment DD5: The modified bacterium of embodiment DD1, wherein the bacterium does not express wild-type lipopolysaccharide (LPS). In one embodiment, the bacteria do not express LPS or express mutant or structurally incomplete LPS.

Embodiment DD6: The modified bacterium of any of the preceding embodiments DD, wherein the strictly hypoxia inducible promoter is selected from the group consisting of pepTp, fnrSp, ysgAp, ssbpl, Hipl, BBa_I14018, BBa_R1074, Ptet-arcA, and Ptet-Fnr.

Embodiment DD7: The modified bacterium of any of the preceding embodiments DD, wherein the strictly hypoxia inducible promoter is the ssbp1 promoter.

Embodiment DD8: The modified bacterium of any one of the preceding embodiments DD, wherein the expression product of the essential gene is responsible for synthesis of 2,6-diaminopimelic acid (DAP) within the bacterium, and when cultured under aerobic conditions, the bacterial growth is dependent on the additional DAP or an analog thereof added to the medium.

Embodiment DD9: The modified bacterium of embodiment DD8, wherein the essential gene is selected from *dapA, dapB, dapD, dapE, argD, dapF,* and any combination thereof.

Embodiment DD10: The modified bacterium of embodiment DD8, wherein the essential gene is selected from *dapA* and *dapE.*

Embodiment DD11: The modified bacterium of any one of the preceding embodiments DD, wherein the gene required for survival in macrophages is gene STM3120, STM3119, slyA, sifA, SPI-2, phoP, or htrA.

Embodiment DD12: The modified bacterium of any one of the preceding embodiments DD, wherein the functional expression product of the gene required for survival in macrophages is an HtrA serine protease family related protein.

Embodiment DD13: The modified bacterium of any one of the preceding embodiments DD, wherein the bacterium is deficient in the activity of HtrA serine protease.

Embodiment DD14: The modified bacterium of any one of the preceding embodiments DD, wherein the gene required for survival in macrophages is *htrA.*

Embodiment DD15: The modified bacterium of any one of the preceding embodiments DD, wherein the bacterium is deficient in *htrA.*

Embodiment DD16: The modified bacterium of any one of embodiments DD1-DD15, wherein the essential gene is a gene naturally occurring in the bacterial chromosome, and the native promoter of the essential gene is functionally replaced by the strictly hypoxia inducible promoter, whereby the expression of the essential gene in the bacterium is completely under the control of the strictly hypoxia inducible promoter.

Embodiment DD17: The modified bacterium of any one of embodiments DD1-DD15, wherein the essential gene expression cassette is exogenous, and the essential gene naturally occurring in the bacterial chromosome is deleted or functionally inactivated, whereby the expression of the essential gene in the bacterium is completely under the control of the strictly hypoxia inducible promoter.

Embodiment DD18: The modified bacterium of embodiment DD17, wherein the exogenous essential gene expression cassette is integrated into the bacterial chromosome.

Embodiment DD19: The modified bacterium of embodiment DD17, wherein the exogenous essential gene expression cassette is outside of the bacterial chromosome.

Embodiment DD20: The modified bacterium of embodiments DD19, wherein the exogenous essential gene expression cassette is present in the plasmid carried by the bacterium.

Embodiment DD21: The modified bacterium of any one of the preceding embodiments DD, which further comprises a pH regulated expression cassette comprising a gene encoding a bacteria-derived hemolysin protein under the control of a promoter that is active under acid pH condition.

Embodiment DD22: The modified bacterium of embodiments DD21, wherein the bacteria-derived hemolysin protein is a gram-negative bacterial hemolysin protein.

Embodiment DD23: The modified bacterium of embodiment DD22, wherein the gene encoding the gram-negative bacterial hemolysin protein is *hlyA* or *hlyE.*

Embodiment DD24: The modified bacterium of embodiment DD23, wherein the *hlyA* or *hlyE* is derived from *Listeria monocytogenes, Vibrio cholerae,* or *Escherichia coli.*

Embodiment DD25: The modified bacterium of any one of embodiments DD21-DD24, wherein the promoter that is active under acid pH condition is active at pH less than 7.0, 6.9, 6.8, 6.7, 6.6, 6.5, 6.4, 6.3, 6.2, 6.1, 6.0, 5.9, 5.8, 5.7, 5.6, or 5.5.

Embodiment DD26: The modified bacterium of any one of embodiments DD21-DD24, wherein the promoter that is active under acid pH condition is selected from the group consisting of sseA, ssrA, ssaB, ssaG, ssaM, and ssaR.

Embodiment DD27: The modified bacterium of any one of embodiments DD21-DD24, wherein the promoter that is active under acid pH condition is sseA.

Embodiment DD28: The modified bacterium of any one of embodiments DD, wherein the unmodified starting strain is a facultative anaerobic bacterium.

Embodiment DD29: The modified bacterium of any one of the preceding embodiments DD, wherein the bacterium is a bacterium of *Enterobacteriaceae.*

Embodiment DD30: The modified bacterium of any one of the preceding embodiments DD, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

Embodiment DD31: The modified bacterium of any one of the preceding embodiments DD, wherein the bacterium is selected from the group consisting of *Escherichia coli, Enterobacter blattae, Enterobacter fergusonii, Enterobacter hermannii, Enterobacter vulneris, Salmonella enterica, Salmonella bongori, Salmonella typhi, Salmonella choleraesuis, Salmonella typhimurium, Shigella dysenteriae, Shigella flexneri, Shigella boydii, Shigella sonnei, Klebsiella pneumoniae, Klebsiella oxytoca, Yersinia pestis, Yersinia enterocolitica, Yersinia pseudotuberculosis, Yersinia aldouae, Yersinia bercovieri, Yersinia frederiksenii, Yersinia intermedia, Yersinia kristersenii, Yersinia mollaretti, Yersinia rohdei, Yersinia ruckeri, Citrobacter freundii, Citrobacter koseri, Citrobacter braakii, Enterobacter aerogenes, Enterobacter cloacae, Enterobacter gergoviae, Enterobacter sakazakii, Enterobacter taylorae, Enterobacter amnigenus, Enterobacter intermedius, Enterobacter asburiae, Enterobacter cancerogenus, Enterobacter dissolvens, Enterobacter nimipressuralis, Serratia marcescens, Serratia entomophila, Serratia ficaria, Serratia fonticola, Serratia grimesii, Serratia liquefaciens, Serratia odorifera, Serratia plymuthica, Serratia proteamaculans, Serratia rubidaea, Serratia ureilytica, Proteus mirabilis, Proteus vulgaris, Proteus myxofaciens, Proteus penneri, Proteus hauseri, Morganella morganii, Providencia alcalifaciens, Providencia rustigianii, Providencia stuartii, Providencia rettgeri, Providencia heimbachae, Hafnia alvei,* and *Pantoea agglomerans.*

Embodiment DD32: The modified bacterium of any one of the preceding embodiments D, wherein the bacterium is of *Salmonella typhimurium.*

Embodiment DD33: The modified bacterium of embodiment DD32, wherein the starting strain is *Salmonella typhimurium* SL7207.

Embodiment DD34: The modified bacterium of any one of the preceding embodiments DD, wherein, when administrated to a subject with a tumor, the bacterium is capable of surviving and proliferating in the tumor tissue but is rapidly cleared in normal tissue.

Embodiment DD35: The modified bacterium of any one of the preceding embodiments DD, wherein, when administrated to a subject with a malignant tumor, the bacterium is capable of inhibiting the growth of the malignant tumor.

Embodiment DD36: The modified bacterium of any one of the preceding embodiments DD, wherein, when administrated to a subject with a malignant tumor, the bacterium is capable of inducing an anti-tumor specific immune response.

Embodiment DD37: The modified bacterium of any one of the preceding embodiments DD, wherein, when administrated to a subject with a malignant tumor, the bacterium is capable of inducing anti-tumor immune memory.

Embodiment DD38: The modified bacterium of any one of the preceding embodiments DD, which does not express a wild-type flagellin.

Embodiment DD39: The modified bacterium of any of the preceding embodiments DD, which is deficient in *the fliC* gene.

Embodiment DD40: The modified bacterium of any one of the preceding embodiments DD, wherein the survival level of the bacterium in macrophages ranges from about 50% to about 30% of the survival level of the unmodified starting strain.

Embodiment DD41: The modified bacterium of any one of embodiments DD1-DD39, wherein the survival level of the bacterium in macrophages ranges from about 30% to about 10% of the survival level of the unmodified starting strain.

Embodiment DD42: The modified bacterium of any one of embodiments DD1-DD39, wherein the survival level of the bacterium in macrophages ranges from about 10% to about 1% of the survival level of the unmodified starting strain.

### Embodiments K

Embodiment K1: A pharmaceutical composition comprising an effective amount of the modified bacterium of any one of the preceding embodiments A, B, C, D, E, F, AA, BB, CC, and DD.

Embodiment K2: The pharmaceutical composition of embodiment K1, which is used for treating a malignant tumor.

Embodiment K3: The pharmaceutical composition of embodiment K1, which is used for inducing an anti-tumor specific immune response in a subject with a malignant tumor.

Embodiment K4: The pharmaceutical composition of embodiment K1, which is used for inducing anti-tumor immune memory in a subject with a malignant tumor.

Embodiment K5: The pharmaceutical composition of embodiment K1, which is used for preventing or treating metastasis or recurrence of a malignant tumor.

Embodiment K6: The pharmaceutical composition of embodiment K1, which is used for treating a malignant tumor that is resistant to, or has failed to, a prior anti-tumor therapy.

Embodiment K7: The pharmaceutical composition of embodiments K1-K6, wherein the modified bacterium is a live bacterium.

### Embodiments L

Embodiment L1: A method of treating a malignant tumor comprising administering to a subject with a malignant tumor an effective amount of the modified bacterium of any one of the preceding embodiments A, embodiments B, embodiments C, embodiments D, embodiments E, embodiments F, embodiments AA, embodiments BB, embodiments CC, and embodiments DD or the pharmaceutical composition of any one of embodiments K.

Embodiment L2: A method of inducing an antitumor specific immune response in a subject with a malignant tumor comprising administering to the subject an effective amount of the modified bacterium of any one of the preceding embodiments A, embodiments B, embodiments C, embodiments D, embodiments E, embodiments F, embodiments AA, embodiments BB, embodiments CC, and embodiments DD or the pharmaceutical composition of any one of embodiments K.

Embodiment L3: A method of inducing anti-tumor immune memory in a subject with a malignant tumor comprising administering to the subject an effective amount of the modified bacterium of any one of the preceding embodiments A, embodiments B, embodiments C, embodiments D, embodiments E, embodiments F, embodiments AA, embodiments BB, embodiments CC, and embodiments DD or the pharmaceutical composition of any one of embodiments K.

Embodiment L4: A method of preventing or treating metastasis or recurrence of a malignant tumor comprising administering to a subject with a malignant tumor an effective amount of the modified bacterium of any one of the preceding embodiments A, embodiments B, embodiments C, embodiments D, embodiments E, embodiments F, embodiments AA, embodiments BB, embodiments CC, and embodiments DD or the pharmaceutical composition of any one of embodiments K.

Embodiment L5: A method of preventing or treating metastasis or recurrence of a malignant tumor comprising administering to a subject with or at a high risk of metastasis or recurrence of the malignant tumor an effective amount of the modified bacterium of any one of the preceding embodiments A, embodiments B, embodiments C, embodiments D, embodiments E, embodiments F, embodiments AA, embodiments BB, embodiments CC, and embodiments DD or the pharmaceutical composition of any one of embodiments K.

Embodiment L6: A method of treating a malignant tumor that is resistant to, or has failed to, a prior anti-tumor therapy comprising administering to a subject having a malignant tumor that is resistant or fails to be treated for a prior anti-tumor treatment an effective amount of the modified bacterium of any one of the preceding embodiments A, embodiments B, embodiments C, embodiments D, embodiments E, embodiments F, embodiments AA, embodiments BB, embodiments CC, and embodiments DD or the pharmaceutical composition of any one of embodiments K.

Embodiment L7: The method of embodiments L1-L6, wherein the modified bacterium is a live bacterium.

### Embodiments M

Embodiment M1: Use of the modified bacteria of any one of the preceding embodiments A, embodiments B, embodiments C, embodiments D, embodiments E, embodiments F, embodiments AA, embodiments BB, embodiments CC, and embodiments DD in the preparation of a medicament for treating a malignant tumor.

Embodiment M2: Use of the modified bacteria of any one of the preceding embodiments A, embodiments B, embodiments C, embodiments D, embodiments E, embodiments F, embodiments AA, embodiments BB, embodiments CC, and embodiments DD in the preparation of a medicament for inducing an anti-tumor specific immune response in a subject with a malignant tumor.

Embodiment M3: Use of the modified bacteria of any of the preceding embodiments A, embodiments B, embodiments C, embodiments D, embodiments E, embodiments F, embodiments AA, embodiments BB, embodiments CC, and embodiments DD in the manufacture of a medicament for inducing anti-tumor immune memory in a subject with a malignant tumor.

Embodiment M4: Use of the modified bacteria of any one of the preceding embodiments A, embodiments B, embodiments C, embodiments D, embodiments E, embodiments F, embodiments AA, embodiments BB, embodiments CC, and embodiments DD in the preparation of a medicament for preventing or treating metastasis or recurrence of a malignant tumor.

Embodiment M5: Use of the modified bacteria of any one of the preceding embodiments A, embodiments B, embodiments C, embodiments D, embodiments E, embodiments F, embodiments AA, embodiments BB, embodiments CC, and embodiments DD in the preparation of a medicament for treating a malignant tumor that is resistant to, or has failed to, a prior anti-tumor therapy.

Embodiment M6: The use of embodiments M1-M5, wherein the modified bacterium is a live bacterium.

### Embodiments N

Embodiment N1: The pharmaceutical composition of any one of embodiments K, the method of any one of embodiments L, or the use of any one of embodiments M, wherein the malignant tumor is a malignant tumor from the nervous system, respiratory system, digestive system, urinary system, reproductive system, hematopoietic system, lymphatic system, endocrine system, or skin mucosa.

Embodiment N2: The pharmaceutical composition of any one of embodiments K, the method of any one of embodiments L, or the use of any one of embodiments M, wherein the malignant tumor is a sarcoma or cancer.

Embodiment N3: The pharmaceutical composition of any one of embodiments K, the method of any one of embodiments L, or the use of any one of embodiments M, wherein the malignant tumor is a solid tumor.

Embodiment N4: The pharmaceutical composition of any one of embodiments K, the method of any one of embodiments L, or the use of any one of embodiments M, wherein malignant tumor is selected from the group consisting of glioma, neuroblastoma, retinoblastoma, nasopharyngeal carcinoma, oral cavity carcinoma, tongue carcinoma, larynx carcinoma, head and neck carcinoma, melanoma, bronchus carcinoma, lung carcinoma, pleural carcinoma, esophagus carcinoma, gastric carcinoma, hepatocellular carcinoma, pancreatic carcinoma, cholangiocarcinoma, colorectal carcinoma, rectal carcinoma, renal cell carcinoma, bladder carcinoma, prostate carcinoma, suprarenoma, thyroid carcinoma, parathyroid gland carcinoma, Pituitary tumor, Hodgkin's disease, non-Hodgkin's lymphoma, multiple myeloma, cervical cancer, ovarian cancer, endometrial cancer, breast cancer, bone cancer, and osteosarcoma.

Embodiment N5: The pharmaceutical composition of any one of embodiments K, the method of any one of embodiments L, or the use of any one of embodiments M, wherein the bacterium, medicament, or pharmaceutical composition of the present invention is administered by intravenous, intratumoral, intramuscular, subcutaneous, intraperitoneal, transcerebral, gastrointestinal, body surface, oral mucosal, transnasal, transrectal, or transvaginal routes.

### Examples

The following Examples are used for illustrating certain specific features and/or embodiments of the invention. These Examples should not be interpreted as limiting the invention to the specific features or embodiments as described.

### Example 1: Construction of Variant Strain of Salmonella typhimurium SL7207 Strain

1.1 The target fragment was obtained by PCR amplification using the PsseA-hlyA-loxp-KnaR-loxp plasmid (SEQ ID NO: 3) and the Pssbp1- dapE-Cm plasmid (SEQ ID NO: 4) as templates. The following primer sequences were used for PsseA-hlyA-loxp-KnaR-loxp: forward primer (SEQ ID NO: 5) and reverse primer (SEQ ID NO: 6); for Pssbp1- dapE-Cm: forward primer (SEQ ID NO: 7) and reverse primer (SEQ ID NO: 8), annealing temperature 55°C. The target fragment 1 is PsseA-hlyA-loxp-KnaR-loxp (SEQ ID NO: 1), and the target fragment 2 is Pssbp1- dapE-Cm (SEQ ID NO: 2).

1.2 The pSim6 plasmid containing a lambda phage Red recombinase (BioVector NTCC Inc.: 3574840) was introduced into a facultative anaerobic Salmonella SL7207 strain (NCBI: ASM1320710v1) to prepare an electrotransformation competent cell SL7207 (pSim6) of the SL7207 strain containing the pSim6 plasmid.

1.3 The target fragment 1 was introduced into SL7207 (pSim6) competent cells by electrotransformation (E=18KV/cm). The dapE gene (which regulates synthesis of diaminopimelic acid essential for cell wall synthesis) in the SL7207 genome was replaced with desired fragment 1 using λ-red homologous recombination. Specifically, the pSim6 plasmid-containing target strain was cultured at 30 °C; the recombinase expression was conducted at 42 ° C for 15 min; the homologous recombination process was completed by adding homologous arms containing 50 bp away from each upstream and downstream of the target gene locus to the PCR primers under the effect of a homologous recombinase to obtain the strain SL7207 (ΔdapE::PsseA-hlyA-KnaR).

1.4 The Cre plasmid (Gene Bridges: A112) containing the P1 phage Cre recombinase was introduced into the strain SL7207 (ΔdapE:: PsseA-hlyA-KnaR) for recombination to remove kanamycin resistance, thereby obtaining the strain SL7207 (ΔdapE:: PsseA-hlyA).

1.5 The pSim6 plasmid was introduced into SL7207 (ΔdapE:: PsseA-hlyA) strain; SL7207 (ΔdapE:: PsseA-hlyA) (pSim6) electrotransform competent cells were prepared.

1.6 The target fragment 2 was introduced into SL7207 (ΔdapE:: PsseA-hlyA) (pSim6) competent cells by electrotransformation (E=18 KV/cm). The htrA gene (encoding a serine protease) in the SL7207 genome (ΔdapE:: PsseA-hlyA) was replaced with desired fragment 2 using λ-red homologous recombination to obtain the strain DB-ZW1, which was an SL7207 strain (ΔdapE:: PsseA-hlyA; ΔhtrA:: Pssbp1-dapE-Cm) deficient in htrA gene, containing an hlyA gene under the control of PsseA and a dapE gene under the control of a strictly hypoxia inducible promoter Pssbp1.

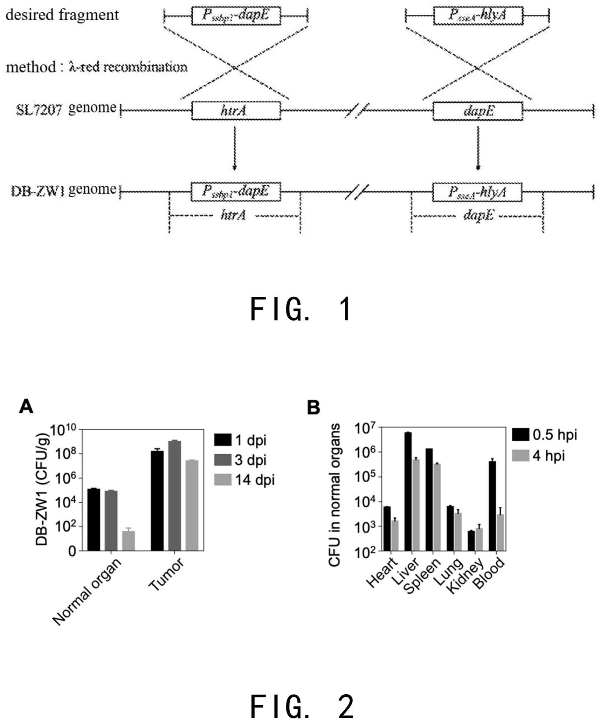

The strain construction scheme was shown in FIG.1.

### Example 2: DB-ZW1 has tumor inhibition effect on various tumor models

### 2.1 Construction of tumor models

C57BL/6 mice (purchased from Beijing Vital River Laboratory Animal Technology Co., Ltd., weighing about 18 g, kept in a SPF environment) were subcutaneously inoculated with 1 × 10 ⁶ MB49 mouse bladder cancer cells/B16 melanoma cells (ATCC: CRL-6475) to establish a mouse subcutaneous tumor model for bladder cancer/melanoma. The experiment was carried out after 14-18 days of inoculation, when the tumor volume was about 100 mm³. In situ colon cancer model was induced by DSS/AOM: 1 week after a single intraperitoneal injection of 10 mg/kg AOM, the mice were fed with water containing 2.5% DSS for 7 days, and then, with normal drinking water for 14 days, which was considered as one cycle of DSS treatment. The DSS treatment was repeated for another 3 cycles to obtain a mouse model with colorectal cancer.

### 2.2 Changes in the distribution of DB-ZW1 in tumors and different organs

1 × 10 ⁷ CFU of DB-ZW1 at a volume of 125µL was injected into tumor-bearing mice for MB49 bladder cancer via tail vein, with 3 mice in each group. Approximately 90% of DB-ZW1 in mice was observed to reside in tumors 1 day post injection (1 dpi). The density of DB-ZW1 in tumors was further elevated 100-fold (to 10 ⁸ CFU/g) within the following 2 days and maintained at a high level for the following two weeks. In contrast, the density of DB-ZW1 in normal organs steadily decreased to < 10² CFU/g over two weeks (FIG. 2A).

To further determine whether DB-ZW1 preferentially proliferated in tumors, the number of bacteria distributed in different organs was determined 24 hours after the injection of 125 µL of 1 ×10 ⁷ CFU of DB-ZW1 bacteria into tumor-bearing mice for MB49 bladder cancer via tail vein, with 3 mice in each group (FIG.2B). The results showed that DB-ZW1 spread rapidly in mice within 30 minutes after injection, and most of DB-ZW1 cells (about 99.9%) were distributed in the liver, spleen and blood. And the number of DB-ZW1 cells was significantly reduced within 4 hours after injection, and the total number of DB-ZW1 cells was reduced by about 90%. Subsequently, DB-ZW1 in tumors proliferated exponentially at a growth rate of about 0.7 h⁻¹, reaching saturation within 3 days. In contrast, in normal organs, the number of DB-ZW1 cells were all steadily reduced. This specific tumor growth profile of DB-ZW1 was key to ensuring the validity of the following findings.

### 2.3 The tumor inhibition effect of DB-ZW1 on bladder cancer, melanoma and colon cancer

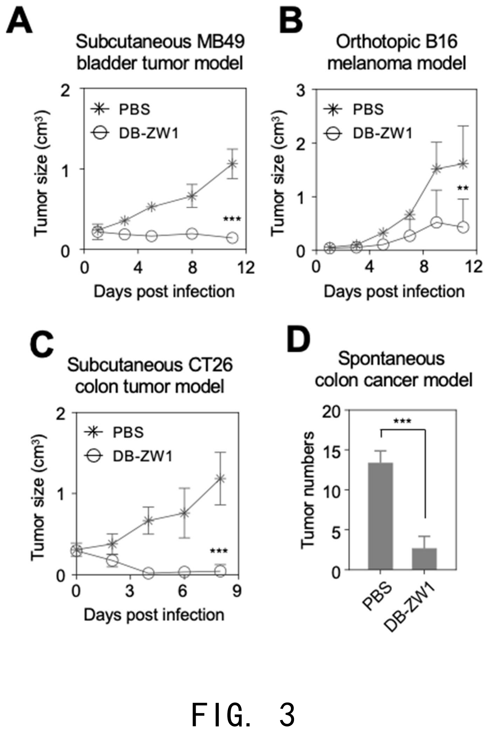

Mice with subcutaneous bladder cancer, orthotopic melanoma and subcutaneous and orthotopic colon cancer were treated with DB-ZW1, and 1×10 ⁷ CFU of DB- ZW1 at a volume of 125µL was injected into tumor-bearing mice via tail vein, with 5 mice per group. As shown in FIGs. 3A-C, DB-ZW1 significantly reduced the tumor size. In addition, the treatment of mice with orthotopic colon cancer using DB-ZW1 also significantly reduced the number of tumors in the colon of mice (FIG. 3D).

### Example 3: Construction of Variant Strains of DB-ZW1

Using pTargetF plasmid (SEQ ID NO: 22) as a template, PCR was performed using PrimeSTAR Max DNA Polymerase (Takara # R054A) and primer pair msbb-HR-Upper/msbb-HR- Down or primer pair lppAB-HR-Upper/lppAB-HR-Down (see Table 3) to obtain homologous recombination fragments 3 and 4, respectively. The PCR system (total volume of 50µL) contained 1 ×PrimeSTAR Max Premix, 0.2-0.3µM each primer, 200 ng of template and water.

The PCR condition was: denaturation: 98 ° C 10 sec, annealing: 55 °C 10 sec, extension: 72 ° C 5 sec/kb, 30 cycles of denaturation-anneal-extension in total.

The homologous recombination fragments 3 and 4 were integrated into the positions of the original msbb gene and lppAB gene in the ZW1 strain by the λ-red homologous recombination method to obtain strains ZW1(Δmsbb)-aadA and ZW1(ΔlppAB)-aadA, respectively.

Further, the corresponding strains ZW1(ΔXXX)-aadA (see Table 3, where XXX represents the corresponding gene) were obtained by a similar method, the corresponding homologous recombination fragments were obtained by amplification with different primer pairs XXX-HR-Upper/XXX-HR-Down, and homologous recombination.

**Table 3**

| Gene | Primer name | Sequence |
|---|---|---|
| msbb | msbb-HR-Upper | TCGCTACACTATTCACAATTCCTTTTCGCGTCAGCAGACCCTG GAAAAGCTCGAGTTCATGTGCAGCTC |
| | msbb-HR-Down | AGGTAGTACAGGGTTTGTCAGCATAAAGCCTCTCTTACGAGAG GCTTTATTAGATCCTTTACGCGTTCGC |
| lppAB | lppAB-HR-Upper | AATACTTGTAACGCTACATGGAGATTAACTCAATCTAGAGGGT ATTAATATCGAGTTCATGTGCAGCTC |
| | lppAB-HR-Down | CGGACAAAAAAATGGCGCACGATGTGCGCCATTTTATATTGTG CGTCAAATAGATCCTTTACGCGTTCGC |
| wzzB | wzzB-HR-Upper | TTTATGGCTACACTGTCTCCAGCTTCATCCTTTTTTTAGTTAG GGTATCTATGATTGAACAAGATGGATTGCAC |
| | wzzB-HR-Down | TTAATGAGAAATTTTACCTGTCGTAGCCGACCACCATCCGGCA AAGAAGCTCAGAAGAACTCGTCAAGAAGG |
| gtrBb | gtrBb-HR-Upper | CTTATTTGCGGATTTTTATATTCCAGATTCATTGTTTTCAGGA ATGAGAAATGATTGAACAAGATGGATTGCAC |
| | gtrBb-HR-Down | AGCCAGTAGTATCATCGCCGTTTTCGGTAATATCTTGAGCATA GTGTTCATCAGAAGAACTCGTCAAGAAGG |
| rfe | rfe-HR-Upper | GTAACGCATTATACTTTAAGGAAATGATATATGCTGAGAGCAC ATACACTATGATTGAACAAGATGGATTGCAC |
| | rfe-HR-Down | AGCGCTCACCGCGCGTGCCCCCGGTAATGGTTGTGTCATCGTA TCCTCATTCAGAAGAACTCGTCAAGAAGG |
| rfab | rfab-HR-Upper | CTATTTAATATCTAAAGTCTTTAGATTAAGGTTTTAACTCTGG GGTATATATGATTGAACAAGATGGATTGCAC |
| | rfab-HR-Down | TAAAGTCTGTTGAATGACTTCTTCTTCAAAATATTTTCTGCTC ATAGTGATCAGAAGAACTCGTCAAGAAGG |
| yibD | yibD-HR-Upper | AGAGCCACAGTATAAAGGCAGGGTAAATTAAGGTTTTTCTGGT AATCGTTATGATTGAACAAGATGGATTGCAC |
| | yibD-HR-Down | GCACCAGTGGCAAGGCTGGGGGAAAATCGCCGCGCCTAAAAAC GTGAGCGTCAGAAGAACTCGTCAAGAAGG |
| rfaI | rfal-HR-Upper | TTTAAAAATTTTAATAATGCAATATTCTCGAAATTACAAAAGT GATCACTATGATTGAACAAGATGGATTGCAC |
| | rfaI-HR-Down | TTCAGCTATTTCTATCTCAGGAAATGAATCCATTACATCACCT ATGGGTTTCAGAAGAACTCGTCAAGAAGG |
| rfaJ | rfaJ-HR-Upper | ATAGCCTACTTTAAACGTAAACTTCTTGAATAAAACCCATAGG TGATGTAATGATTGAACAAGATGGATTGCAC |
| | rfaJ-HR-Down | AGTTTTTAATCTTTTTTTCAATAATCATAATGGAGATTTAGGG AGGGGAATCAGAAGAACTCGTCAAGAAGG |
| rfaK | rfaK-HR-Upper | TTATAACATTTCTTTTTCTATAAAGTTAACTATTCTGGATTAT AGCTATTATGATTGAACAAGATGGATTGCAC |
| | rfaK-HR-Down | GCGCTGATACTTATTACGGTATCAGCGCGTTTTCCATCATCAG GACTCAATCAGAAGAACTCGTCAAGAAGG |
| rfaL | rfaL-HR-Upper | AGATTCATTAAAGAGACTCTGTCTCATCCCAAACCTATTGTGG AGAAAAGATGATTGAACAAGATGGATTGCAC |
| | rfaL-HR-Down | CCTGATGATGGAAAACGCGCTGATACCGTAATAAGTATCAGCG CGTTTTTTCAGAAGAACTCGTCAAGAAGG |
| rfaP | rfaP-HR-Upper | TTTATACAGCTTACCGGAGAAGGCCGCGGATATTATTACAGGT GATTTAGATGATTGAACAAGATGGATTGCAC |
| | rfaP-HR-Down | CTATCCTTGTCTCACTCATAAATTACTCACTGAGTGCATAATT ATTATAATCAGAAGAACTCGTCAAGAAGG |
| rfaQ | rfaQ-HR-Upper | GTGCTTTTTTCTGCTTATTTATCCGGACAATTTCAACCAAAGA GTCGCTTATGATTGAACAAGATGGATTGCAC |
| | rfaQ-HR-Down | ACGCTGCAGACCGCCAAAAGGAAAATATTTATATAAGCAAAAG GCAACTCTCAGAAGAACTCGTCAAGAAGG |
| rfaZ | rfaZ-HR-Upper | TTGAATATAACATTTATTTATAGGTTGCATGATAAACATACGA GTTTTTTATGATTGAACAAGATGGATTGCAC |
| | rfaZ-HR-Down | AAAAAAGAAATGCGTGCCGAAGCAACGAAAAATAATGATTTAT CTAGTGTTCAGAAGAACTCGTCAAGAAGG |
| yijP | yijP-HR-Upper | TAAGGTTCAGGCGTTACGTTTGGCGCTACGATTAAAGACAGGC TCTCATTATGATTGAACAAGATGGATTGCAC |
| | yijP-HR-Down | AGAAGCACGCCAGGCCCGATAAGCGTTAGCGTTATCGGGCAAC ATACAGGTCAGAAGAACTCGTCAAGAAGG |
| gmhA | gmhA-HR-Upper | ATCCGGTACACTGCATACTGTCATTTACATTCATGCTGAAGGA TATCCTCATGATTGAACAAGATGGATTGCAC |
| | gmhA-HR-Down | GTTTCTGAGCGCAACCAACGCACCTGCAGCTTGAAGTATGACG GGTATAGTCAGAAGAACTCGTCAAGAAGG |
| yaeD | yaeD-HR-Upper | CTGAAACATGCGATACTAGCGCCACTTGCTTTACTCAGGAGCT ATAAAAGATGATTGAACAAGATGGATTGCAC |
| | yaeD-HR-Down | AGCGGAAAAATGCATTTTTGTTTTAACCGCTCATCTTTTATCC ATAACGCTCAGAAGAACTCGTCAAGAAGG |
| rfaE | rfaE-HR-Upper | TGAATTATGCTATTATCGCGCGCAAATTTTGAATCTCTCAGGA GACAGGAATGATTGAACAAGATGGATTGCAC |
| | rfaE-HR-Down | CGCCTTCAAAATCAGCGCCCGCCGCGTCTGTAACGCCTGATGA CGCGGTTTCAGAAGAACTCGTCAAGAAGG |
| rfaD | rfaD-HR-Upper | GCATTCATGTCTGAGACAGTCTCTGACACCATAATTCAAAGGT TACAGTTATGATTGAACAAGATGGATTGCAC |
| | rfaD-HR-Down | CATCATCATGTCGCCCACCCAGGACGGGCCAATGACCAAAATT TTCATACTCAGAAGAACTCGTCAAGAAGG |
| ompT | ompT-HR-Upper | GAAAACTTCATCACCTCTCCAGATTACATCTGAATATGAGGAC AAGAGAAATGATTGAACAAGATGGATTGCAC |
| | ompT-HR-Down | AGCAAAAAGTCGTCATCGGCCGGTTATGACCGATGACATCCCG ATGTGGTTCAGAAGAACTCGTCAAGAAGG |
| lpxP | lpxP-HR-Down | GAATATTATGACAATGCGAAATGCCAAAATAAAAGGCCTTGCG GCCTTTTTCAGAAGAACTCGTCAAGAAGG |
| | lpxP-HR-Upper | ATAATGACTCTTCTGCGATACATTATTTTCGTATCTACAGAAG GTATTTTATGATTGAACAAGATGGATTGCAC |
| arnT | arnT-HR-Down | GAGCTGACCGCCAACGCTGAGCAGACTGGCAAGCACCAGAACG ACGCCGATCAGAAGAACTCGTCAAGAAGG |
| | arnT-HR-Upper | ATCCCTGGCCGTGAAGGTTGGCTGGGGTGTCAACAGGCAGCGA GCGCCTCATGATTGAACAAGATGGATTGCAC |
| yjdB | yjdB-HR-Upper | TAATTTTACTTTGTCACGATTAGCGTCACCGAATCGATGGACG CATCAACATGATTGAACAAGATGGATTGCAC |
| | yjdB-HR-Down | GAGTATTAACCCCTGTAATAATAGCGTGTCGTCTTCAACAATC AGTATCTTCAGAAGAACTCGTCAAGAAGG |
| yrbI | yrbI-HR-Down | TACCAGAATGGCCAGCGACAGTAGAATGATAACCCAACGTCTG GTTTTACTCAGAAGAACTCGTCAAGAAGG |
| | yrbI-HR-Upper | CATGATCTCCTGCGTGCAGGCGTCGTGTAGAAACTCAAGGATA ATTAACGATGATTGAACAAGATGGATTGCAC |
| yrbH | yrbH-HR-Down | GGTCGGAAGCGACGCACCTGCTTTACTCATCGTTAATTATCCT TGAGTTTTCAGAAGAACTCGTCAAGAAGG |
| | yrbH-HR-Upper | GATGCTCTATTGGCTATCGCCACTTCTTGTTGGATAACTGGAA ACGGATTATGATTGAACAAGATGGATTGCAC |
| lpxA | lpxA-HR-Down | GGACGTTTCACCGGCGACCAGGGCAATCGTTAAAGGACGCTGT GCCGCCATCAGAAGAACTCGTCAAGAAGG |
| | lpxA-HR-Upper | TCGTGTGCGAAGCAACCATGATGTGTGCTCGTAGCCGGGAGGC CTGATTCATGATTGAACAAGATGGATTGCAC |
| lpxC | lpxC-HR-Down | GGAGAGAGTGCCAGGTTAACCAGTCGAATTTTATACGACAGCG TATGTCGTCAGAAGAACTCGTCAAGAAGG |
| | lpxC-HR-Upper | CGAATGTATAGTACACTTCGGTTGGATAGGTAATTTGGCGAGA TTATACGATGATTGAACAAGATGGATTGCAC |
| lpxD | lpxD-HR-Down | TCGTATGAATGTGACAGGCCGGAAAAGAATGGCAACTACAAGG CGGAACGTCAGAAGAACTCGTCAAGAAGG |
| | lpxD-HR-Upper | GCGATGTGAAAGACATCACCGCTGACGTACTGAAACAGGTTAA ATAAGTAATGATTGAACAAGATGGATTGCAC |
| lpxH | lpxH-HR-Upper | GTACCAAAAGAAGACGTCATCATTGAAAACGTGACCGTCAGCG AGTAATCATGATTGAACAAGATGGATTGCAC |
| | lpxH-HR-Down | GGCAAGGAAAACGGTTGCGTGCGCGCCGTAACGGACGATTCGA CGAACATTCAGAAGAACTCGTCAAGAAGG |
| lpxO | lpxO-HR-Upper | GGTTGCCTGAAAATCCCGGGATGAGGTTTTCTTAGAAGGACAG TGACGTTATGATTGAACAAGATGGATTGCAC |
| | lpxO-HR-Down | CGCCAGGATAGAACAGGCCGCGGCAGGCAGTTCCATACCGCGG CCCGGCTTCAGAAGAACTCGTCAAGAAGG |
| kdsB | kdsB-HR-Down | TCATACCGCGCTGTTCATGACCGTCTAAGACGATCATGAACAG GATCATGTCAGAAGAACTCGTCAAGAAGG |
| | kdsB-HR-Upper | GTATTGCTGGAAAATGAAGCTCGCGCCCTGACATCTGATGAGA GTAAATCATGATTGAACAAGATGGATTGCAC |
| rfbU | rfbU-HR-Upper | CTTATGATTATTGCTTTAAAGATGCGTATGGAAAAACGGAGAG CTATTCAATGATTGAACAAGATGGATTGCAC |
| | rfbU-HR-Down | GGCCAAAGCGACCCTGCATTATATGTGGGAATAATTAATGTTA TTTTCATTCAGAAGAACTCGTCAAGAAGG |
| gtrBa | gtrBa-HR-Upper | CTGATATGCGGATTCATTTATTCAAAGTTCATTGTCTTTAGGG ATGCGAAATGATTGAACAAGATGGATTGCAC |
| | gtrBa-HR-Down | ACACCAACGAGAAAACTATAAGGATAGCGATTACCATTTTTAA TCTATTGTCAGAAGAACTCGTCAAGAAGG |
| rfbP | rfbP-HR-Upper | TAATATGCCTATTTTATTTACATTATGCACGGTCAGAGGGTGA GGATTAAATGATTGAACAAGATGGATTGCAC |
| | rfbP-HR-Down | TTTTACGCAGGCTAATTTATACAATTATTATTCAGTACTTCTC GGTAAGCTCAGAAGAACTCGTCAAGAAGG |
| msbA | msbA-HR-Down | CGGCAGCAGTAACCGCCATAGCGGGGATTCACCCGACCAGATA CGCGCAATCAGAAGAACTCGTCAAGAAGG |
| | msbA-HR-Upper | AGGATAACGGGTAGAATATGCGGCTATTTCAACAAATGCTGGT TTTTTGAATGATTGAACAAGATGGATTGCAC |
| wzy | wzy-HR-Upper | TGCCTGATGGTAATATTTTTAATACTAAGCATTTTTTCTAAAG GCTCTATATGATTGAACAAGATGGATTGCAC |
| | wzy-HR-Down | ATTTTTACGCTTCAGAGCCAAATAAAACGGCGGCATTGCCGCC GTATAACTCAGAAGAACTCGTCAAGAAGG |
| lptA | lptA-HR-Down | CCTTTATAGGCCTTCGCCAGATTCTTTGCAGTTAATGTTGCCA TAACGAATCAGAAGAACTCGTCAAGAAGG |
| | lptA-HR-Upper | TAACTTACGCAGCAAGAACGCCGAGCTGATTGAAAAGGTTAGA ACCTCTTATGATTGAACAAGATGGATTGCAC |
| lptB | lptB-HR-Down | CATATTCAGAGTCGTTTTCTCCCGCTAGAGCAGCATAGCCCTC TACCCTATCAGAAGAACTCGTCAAGAAGG |
| | lptB-HR-Upper | ACAAAAACAAAGGCCAGACTCCGGCGCAAAAGAAGAGTAACTA ATTCGTTATGATTGAACAAGATGGATTGCAC |
| lptD | lptD-HR-Upper | ACCGTTTGTCACGCGCAACGCTACCGATGATGGAACAATAAAT ACAACGTATGATTGAACAAGATGGATTGCAC |
| | lptD-HR-Down | TTTTCCATTTCAATTAACCGCAATGCGGATTACGTTGTAAATC AATCAGATCAGAAGAACTCGTCAAGAAGG |
| lptG | lptG-HR-Down | TCATGGTAGGCCTGATAAGCGCAGCGCCATCAGGCCTACGATT ACTGTGATCAGAAGAACTCGTCAAGAAGG |
| | lptG-HR-Upper | GTGCCGGTCCGCCGCCTGCGTGCCCGTTTTCTGCGTAAAGGAG CGGTATAATGATTGAACAAGATGGATTGCAC |
| | Reverse primer 1 | GGCGCGCCGAGTCTCTTC |
| | Forward primer 1 | CTCACTCAAAGGCGGTAATAC |
| rfaC | rfaC-OS-Upper | CAGCGGGTTCTGGAAGAGCTTCATTCGCTGTTGTCGGAAGAGG GCGTTTAATGATTGAACAAGATGGATTGCAC |
| | rfaC-OS-Down | GTGGTTAGCATCTTTTCTCCACAATAGGTTTGGGATGAGACAG AGTCTCTTCAGAAGAACTCGTCAAGAAGG |
| rfaF | rfaF-OS-Upper | GCCGAAGGCGTCACGGAGTATATGGCCTGGCTGAACCGCGACG CGTAAGTATGATTGAACAAGATGGATTGCAC |
| | rfaF-OS-Down | GGTATGTAATACGTCGCCCATCGATGATGTTTTAACGATCAAA ACCCGCATCAGAAGAACTCGTCAAGAAGG |
| rfaG | rfaG-OS-Upper | GAAAAAATGCTGCCGCATGAGGCACGCACCATAGATTTGGACA GCCTGCTATGATTGAACAAGATGGATTGCAC |
| | rfaG -OS-Down | CCTCAAAAGCATCTTTACCGCGCCATAGTGTGGTTAACGGCGC TTTCAGCTCAGAAGAACTCGTCAAGAAGG |
| rfaY | rfaY-OS-Upper | TCTTTATCGTAAACTTTTCCACAAATAATTCCCCTCCCTAAAT CTCCATTATGATTGAACAAGATGGATTGCAC |
| | rfaY -OS-Down | CAAATAAAAGTCAGTCAGCCCCTTAAATTAAACCAAACGTGCT ACATTGATCAGAAGAACTCGTCAAGAAGG |
| htrB/lpxL | htrB/lpxL-OS-Upper | CAAAAAGATGCGAGAATACGGGGAATTGTTCGTTGAAAGACAG GATAGAAATGATTGAACAAGATGGATTGCAC |
| | htrB/lpxL -OS-Down | TTTTAAAGCTAAAAGAGGGGAAAAATTGCAGCCTGACGGCTGC AATCCTGTCAGAAGAACTCGTCAAGAAGG |
| yeiU | yeiU-OS-Upper | TACCTTCAACGATGTAAAACTATCGCTGAAGGTAATGTTAATT ACATAGGTCAGAAGAACTCGTCAAGAAGG |
| | yeiU -OS-Down | CGTTTAGCGACGCACGCGTAAGGTTGCCACCGTTTAAAAACTA TGGCGAAATGATTGAACAAGATGGATTGCAC |
| pagP | pagP-OS-Upper | TATTGTTTTTTAAGACTTTTTAATTCACAACTGAAGCATACCC TTCCCCATCAGAAGAACTCGTCAAGAAGG |
| | pagP -OS-Down | ATTTTATTCAGGTTAATGTTGTTATTATCACAGTCGAATTTTT GAACGGTATGATTGAACAAGATGGATTGCAC |
| yhjw | yhjw-OS-Upper | AAGAATCGTTGTACAGGTCGTTTTTATCCCGATTCCCAGGGTT TGTTTGCATGATTGAACAAGATGGATTGCAC |
| | yhjw -OS-Down | TCCCTTAGTCGCAAAATACAATTCAATCGGCGAGAAAGTCGGC AGACAACTCAGAAGAACTCGTCAAGAAGG |
| kdsA | kdsA-OS-Upper | TGTTCTACATTAATTAACCGACATTTTACCTATAAGTGTAAGG CGATCCTATGATTGAACAAGATGGATTGCAC |
| | kdsA -OS-Down | AAAAAAGCCGGATAGTATCTTATCCGGCCTACACAAGCTAAAA ACCGCAATCAGAAGAACTCGTCAAGAAGG |
| lpxB | lpxB-OS-Upper | CACTTCATCCACACCCGCCACTAAATGGGTGTGTGGATAAACA AATTCGATCAGAAGAACTCGTCAAGAAGG |
| | lpxB -OS-Down | AAGGCGTTTACCGAGTTCTTTGAGCGTTCAACGCGCGGTCCGA TTCGTTAATGATTGAACAAGATGGATTGCAC |
| lpxK | lpxK-OS-Upper | ATGACATTCATGACTCCATAAATTCATCACTGCCGCTGCATTG GGGGACGTCAGAAGAACTCGTCAAGAAGG |
| | lpxK -OS-Down | CTGGCGCAACACGGCGTTTACGCCCAGCTACATAAGATGCAAT TTGGCCAATGATTGAACAAGATGGATTGCAC |
| kdtA/waaA | kdtA/waaA-OS-Upper | AATCGGATCAAACGTACCTGGATAAATCGCCCGTTTTTGCATG ACAGTCCTCAGAAGAACTCGTCAAGAAGG |
| | kdtA/waaA -OS-Down | CAGGCTAAATACATAGAATCCCCAGCACATCCATAAGTCAGCT ATTTACTATGATTGAACAAGATGGATTGCAC |
| rfbN- | rfbN-OS-Upper | TTGATAATATGAAGCAGATGGTTGGCAATTGGTTGGCGGAATC AAAATAAATGATTGAACAAGATGGATTGCAC |
| | rfbN -OS-Down | CATAAACGGCTACCTGTGCCGCCAGCCATAATTACGGGAAGAA AAGACATTCAGAAGAACTCGTCAAGAAGG |
| rfbV | rfbV-OS-Upper | TTGGGGCTTCCACTAACTTACTTAATTAAGGCAAATAAGGGA TAATCATATGATTGAACAAGATGGATTGCAC |
| | rfbV -OS-Down | GAATCCAGAAAGGTTAAATCTACATTTAATCTCAATATAATAA CTTACTATCAGAAGAACTCGTCAAGAAGG |
| wzxE | wzxE-OS-Upper | GGTGTGGTTATGGTGAGGGATATCCGATCCCAGGACGTGAATC AGTACAGTCAGAAGAACTCGTCAAGAAGG |
| | wzxE -OS-Down | CGTCGATCAACGAACGGTGATTACTACCTTGCTAAATTATTTC TCCTGATATGATTGAACAAGATGGATTGCAC |
| lptC/yrbK | lptC/yrbK-OS-Upper | CGGCGAAAGCCGGAATGCTGGCGGCCAGCAGCGAGCCGGCAAG CATAAGATCAGAAGAACTCGTCAAGAAGG |
| | lptC/yrbK -OS-Down | TTGCTGCTGCTGGCGCAGGGCAAGCTGGATGAGGCCAAAGGGC AATCGATATGATTGAACAAGATGGATTGCAC |
| | Forward primer 2 | TCGCTACACTATTCACAATTCCTTTTCGCGTCAGCAGACCCTG GAAAAGCCGAAGAAAGGCCCACCCGTG |
| | Reverse primer 2 | AGGTAGTACAGGGTTTGTCAGCATAAAGCCTCTCTTACGAGAG GCTTTATATTTGTCCTACTCAGGAGAGCGTTC |
| | Forward primer 3 | AATACTTGTAACGCTACATGGAGATTAACTCAATCTAGAGGGT ATTAATACGAAGAAAGGCCCACCCGTG |
| | Reverse primer 3 | CGGACAAAAAAATGGCGCACGATGTGCGCCATTTTATATTGTG CGTCAAAATTTGTCCTACTCAGGAGAGCGTTC |

The identification primers are shown in Table 4 (using primer iden-XXX-F/R and primer iden-KanR-KI-R, XXX is the gene name).

**Table 4**

| | |
|---|---|
| iden-htrB-F | CGTTCCACAGTATGATGCG |
| iden-msbB-F | CGGTTCAACCAATACCACG |
| iden-lpxP-F | CCTGATGAGCTTACTCAAGTAAG |
| iden-yeiU-F | CACAGAAACCGACTGGAATAC |
| iden-arnT-F | CTTTTGTCCTCTGGGCGAAC |
| iden-pagP-F | CGGTTCTGAGTGCTAAATCAA |
| iden-yidB-F | CAGACTCCAGACTATGCTCG |
| iden-yhjw-F | CATTGAGCAGGGTTTCTGAAT |
| iden-kdsA-F | CATATTTCGTTCTCTACCGGAC |
| iden-yrbI-F | CATATCACCTCCGTACTGGTT |
| iden-yrbH-F | CGTGATTATAGCGTGATGTTGC |
| iden-rfbU-R | CGACATAGTTGTTAAATCGGGAAA |
| iden-rfbN-R | CATCTTTATGAGGAAGTTGTCTACC |
| iden-rfbV-R | CTGCTTGAGAATCGAGATAAGT |
| iden-gtrba-R | CTCAATGAGATGGGGGATTAC |
| iden-lpxA-R | CTACCGTACAATCATGTGCG |
| iden-lpxB-R | CGGGCTGACATAATGAATGG |
| iden-lpxC-R | CAGCATTCAGGTGCTCAAC |
| iden-lpxD-R | CGTAGTATCTAAAATTTGCGCC |
| iden-lpxH-R | CAACACATTGCGGCCATAC |
| iden-lpxK-R | CATCGTCGGTGATAATAATCTGC |
| iden-lpxO-R | CTTGAAAAAGGTATTGAATCCGGC |
| iden-kdtA-R | CAAGTAAACGTGCTGAACATC |
| iden-kdsB-R | CGCACTTCTCTACCACTTCC |
| iden-KanR-KI-R | CAAGGTGAGATGACAGGAGAT |
| iden-KO-wzzB-F | CTGTTGCAGGATCAGGATCT |
| iden-KO-gtrBb-F | CTATAATCCTGCCACTCCATG |
| iden-KO-rfe-F | CAGAGTAACACCATGCACAAT |
| iden-KO-rfaB-F | CAATAGAGACACCACAGCCA |
| iden-KO-rfaC-F | CATTACGGGTTATCACAAAGTG |
| iden-KO-rfaF-F | CGACAAACCCTTTAAGACCG |
| iden-KO-rfaG-F | CAGGAAGTAGCTTTTACCGT |
| iden-KO-yibD-F | CGAGAGCATTACGCAAAGTT |
| iden-KO-rfaI-F | CATCAACGCCATAAGAAATGTT |
| iden-KO-rfaJ-F | CCGTAGTCTGTATTAATCAATACAACTT |
| iden-KO-rfaK-F | CGATATTGCTCAAATTGGTATCAT |
| iden-KO-rfaL-F | CTGGGTTAATTGGAGGTTATGG |
| iden-KO-rfaP-F | CCAGTCTTCTTCCAAAAAAATCA |
| iden-KO-rfaQ-F | CATGACTGAGTATAAACACGAACC |
| iden-KO-rfaY-F | CAGAACATCGGCGTGAGTTA |
| iden-KO-rfaZ-F | CGGTATTGATAGTCGTTTTGC |
| iden-KO-yijP-F | CGTGATACATGATAATCCCCT |
| iden-KO-gmhA-F | CTGACCACTTGTGATGATTAGG |
| iden-KO-yaeD-F | CGTAATGAAATGAGCGGAGG |
| iden-KO-rfaE-F | CAGCTTACTAACAGACAATCAGAG |
| iden-KO-rfaD-F | CTTCTTTAAACAATCCTTCCGC |
| iden-KO-rfbN-F | CGCTTTATAGTATTTTGCAGCA |
| iden-KO-rfbU-F | CTGCTTAATCGCATCCAGAAC |
| iden-KO-rfbV-F | CCATACGCATCTTTAAAGCAAT |
| iden-KO-gtrBa-F | CGAAAGCTCTCTTAGGGCA |
| iden-KO-lpxA-F | CATGGAAGTCACTTTCGAGAA |
| iden-KO-lpxB-F | CAGTGGTAAAACGCTCGAC |
| iden-KO-lpxC-F | CCGGATTATCTGGATATCCCC |
| iden-KO-lpxD-F | CGAAGAACGCAACAAACTG |
| iden-KO-lpxH-F | CATGCGTCTTAAAACTCCTG |
| iden-KO-lpxK-F | CCTTGATGAAGCTACGTCC |
| iden-KO-lpxO-F | CATTTATTATGGGGAGAAACGC |
| iden-KO-kdtA-F | CGCAAAAATGGGAAAAGTAATG |
| iden-KO-kdsB-F | CAAACTCTGGTATAACCAGGAAC |
| iden-KO-rfbP-F | CATTACATACTCCTGTCAGGTG |
| iden-msbA-KO | CAGTGGTTTGATACACCCCA |
| iden-wzy-KO | CTCAGCAAACCCAACAAGCT |
| iden-wzxE-KO | CTACACCACAAAAGAGAGCG |
| iden-lptA-KO | CAGGATGTTACCTCGAACGAT |
| iden-lptB-KO | CTGGTGAAAGAGCAGAAAATGC |
| iden-lptC-KO | CTGACTATGTGACGCACATT |
| iden-lptD-KO | CTGCGACTTTATCGACTACC |
| iden-lptG-KO | CCTGATCTATCTGGCGTTGG |

### Example 4: Construction of DB-ZW1 Strain with aTc-Induced Expression of LPS-Related Essential Genes

The fragment Backbone-ptet-tetR-aad was obtained by PCR with ptet-tetR-aaad plasmid (SEQ ID NO: 23) as the template, reverse primer 1/forward primer 1 (see Table 3) and the PCR reaction system and condition described in Example 3.

Plasmids ptet-tetR-XXX-aaad were constructed by one-step cloning with Vazyme one-step cloning kit (ClonExpress II One Step Cloning Kit, Vazyme # C112) using DB-ZW1 genomic DNA as the template, and a primer pair XXX-OS-Upper/XXX-OS-Down (see Table 3, where XXX represents the corresponding gene) and the fragment Backbone-ptet-tetR-aaad.

The corresponding homologous recombination fragments were obtained by PCR with the plasmid ptet-tetR-XXX-aaad as the template and the primer pair XXX-HR-Upper/XXX-HR-Down (see Table 3, where XXX represents the corresponding gene) and the PCR reaction system and conditions described in Example 3.

The homologous recombination fragment was integrated into the original gene position of the strain ZW1 by the λ-red homologous recombination method to obtain the strains ZW1(ΔXXX::ptet-tetR-XXX)-aadA, the identification primers were shown in Table 4 (the primers idel-KO-XXXX-F or idel-XXXX-KO and idel-KanR-KI-R were used, XXX was the gene name).

### Example 5: Construction of Strains with Hypoxia Induced Expression of Bacterial LPS-Associated Genes or Mureinlipoproteins

### 5.1 Construction of strain for the hypoxia induced expression of msbb gene

Using plasmid Psc101-Fnr-SP-msbb-KnaR (SEQ ID NO: 24) as the template, forward primer 2 and reverse primer 2 were used for PCR with the PCR reaction system and condition as described in Example 3 to obtain homologous recombination fragment 5.

The homologous recombination fragment 5 was integrated into the position of the homologous recombination fragment 3 in the strain ZW1(Δmsbb)-aadA by the λ-red homologous recombination method to obtain the strain ZW1(Δmsbb)-Fnr-SP-msbb-KnaR.

### 5.2 Construction of a strain for the hypoxia induced expression of lppAB gene

Using plasmid Psc101-Fnr-SP-lppAB-KnaR (SEQ ID NO: 25) as the template, forward primer 3 and reverse primer 3 were used for PCR with the PCR reaction system and condition as described in Example 3 to obtain homologous recombination fragment 6;

The homologous recombination fragment 6 was integrated into the position of the homologous recombination fragment 4 in the strain ZW1(ΔlppAB)-aadA by the λ-red homologous recombination method to obtain the strain ZW1(Δ1ppAB)-Fnr-SP-lppAB-KnaR.

### 5.3 Construction of Plasmid for Hypoxia Induced Expression of Bacterial LPS-Associated Genes

Using the genome of strain DB-ZW1 as the template, PCR was performed using the primer pair **F/**R (see Table 5) with the PCR reaction system and condition as described in Example 3 to obtain homologous recombination target fragments, respectively. Using psc101-FNR-sp-msbb-KanR as the template, PCR was performed using a primer pair P-backbone-R1101/P-backbone-SC101-F1130(P-R1101/P-F1130) with the PCR reaction system and condition as described in Example 3 to obtain a linear vector backbone fragment for homologous recombination.

The PCR system (total volume 50µL) contained 1×PrimeSTAR Max Premix, 0.2-0.3µM each primer, 200 ng of template and water.

PCR reaction condition was: denaturation: 98 ° C 10 sec, annealing: 55 ° C 10 sec, extension: 72 °C 5 sec/kb.

**Table 5**

| Gene | Primer name | Primer Sequence |
|---|---|---|
| | P- R1101 | CAAGTAGGCCATCGCTTG |
| | P-F1130 | CATACGATGAAGGCGATAAGT |
| rfbN | 1F | GTCAAGCGATGGCCTACTTGATGAAAATAACATTAATTATTCCCACA |
| | 1R | CTTATCGCCTTCATCGTATGTTATTTTATCTCTTTTGACGAAGAAT |
| rfbU | 2F | GTCAAGCGATGGCCTACTTGTATTTTGATTCCGCCAACCAA |
| | 2R | CTTATCGCCTTCATCGTATGATGATCGTAAACCTATCACGTTT |
| rfuV | 3F | GTCAAGCGATGGCCTACTTGATGCTTATATCATTTTGTATTCCAAC |
| | 3R | CTTATCGCCTTCATCGTATGCTACTTAATTATCCGTTTAGTAAAATTC |
| wzzB | 4F | GTCAAGCGATGGCCTACTTGATGACAGTGGATAGTAATACGTCT |
| | 4R | CTTATCGCCTTCATCGTATGTTACAAGGCTTTTGGCTTATAGC |
| gtrBb | 5F | GTCAAGCGATGGCCTACTTGATGAAAATTTCATTAGTGGTTCCC |
| | 5R | CTTATCGCCTTCATCGTATGTTATTCCATCATTGTTTTTTTGTTTTTCAC |
| gtrBa | 6F | GTCAAGCGATGGCCTACTTGATGAAAATCTCGTTAGTCGTTCC |
| | 6R | CTTATCGCCTTCATCGTATGTTAACCATTTTTCTTCTCATTCTTAAT |
| rtbP/wba P | 7F | GTCAAGCGATGGCCTACTTGATGGATAATATTGATAATAAGTATAATCCAC AG |
| | 7R | CTTATCGCCTTCATCGTATGTTAATACGCACCATCTCGCC |
| rfe | 8F | GTCAAGCGATGGCCTACTTGGTGAAGTTACTCACCGCTTT |
| | 8R | CTTATCGCCTTCATCGTATGTTACTTGGTTAAGTTTGGCCG |
| rfaB | 9F | GTCAAGCGATGGCCTACTTGATGAAAATAGCTTTTATTGGCGAA |
| | 9R | CTTATCGCCTTCATCGTATGTCACTTTTGTAATTTCGAGAAT |
| rfaC/waa C | 10F | GTCAAGCGATGGCCTACTTGATGCGGGTTTTGATCGTTA |
| | 10R | CTTATCGCCTTCATCGTATGTTAATGAATCTTTCCAAATACGCTTG |
| rfaF | 11F | GTCAAGCGATGGCCTACTTGATGAAAATTTTGGTCATTGGCC |
| | 11R | CTTATCGCCTTCATCGTATGTTAAACGCCCTCTTCCGACAA |
| rfaG | 12F | GTCAAGCGATGGCCTACTTGATGAGAGTTGCCTTTTGCTT |
| | 12R | CTTATCGCCTTCATCGTATGTCAACCATCTAAATCACCTGTAATAAT |
| yibD | 13F | GTCAAGCGATGGCCTACTTGATGAAAAATAGTAAAACCAAAGTGAGT |
| | 13R | CTTATCGCCTTCATCGTATGCTAAGACAGATTGAGCGCC |
| rfaI | 14F | GTCAAGCGATGGCCTACTTGATGAGCAGAAAATATTTTGAAGAAGA |
| | 14R | CTTATCGCCTTCATCGTATGTTATTCAAGAAGTTTACGTTTAAAGTAGG |
| rfaJ | 15F | GTCAAGCGATGGCCTACTTGATGGATTCATTTCCTGAGATAGAAAT |
| | 15R | CTTATCGCCTTCATCGTATGTTATTTGTGGAAAAGTTTACGATAAAGATA |
| rfaK | 16F | GTCAAGCGATGGCCTACTTGATGATTAAAAAAATCATATTTACTGT |
| | 16R | CTTATCGCCTTCATCGTATGTCACTTATCAAACCAGTTTTTCATTTG |
| rfaL | 17F | GTCAAGCGATGGCCTACTTGATGCTAACCACATCATTAACGT |
| | 17R | CTTATCGCCTTCATCGTATGTTATCTATTTCTTAGCGCCAGC |
| rfaP | 18F | GTCAAGCGATGGCCTACTTGATGGTTGAGCTGAAAGCG |
| | 18R | CTTATCGCCTTCATCGTATGTTACAGATTTTTTCTTATTGTCCTTTC |
| rfaQ | 19F | GTCAAGCGATGGCCTACTTGGTGGAAAAGCCATTTCGAAG |
| | 19R | CTTATCGCCTTCATCGTATGTCATAGCAGGCTGTCCAAAT |
| rfaY | 20F | GTCAAGCGATGGCCTACTTGATGATTATTGAAAAAAAGATTAAAAACTATA CCG |
| | 20R | CTTATCGCCTTCATCGTATGTCAGCGTTTTTCCTTACCC |
| rfaZ | 21F | GTCAAGCGATGGCCTACTTGATGGGCAGCGTTAACTTCATA |
| | 21R | CTTATCGCCTTCATCGTATGCTAGACAATTTTATCGTAATATATTTCATCC TC |
| yijP | 22F | GTCAAGCGATGGCCTACTTGTATGCAATCCACATTACTCCAG |
| | 22R | CTTATCGCCTTCATCGTATGCTATTGATTACCGACCTGATCG |
| gmhA | 23F | GTCAAGCGATGGCCTACTTGATGTACCAGGATCTTATTCGTAAC |
| | 23R | CTTATCGCCTTCATCGTATGTTATTTAACCATCTCTTTTTCGATCA |
| yaeD | 24F | GTCAAGCGATGGCCTACTTGGTGGCAAAGTTTGTACCCG |
| | 24R | CTTATCGCCTTCATCGTATGTTATTTTTGCTGCTTTTTTATCGC |
| rfaE | 25F | GTCAAGCGATGGCCTACTTGATGAAAGTAAATCTGCCAGC |
| | 25R | CTTATCGCCTTCATCGTATGTTACTTCTCGCTCTCGGTC |
| rfaD | 26F | GTCAAGCGATGGCCTACTTGATGATCATCGTTACCGGCGG |
| | 26R | CTTATCGCCTTCATCGTATGTTACGCGTCGCGGTTCAGCC |
| lpxA | 27F | GTCAAGCGATGGCCTACTTGGTGATTGATAAATCCGCCTTTAT |
| | 27R | CTTATCGCCTTCATCGTATGTTAACGAATCGGACCGCGCG |
| lpxB | 28F | GTCAAGCGATGGCCTACTTGTCATTGTGCTAACTCCAGGACGG |
| | 28R | CTTATCGCCTTCATCGTATGATGGCGGCACAGCGTCCTTT |
| lpxC | 29F | GTCAAGCGATGGCCTACTTGATGATCAAACAAAGGACACTT |
| | 29R | CTTATCGCCTTCATCGTATGTTATGCCAGTACCGTCGAAG |
| lpxD | 30F | GTCAAGCGATGGCCTACTTGATGCCTTCAATTCGACTGG |
| | 30R | CTTATCGCCTTCATCGTATGTTAGTCTTGTTGATTAACCTTGCG |
| lpxH | 31F | GTCAAGCGATGGCCTACTTGGTGGCGACACTGTTTATTGC |
| | 31R | CTTATCGCCTTCATCGTATGTTACAGGGGGAAGGCGATTA |
| lpxK | 32F | GTCAAGCGATGGCCTACTTGATGATTGCGCGTATCTGGTCG |
| | 32R | CTTATCGCCTTCATCGTATGCTAGCGAACCAGCGAGGTGA |
| htrB | 33F | GTCAAGCGATGGCCTACTTGTCAATAGCGCGACGGTACGC |
| | 33R | CTTATCGCCTTCATCGTATGATGACGAAGTTGCCTAAGTTC |
| lpxM | 34F | GTCAAGCGATGGCCTACTTGATGGAAACCAAAAAAAATAATAGTGAGT |
| | 34R | CTTATCGCCTTCATCGTATGTTATTTGATGGGATAAAGATCTTTACGC |
| lpxO | 35F | GTCAAGCGATGGCCTACTTGATGTTCGCAGCAATCATTATCG |
| | 35R | CTTATCGCCTTCATCGTATGTCAGAGGAGGCTGAAAAGGA |
| lpxP | 36F | GTCAAGCGATGGCCTACTTGATGTTTCCTCAAAGCAAATTTTCA |
| | 36R | CTTATCGCCTTCATCGTATGTCAGATGTAGAGTGACGCTT |
| yeiU | 37F | GTCAAGCGATGGCCTACTTGATGACGATGAAAACCCGCTA |
| | 37R | CTTATCGCCTTCATCGTATGTTATTTGTTTAAAATTTGTTTATTTCCGCC |
| kdtA | 38F | GTCAAGCGATGGCCTACTTGATGCTCGAATTGCTTTACACC |
| | 38R | CTTATCGCCTTCATCGTATGTCAATGCGTTTTCGGTGG |
| arnT | 39F | GTCAAGCGATGGCCTACTTGATGATGAAATCGATACGCTATTATCT |
| | 39R | CTTATCGCCTTCATCGTATGTCATTTAGGCCGATACTGAAT |
| pagP | 40F | GTCAAGCGATGGCCTACTTGATGTATGTTGCGATGATCATCA |
| | 40R | CTTATCGCCTTCATCGTATGTCAAAACTGGAAACGCATCC |
| yjdB | 41F | GTCAAGCGATGGCCTACTTGATGTTAAAGCGCTTTCTTAAAAGA |
| | 41R | CTTATCGCCTTCATCGTATGTCATTCGCTTAGTCTCCTGC |
| yhjw | 42F | GTCAAGCGATGGCCTACTTGATGAGATACATTAAATCGATGACGC |
| | 42R | CTTATCGCCTTCATCGTATGCTACTTCGCGGCGACTTTC |
| kdsA | 43F | GTCAAGCGATGGCCTACTTGATGAAACAAAAAGTGGTTAACATTG |
| | 43R | CTTATCGCCTTCATCGTATGTCAGTTCTCGGTATCCAGC |
| kdsB | 44F | GTCAAGCGATGGCCTACTTGATGAGTTTCGTCGTCATCATT |
| | 44R | CTTATCGCCTTCATCGTATGTTAACGCATTTCAGCGCG |
| yrbI | 45F | GTCAAGCGATGGCCTACTTGATGAGTAAAGCAGGTGCGTC |
| | 45R | CTTATCGCCTTCATCGTATGTCATATCGATTGCCCTTTGG |
| yrbH | 46F | GTCAAGCGATGGCCTACTTGATGTCGCATTTAGCGTTGC |
| | 46R | CTTATCGCCTTCATCGTATGCTACACGACGCCTGCACG |
| msbA | 47F | GTCAAGCGATGGCCTACTTGATGCATAACGATAAAGATCTCTC |
| | 47R | CTTATCGCCTTCATCGTATGTCATTGGCCAAATTGCATCT |
| wzy | 48F | GTCAAGCGATGGCCTACTTGATGCTTATAATTTCATACATTGCATT |
| | 48R | CTTATCGCCTTCATCGTATGTTATTTATTGTTTCTTAGTAAAACGAATCT |
| wzxE | 49F | GTCAAGCGATGGCCTACTTGATGTCGCTTGCTAAAGCC |
| | 49R | CTTATCGCCTTCATCGTATGTCATGCTCGCCTACGCCAGA |
| lptA | 50F | GTCAAGCGATGGCCTACTTGATGAAATTCAAAACAAACAAACTCA |
| | 50R | CTTATCGCCTTCATCGTATGTTAGTTACTCTTCTTTTGCGC |
| lptB | 51F | GTCAAGCGATGGCCTACTTGATGGCAACATTAACTGCAAAG |
| | 51R | CTTATCGCCTTCATCGTATGTCAGAGTCTGAAGTCTTCCC |
| lptC/yrbK | 52F | GTCAAGCGATGGCCTACTTGATGAGTAAAACCAGACGTTGG |
| | 52R | CTTATCGCCTTCATCGTATGTTAAGGCTGAGTTTGTTTGTTTT |
| lptD | 53F | GTCAAGCGATGGCCTACTTGTTACATAGAGCTTTGGTACGG |
| | 53R | CTTATCGCCTTCATCGTATGATGAAAAAACGTATTCCCACTCTTC |
| lptG | 54F | GTCAAGCGATGGCCTACTTGATGCAGCCATTTGGTGTACT |
| | 54R | CTTATCGCCTTCATCGTATGTTACGACTTACGCAGCATTAG |

Recombination of vector fragments and target gene fragments was carried out by a Vazyme one-step cloning kit (ClonExpress II One Step Cloning Kit, Vazyme # C112), and the recombinant plasmid was chemically transformed into cloning competent cells (DH5α Compound E. coli Strain, Vazyme # C502) and designated as: psc101-FNR-sp-####-KanR, where ### corresponds to the gene name listed in Table 5.

### 5.4 Construction of a strain for the hypoxia induced expression of bacterial LPS-associated genes:

The constructed anaerobic expression plasmid psc101-FNR-sp- ###-KanR was used as the template, and the primer pair **F-in/**R-in shown in Table 6 was used for PCR with the PCR reaction system and condition as described in Example 3 to obtain a fragment for homologous recombination knock-in, that is, a fragment for anaerobically expressing LPS-related genes and carrying the Kana-resistance gene.

**Table 6**

| Gene | Primer name | Primer Sequence |
|---|---|---|
| rfbN | 1F-in | CATAAACGGCTACCTGTGCCGCCAGCCATAATTACGGGAAGAAAAGAC ATCTAGTGCCGATCATATTCAATAACCC |
| | 1R-in | TTGATAATATGAAGCAGATGGTTGGCAATTGGTTGGCGGAATCAAAAT AACTTATCGCCTTCATCGTATG |
| rfbU | 2F-in | CTTATGATTATTGCTTTAAAGATGCGTATGGAAAAACGGAGAGCTATT CACTAGTGCCGATCATATTCAATAACCC |
| | 2R-in | GGCCAAAGCGACCCTGCATTATATGTGGGAATAATTAATGTTATTTTC ATCTTATCGCCTTCATCGTATG |
| rtbV | 3F-in | TTGGGGGCTTCCACTAACTTACTTAATTAAGGCAAATAAGGGATAATC ATCTAGTGCCGATCATATTCAATAACCC |
| | 3R-in | GAATCCAGAAAGGTTAAATCTACATTTAATCTCAATATAATAACTTAC TACTTATCGCCTTCATCGTATG |
| wzzB | 4F-in | TTTATGGCTACACTGTCTCCAGCTTCATCCTTTTTTTAGTTAGGGTAT CTCTAGTGCCGATCATATTCAATAACCC |
| | 4R-in | TTAATGAGAAATTTTACCTGTCGTAGCCGACCACCATCCGGCAAAGAA GCCTTATCGCCTTCATCGTATG |
| gtrBb | 5F-in | CTTATTTGCGGATTTTTATATTCCAGATTCATTGTTTTCAGGAATGAG AACTAGTGCCGATCATATTCAATAACCC |
| | 5R-in | AGCCAGTAGTATCATCGCCGTTTTCGGTAATATCTTGAGCATAGTGTT CACTTATCGCCTTCATCGTATG |
| gtrBa | 6F-in | CTGATATGCGGATTCATTTATTCAAAGTTCATTGTCTTTAGGGATGCG AACTAGTGCCGATCATATTCAATAACCC |
| | 6R-in | ACACCAACGAGAAAACTATAAGGATAGCGATTACCATTTTTAATCTAT TGCTTATCGCCTTCATCGTATG |
| rtbP/wba P | 7F-in | TAATATGCCTATTTTATTTACATTATGCACGGTCAGAGGGTGAGGATT AACTAGTGCCGATCATATTCAATAACCC |
| | 7R-in | TTTTACGCAGGCTAATTTATACAATTATTATTCAGTACTTCTCGGTAA GCCTTATCGCCTTCATCGTATG |
| rfe | 8F-in | GTAACGCATTATACTTTAAGGAAATGATATATGCTGAGAGCACATACA CTCTAGTGCCGATCATATTCAATAACCC |
| | 8R-in | AGCGCTCACCGCGCGTGCCCCCGGTAATGGTTGTGTCATCGTATCCTC ATCTTATCGCCTTCATCGTATG |
| rfaB | 9F-in | CTATTTAATATCTAAAGTCTTTAGATTAAGGTTTTAACTCTGGGGTAT ATCTAGTGCCGATCATATTCAATAACCC |
| | 9R-in | TAAAGTCTGTTGAATGACTTCTTCTTCAAAATATTTTCTGCTCATAGT GACTTATCGCCTTCATCGTATG |
| rfaC/waa C | 10F-in | CAGCGGGTTCTGGAAGAGCTTCATTCGCTGTTGTCGGAAGAGGGCGTT TACTAGTGCCGATCATATTCAATAACCC |
| | 10R-in | GTGGTTAGCATCTTTTCTCCACAATAGGTTTGGGATGAGACAGAGTCT CTCTTATCGCCTTCATCGTATG |
| rfaF | 11F-in | GCCGAAGGCGTCACGGAGTATATGGCCTGGCTGAACCGCGACGCGTAA GTCTAGTGCCGATCATATTCAATAACCC |
| | 11R-in | GGTATGTAATACGTCGCCCATCGATGATGTTTTAACGATCAAAACCCG CACTTATCGCCTTCATCGTATG |
| rfaG | 12F-in | GAAAAAATGCTGCCGCATGAGGCACGCACCATAGATTTGGACAGCCTG CTCTAGTGCCGATCATATTCAATAACCC |
| | 12R-in | CCTCAAAAGCATCTTTACCGCGCCATAGTGTGGTTAACGGCGCTTTCA GCCTTATCGCCTTCATCGTATG |
| yibD | 13F-in | AGAGCCACAGTATAAAGGCAGGGTAAATTAAGGTTTTTCTGGTAATCG TTCTAGTGCCGATCATATTCAATAACCC |
| | 13R-in | GCACCAGTGGCAAGGCTGGGGGAAAATCGCCGCGCCTAAAAACGTGAG CGCTTATCGCCTTCATCGTATG |
| rfaI | 14F-in | TTTAAAAATTTTAATAATGCAATATTCTCGAAATTACAAAAGTGATCA CTCTAGTGCCGATCATATTCAATAACCC |
| | 14R-in | TTCAGCTATTTCTATCTCAGGAAATGAATCCATTACATCACCTATGGG TTCTTATCGCCTTCATCGTATG |
| rfaJ | 15F-in | ATAGCCTACTTTAAACGTAAACTTCTTGAATAAAACCCATAGGTGATG TACTAGTGCCGATCATATTCAATAACCC |
| | 15R-in | AGTTTTTAATCTTTTTTTCAATAATCATAATGGAGATTTAGGGAGGGG AACTTATCGCCTTCATCGTATG |
| rfaK | 16F-in | TTATAACATTTCTTTTTCTATAAAGTTAACTATTCTGGATTATAGCTA TTCTAGTGCCGATCATATTCAATAACCC |
| | 16R-in | GCGCTGATACTTATTACGGTATCAGCGCGTTTTCCATCATCAGGACTC AACTTATCGCCTTCATCGTATG |
| rfaL | 17F-in | AGATTCATTAAAGAGACTCTGTCTCATCCCAAACCTATTGTGGAGAAA AGCTAGTGCCGATCATATTCAATAACCC |
| | 17R-in | CCTGATGATGGAAAACGCGCTGATACCGTAATAAGTATCAGCGCGTTT TTCTTATCGCCTTCATCGTATG |
| rfaP | 18F-in | TTTATACAGCTTACCGGAGAAGGCCGCGGATATTATTACAGGTGATTT AGCTAGTGCCGATCATATTCAATAACCC |
| | 18R-in | CTATCCTTGTCTCACTCATAAATTACTCACTGAGTGCATAATTATTAT AACTTATCGCCTTCATCGTATG |
| rfaQ | 19F-in | GTGCTTTTTTCTGCTTATTTATCCGGACAATTTCAACCAAAGAGTCGC TTCTAGTGCCGATCATATTCAATAACCC |
| | 19R-in | ACGCTGCAGACCGCCAAAAGGAAAATATTTATATAAGCAAAAGGCAAC TCCTTATCGCCTTCATCGTATG |
| rfaY | 20F-in | TCTTTATCGTAAACTTTTCCACAAATAATTCCCCTCCCTAAATCTCCA TTCTAGTGCCGATCATATTCAATAACCC |
| | 20R-in | CAAATAAAAGTCAGTCAGCCCCTTAAATTAAACCAAACGTGCTACATT GACTTATCGCCTTCATCGTATG |
| rfaZ | 21F-in | TTGAATATAACATTTATTTATAGGTTGCATGATAAACATACGAGTTTT TTCTAGTGCCGATCATATTCAATAACCC |
| | 21R-in | AAAAAAGAAATGCGTGCCGAAGCAACGAAAAATAATGATTTATCTAGT GTCTTATCGCCTTCATCGTATG |
| yijP | 22F-in | TAAGGTTCAGGCGTTACGTTTGGCGCTACGATTAAAGACAGGCTCTCA TTCTAGTGCCGATCATATTCAATAACCC |
| | 22R-in | AGAAGCACGCCAGGCCCGATAAGCGTTAGCGTTATCGGGCAACATACA GGCTTATCGCCTTCATCGTATG |
| gmhA | 23F-in | ATCCGGTACACTGCATACTGTCATTTACATTCATGCTGAAGGATATCC TCCTAGTGCCGATCATATTCAATAACCC |
| | 23R-in | GTTTCTGAGCGCAACCAACGCACCTGCAGCTTGAAGTATGACGGGTAT AGCTTATCGCCTTCATCGTATG |
| yaeD | 24F-in | CTGAAACATGCGATACTAGCGCCACTTGCTTTACTCAGGAGCTATAAA AGCTAGTGCCGATCATATTCAATAACCC |
| | 24R-in | AGCGGAAAAATGCATTTTTGTTTTAACCGCTCATCTTTTATCCATAAC GCCTTATCGCCTTCATCGTATG |
| rfaE | 25F-in | TGAATTATGCTATTATCGCGCGCAAATTTTGAATCTCTCAGGAGACAG GACTAGTGCCGATCATATTCAATAACCC |
| | 25R-in | CGCCTTCAAAATCAGCGCCCGCCGCGTCTGTAACGCCTGATGACGCGG TTCTTATCGCCTTCATCGTATG |
| rfaD | 26F-in | GCATTCATGTCTGAGACAGTCTCTGACACCATAATTCAAAGGTTACAG TTCTAGTGCCGATCATATTCAATAACCC |
| | 26R-in | CATCATCATGTCGCCCACCCAGGACGGGCCAATGACCAAAATTTTCAT ACCTTATCGCCTTCATCGTATG |
| lpxA | 27F-in | TCGTGTGCGAAGCAACCATGATGTGTGCTCGTAGCCGGGAGGCCTGAT TCCTAGTGCCGATCATATTCAATAACCC |
| | 27R-in | GGACGTTTCACCGGCGACCAGGGCAATCGTTAAAGGACGCTGTGCCGC CACTTATCGCCTTCATCGTATG |
| lpxB | 28F-in | AAGGCGTTTACCGAGTTCTTTGAGCGTTCAACGCGCGGTCCGATTCGT TACTAGTGCCGATCATATTCAATAACCC |
| | 28R-in | CACTTCATCCACACCCGCCACTAAATGGGTGTGTGGATAAACAAATTC GACTTATCGCCTTCATCGTATG |
| lpxC | 29F-in | CGAATGTATAGTACACTTCGGTTGGATAGGTAATTTGGCGAGATTATA CGCTAGTGCCGATCATATTCAATAACCC |
| | 29R-in | GGAGAGAGTGCCAGGTTAACCAGTCGAATTTTATACGACAGCGTATGT CGCTTATCGCCTTCATCGTATG |
| lpxD | 30F-in | GCGATGTGAAAGACATCACCGCTGACGTACTGAAACAGGTTAAATAAG TACTAGTGCCGATCATATTCAATAACCC |
| | 30R-in | TCGTATGAATGTGACAGGCCGGAAAAGAATGGCAACTACAAGGCGGAA CGCTTATCGCCTTCATCGTATG |
| lpxH | 31F-in | GTACCAAAAGAAGACGTCATCATTGAAAACGTGACCGTCAGCGAGTAA TCCTAGTGCCGATCATATTCAATAACCC |
| | 31R-in | GGCAAGGAAAACGGTTGCGTGCGCGCCGTAACGGACGATTCGACGAAC ATCTTATCGCCTTCATCGTATG |
| lpxK | 32F-in | CTGGCGCAACACGGCGTTTACGCCCAGCTACATAAGATGCAATTTGGC CACTAGTGCCGATCATATTCAATAACCC |
| | 32R-in | ATGACATTCATGACTCCATAAATTCATCACTGCCGCTGCATTGGGGGA CGCTTATCGCCTTCATCGTATG |
| htrB | 33F-in | CAAAAAGATGCGAGAATACGGGGAATTGTTCGTTGAAAGACAGGATAG AACTAGTGCCGATCATATTCAATAACCC |
| | 33R-in | TTTTAAAGCTAAAAGAGGGGAAAAATTGCAGCCTGACGGCTGCAATCC TGCTTATCGCCTTCATCGTATG |
| lpxM | 34F-in | TCGCTACACTATTCACAATTCCTTTTCGCGTCAGCAGACCCTGGAAAA GCCTAGTGCCGATCATATTCAATAACCC |
| | 34R-in | AGGTAGTACAGGGTTTGTCAGCATAAAGCCTCTCTTACGAGAGGCTTT ATCTTATCGCCTTCATCGTATG |
| lpxO | 35F-in | GGTTGCCTGAAAATCCCGGGATGAGGTTTTCTTAGAAGGACAGTGACG TTCTAGTGCCGATCATATTCAATAACCC |
| | 35R-in | CGCCAGGATAGAACAGGCCGCGGCAGGCAGTTCCATACCGCGGCCCGG CTCTTATCGCCTTCATCGTATG |
| lpxP | 36F-in | ATAATGACTCTTCTGCGATACATTATTTTCGTATCTACAGAAGGTATT TTCTAGTGCCGATCATATTCAATAACCC |
| | 36R-in | GAATATTATGACAATGCGAAATGCCAAAATAAAAGGCCTTGCGGCCTT TTCTTATCGCCTTCATCGTATG |
| yeiU | 37F-in | CGTTTAGCGACGCACGCGTAAGGTTGCCACCGTTTAAAAACTATGGCG AACTAGTGCCGATCATATTCAATAACCC |
| | 37R-in | TACCTTCAACGATGTAAAACTATCGCTGAAGGTAATGTTAATTACATA GGCTTATCGCCTTCATCGTATG |
| kdtA | 38F-in | CAGGCTAAATACATAGAATCCCCAGCACATCCATAAGTCAGCTATTTA CTCTAGTGCCGATCATATTCAATAACCC |
| | 38R-in | AATCGGATCAAACGTACCTGGATAAATCGCCCGTTTTTGCATGACAGT CCCTTATCGCCTTCATCGTATG |
| arnT | 39F-in | ATCCCTGGCCGTGAAGGTTGGCTGGGGTGTCAACAGGCAGCGAGCGCC TCCTAGTGCCGATCATATTCAATAACCC |
| | 39R-in | GAGCTGACCGCCAACGCTGAGCAGACTGGCAAGCACCAGAACGACGCC GACTTATCGCCTTCATCGTATG |
| pagP | 40F-in | ATTTTATTCAGGTTAATGTTGTTATTATCACAGTCGAATTTTTGAACG GTCTAGTGCCGATCATATTCAATAACCC |
| | 40R-in | TATTGTTTTTTAAGACTTTTTAATTCACAACTGAAGCATACCCTTCCC CACTTATCGCCTTCATCGTATG |
| yjdB | 41F-in | TAATTTTACTTTGTCACGATTAGCGTCACCGAATCGATGGACGCATCA ACCTAGTGCCGATCATATTCAATAACCC |
| | 41R-in | GAGTATTAACCCCTGTAATAATAGCGTGTCGTCTTCAACAATCAGTAT CTCTTATCGCCTTCATCGTATG |
| yhjw | 42F-in | AAGAATCGTTGTACAGGTCGTTTTTATCCCGATTCCCAGGGTTTGTTT GCCTAGTGCCGATCATATTCAATAACCC |
| | 42R-in | TCCCTTAGTCGCAAAATACAATTCAATCGGCGAGAAAGTCGGCAGACA ACCTTATCGCCTTCATCGTATG |
| kdsA | 43F-in | TGTTCTACATTAATTAACCGACATTTTACCTATAAGTGTAAGGCGATC CTCTAGTGCCGATCATATTCAATAACCC |
| | 43R-in | AAAAAAGCCGGATAGTATCTTATCCGGCCTACACAAGCTAAAAACCGC AACTTATCGCCTTCATCGTATG |
| kdsB | 44F-in | GTATTGCTGGAAAATGAAGCTCGCGCCCTGACATCTGATGAGAGTAAA TCCTAGTGCCGATCATATTCAATAACCC |
| | 44R-in | TCATACCGCGCTGTTCATGACCGTCTAAGACGATCATGAACAGGATCA TGCTTATCGCCTTCATCGTATG |
| yrbI | 45F-in | CATGATCTCCTGCGTGCAGGCGTCGTGTAGAAACTCAAGGATAATTAA CGCTAGTGCCGATCATATTCAATAACCC |
| | 45R-in | TACCAGAATGGCCAGCGACAGTAGAATGATAACCCAACGTCTGGTTTT ACCTTATCGCCTTCATCGTATG |
| yrbH | 46F-in | GATGCTCTATTGGCTATCGCCACTTCTTGTTGGATAACTGGAAACGGA TTCTAGTGCCGATCATATTCAATAACCC |
| | 46R-in | GGTCGGAAGCGACGCACCTGCTTTACTCATCGTTAATTATCCTTGAGT TTCTTATCGCCTTCATCGTATG |
| msbA | 47F-in | AGGATAACGGGTAGAATATGCGGCTATTTCAACAAATGCTGGTTTTTT GACTAGTGCCGATCATATTCAATAACCC |
| | 47R-in | CGGCAGCAGTAACCGCCATAGCGGGGATTCACCCGACCAGATACGCGC AACTTATCGCCTTCATCGTATG |
| wzy | 48F-in | TGCCTGATGGTAATATTTTTAATACTAAGCATTTTTTCTAAAGGCTCT ATCTAGTGCCGATCATATTCAATAACCC |
| | 48R-in | ATTTTTACGCTTCAGAGCCAAATAAAACGGCGGCATTGCCGCCGTATA ACCTTATCGCCTTCATCGTATG |
| wzxE | 49F-in | CGTCGATCAACGAACGGTGATTACTACCTTGCTAAATTATTTCTCCTG ATCTAGTGCCGATCATATTCAATAACCC |
| | 49R-in | GGTGTGGTTATGGTGAGGGATATCCGATCCCAGGACGTGAATCAGTAC AGCTTATCGCCTTCATCGTATG |
| lptA | 50F-in | TAACTTACGCAGCAAGAACGCCGAGCTGATTGAAAAGGTTAGAACCTC TTCTAGTGCCGATCATATTCAATAACCC |
| | 50R-in | CCTTTATAGGCCTTCGCCAGATTCTTTGCAGTTAATGTTGCCATAACG AACTTATCGCCTTCATCGTATG |
| lptB | 51F-in | ACAAAAACAAAGGCCAGACTCCGGCGCAAAAGAAGAGTAACTAATTCG TTCTAGTGCCGATCATATTCAATAACCC |
| | 51R-in | CATATTCAGAGTCGTTTTCTCCCGCTAGAGCAGCATAGCCCTCTACCC TACTTATCGCCTTCATCGTATG |
| lptC/yrbK | 52F-in | TTGCTGCTGCTGGCGCAGGGCAAGCTGGATGAGGCCAAAGGGCAATCG ATCTAGTGCCGATCATATTCAATAACCC |
| | 52R-in | CGGCGAAAGCCGGAATGCTGGCGGCCAGCAGCGAGCCGGCAAGCATAA GACTTATCGCCTTCATCGTATG |
| lptD | 53F-in | ACCGTTTGTCACGCGCAACGCTACCGATGATGGAACAATAAATACAAC GTCTAGTGCCGATCATATTCAATAACCC |
| | 53R-in | TTTTCCATTTCAATTAACCGCAATGCGGATTACGTTGTAAATCAATCA GACTTATCGCCTTCATCGTATG |
| lptG | 54F-in | GTGCCGGTCCGCCGCCTGCGTGCCCGTTTTCTGCGTAAAGGAGCGGTA TACTAGTGCCGATCATATTCAATAACCC |
| | 54R-in | TCATGGTAGGCCTGATAAGCGCAGCGCCATCAGGCCTACGATTACTGT GACTTATCGCCTTCATCGTATG |

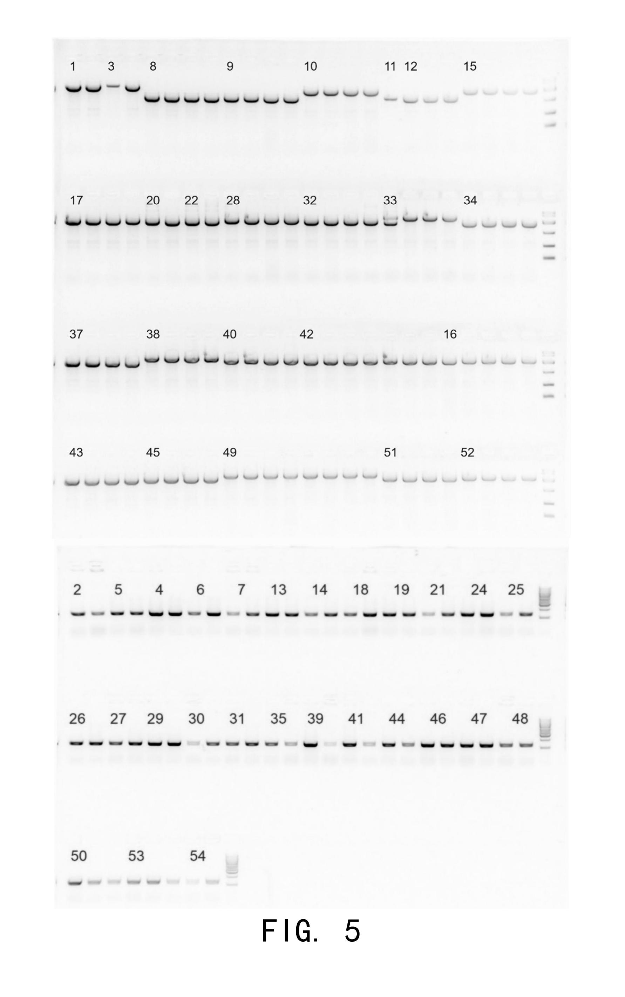

The target gene fragment constructed above was introduced into competent cells of strain DB-ZW1 by electrotransformation (E=18 KV/cm) through λ-red homologous recombination to obtain a pool of a series of strains ZW1(Δlps gene)-Fnr-SP-lps gene-KanR, that is, the gene at the original site in the bacterial genome was replaced with a hypoxia induced expression cassette. The PCR system and condition as described in Example 3 were used to carry out PCR using the identification primers ide-bb-fnr-F (CGAAATAGACAGATCGCTGA)/ide-bb-fnr-R (CTTCCCAACCTTACCAGAGG), and the amplification of the corresponding site sequence proved that the strain was successfully constructed. As shown in FIG. 5, strains for the hypoxia induced expression of each LPS-associated gene or murein-lipoprotein gene were successfully constructed.

### Example 6: Therapeutic Effect of Strains for the Hypoxia Induced Expression of LPS-Related Genes or Murein-lipoprotein Gene

6.1. The strains ZW1(Δmsbb)-Fnr-SP-mspb-KnaR and ZW1(ΔlppAB)-Fnr-SP-lppAB-KnaR obtained in Example 5.2 were used to treat MB49 subcutaneous tumor-bearing mouse model (1 × 10⁶ mouse MB49 bladder cancer cells were subcutaneously injected into mice (C57BL/6 purchased from Beijing Vital River Experimental Animal Technology Co., Ltd., with the body weight of about 18 g), and the mice were raised to a tumor volume of 150-200 mm³).

In particular, the strain was cultured overnight in a shaker at 37 °C at 150 rpm, transferred to fresh LB medium at a ratio of 1:100 and cultured to OD600=0.4-0.5, and the bacteria were collected and counted by flow cytometry. 1 × 10 ⁷ cfu of bacteria were suspended in 125µL of PBS and administered to mice by tail vein injection, with mice injected with ZW1(Δmsbb)-Fnr-SP-msbb-KnaR and ZW1(ΔlppAB)-Fnr-SP-lppAB-KnaR as experimental groups (n=5), mice injected with strain DB-ZW1 and ZW1(Δmsbb)- aadA or ZW1(ΔlppAB)-aadA as control groups (n=5), and mice injected with PBS as blank control groups (n=4). Tumor volume, body weight, and survival rate of tumor-bearing mice were observed 0, 1, 3, 5, 7, 9, 12 and 14 days post injection.

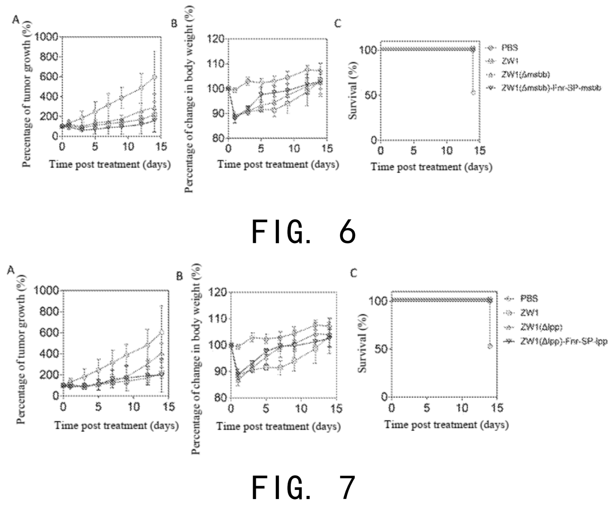

Within 14 days, the tumor volume of two mice in the blank control group exceeded the ethical value, which were thus considered dead. None of the other mice showed death (FIGS. 6C and 7C).

As shown in FIG. 6A, all of strains DB-ZW1, ZW1(Δmsbb)-aadA and ZW1(Δmsbb)-Fnr-SP-mspb-KnaR inhibited tumor growth, among which strain ZW1(Δmsbb)-aadA inhibited tumor growth less than strain DB-ZW1, while strain ZW1(Δmsbb)-Fnr-SP-mspb-KnaR showed the highest tumor inhibition.

As shown in FIG. 6B, the body weights of the mice injected with the three bacteria were all decreased within 1 day without significant difference. 1 day post injection of bacteria, the body weight recovery of mice injected with the strains DB-ZW1, ZW1(Δmsbb)-aadA, and ZW1(Δmsbb)-Fnr-SP-msbb-KnaR increased in the above order.

The results showed that the strain ZW1(Δmsbb)-Fnr-SP-msbb had the strongest inhibition to tumor growth and the lowest toxicity.

As shown in FIG. 7A, all of strains DB-ZW1, ZW1(ΔlppAB)aadA and ZW1(ΔlppAB)-Fnr-SP-lppAB-KnaR can inhibit tumor growth, among which the inhibitions of strains DB-ZW1 and ZW1(ΔlppAB)-Fnr-SP-lppAB-KnaR to tumor growth were not significantly different and were higher than that of strain ZW1(ΔlppAB)-aadA.

As shown in FIG. 7B, the mice injected with the three bacteria all decreased in body weight within 1 day without significant difference. 1 day post injection of bacteria, mice injected with strains ZW1(ΔlppAB)-aadA and ZW1(ΔlppAB)-Fnr-SP-lppAB-KnaR had no significant difference in body weight recovery and were both higher than mice injected with strain DB-ZW1. 14 day post injection, there was no significant difference in body weight of mice in each group, indicating that bacteria in normal tissues of mice were cleared.

The results showed that the ability of strain ZW1(ΔlppAB)-Fnr-SP-lppAB-KnaR for inhibiting tumor growth was comparable to that of DB-ZW1 with lower toxicity.

6.2. The strains prepared in Examples 5.4 were tested for therapeutic effect as described in Examples 6.1.

### List of Sequences

SEQ ID NO:1: PsseA-hlyA-loxp-KnaR-loxp 
TTCACAGAAAAGTGTTGCCCCCTTCCATGGCGGAAGGGGGACAAAGGTGAATTTGTCCTACTCAGGAGAGCGTTCACCGACAA
ACAACAGATAAAACGAAAGGCCCAGTCTTTCGACTGAGCCTTTCGTTTTATTTGATGCACTAGTGCCGATCATATTCAATAAC
CCTTAATATAACTTCGTATAATGTATGCTATACGAAGTTATTAGGTCTGAAGAGGAGTTTACGTCCCATATGGAGTCCCGCTC
AGAAGAACTCGTCAAGAAGGCGATAGAAGGCGATGCGCTGCGAATCGGGAGCGGCGATACCGTAAAGCACGAGGAAGCGGTCA
GCCCATTCGCCGCCAAGCTCTTCAGCAATATCACGGGTAGCCAACGCTATGTCCTGATAGCGGTCCGCCACACCCAGCCGGCC
ACAGTCGATGAATCCAGAAAAGCGGCCATTTTCCACCATGATATTCGGCAAGCAGGCATCGCCATGGGTCACGACGAGATCCT
CGCCGTCGGGCATGCGCGCCTTGAGCCTGGCGAACAGTTCGGCCGGCGCGAGCCCCTGATGCTCTTCGTCCAGATCATCCTGA
TCGACAAGACCGGCTTCCATCCGAGTACGTGCTCGCTCGATGCGATGTTTCGCTTGGTGGTCGAATGGGCAGGTAGCCGGATC
AAGCGTATGCAGCCGCCGCATTGCATCAGCCATGATGGATACTTTCTCGGCAGGAGCAAGGTGAGATGACAGGAGATCCTGCC
CCGGCACTTCGCCCAATAGCAGCCAGTCCCTTCCCGCTTCAGTGACAACGTCGAGCACAGCTGCGCAAGGAACGCCCGTCGTG
GCCAGCCACGATAGCCGCGCTGCCTCGTCCTGCAGTTCATTCAGGGCACCGGACAGGTCGGTCTTGACAAAAAGAACCGGGCG
CCCCTGCGCTGACAGCCGGAACACGGCGGCATCAGAGCAGCCGATTGTCTGTTGTGCCCAGTCATAGCCGAATAGCCTCTCCA
CCCAAGCGGCCGGAGAACCTGCGTGCAATCCATCTTGTTCAATCATGCGAAACGATCCTCATCCTGTCTCTTGATCAGATCCT
GATCCCCTGCGCCATCAGATCCTTGGCGGCAAGAAAGCCATCCAGTTTACTTTGCAGGGCTTCCCAACCTTACCAGAGGGCGC
CCCAGCTGGCAATTCCGACGTCATTGCGTTGCTAGCCAAGCTTAGGATCCGGAACCCTTAATATAACTTCGTATAATGTATGC
TATACGAAGTTATTAGGTCCCTCGAAGAGGTTCACTAGCTATCTAGAGCTTGGACTCCTGTTGATAGATCCAGTAATGACCTC
AGAACTCCATCTGGATTTGTTCAGAACGCTCGGTTGCCGCCGGGCGTTTTTTATTGGTGAGAATCCAAGCCCGGGAAACGGGA
TCCTGATCTTGATCAGGCTGTGGGTCAGGCCACTCTTGGCATCGACACCAATGTGGGCCTTCATGCCAAAGTGCCACTGATTG
CCTTTCTTGGTCTGATGCATCTCCGGATCGCGTTGCTGCTCTTTGTTCTTGGTCGAGCTGGGTGCCTCAATGATGGTGGCATC
GACCAAGGTGCCTTGAGTCATCATGACGCCTGCTTCGGCCAGCCAGCGATTGATGGTCTTGAACAATTGGCGGGCCAGTTGAT
GCTGCTCCAGCAGGTGGCGGAAATTCATGATGGTGGTGCGGTCCGGCAAGGCGCTATCCAGGGATAACCGGGCAAACAGACGC
ATGGAGGCGATTTCGTACAGAGCATCTTCCATCGCGCCATCGCTCAGGTTGTACCAATGCTGCATGCAGTGAATGCGTAGCAT
GGTTTCCAGCGGATAAGGTCGCCGGCCATTACCAGCCTTGGGGTAAAACGGCTCGATGACTTCCACCATGTTTTGCCATGGCA
GAATCTGCTCCATGCGGGACAAGAAAATCTCTTTTCTGGTCTGACGGCGCTTACTGCTGAATTCACTGTCGGCGAAGGTAAGT
TGATGACTCATGATGAACCCTGTTCTATGGCTCCAGATGACAAACATGATCTCATATCAGGGACTTGTTCGCACCTTCCTTAG
CACGTTAATTATCTATCGTGTATATGGAGGGGATCTAGAATGAAAAAAATAATGCTAGTTTTTATTACACTTATATTAGTTAG
TCTACCAATTGCGCAACAAACTGAAGCAAAGGATGCATCTGCATTCAATAAAGAAAATTCAATTTCATCCATGGCACCACCAG
CATCTCCGCCTGCAAGTCCTAAGACGCCAATCGAAAAGAAACACGCGGATGAAATCGATAAGTATATACAAGGATTGGACTAC
AATAAAAACAATGTATTAGTATACCACGGAGATGCAGTGACAAATGTGCCGCCAAGAAAAGGTTACAAAGATGGAAATGAATA
TATTGTTGTGGAGAAAAAGAAGAAATCCATCAATCAAAATAATGCAGACATTCAAGTTGTGAATGCAATTTCGAGCCTAACCT
ATCCAGGTGCTCTCGTAAAAGCGAATTCGGAATTAGTAGAAAATCAACCAGATGTTCTCCCTGTAAAACGTGATTCATTAACA
CTCAGCATTGATTTGCCAGGTATGACTAATCAAGACAATAAAATCGTTGTAAAAAATGCCACTAAATCAAACGTTAACAACGC
AGTAAATACATTAGTGGAAAGATGGAATGAAAAATATGCTCAAGCTTATCCAAATGTAAGTGCAAAAATTGATTATGATGACG
AAATGGCTTACAGTGAATCACAATTAATTGCGAAATTTGGTACAGCATTTAAAGCTGTAAATAATAGCTTGAATGTAAACTTC
GGCGCAATCAGTGAAGGGAAAATGCAAGAAGAAGTCATTAGTTTTAAACAAATTTACTATAACGTGAATGTTAATGAACCTAC
AAGACCTTCCAGATTTTTCGGCAAAGCTGTTACTAAAGAGCAGTTGCAAGCGCTTGGAGTGAATGCAGAAAATCCTCCTGCAT
ATATCTCAAGTGTGGCGTATGGCCGTCAAGTTTATTTGAAATTATCAACTAATTCCCATAGTACTAAAGTAAAAGCTGCTTTT
GATGCTGCCGTAAGCGGAAAATCTGTCTCAGGTGATGTAGAACTAACAAATATCATCAAAAATTCTTCCTTCAAAGCCGTAAT
TTACGGAGGTTCCGCAAAAGATGAAGTTCAAATCATCGACGGCAACCTCGGAGACTTACGAGATATTTTGAAAAAAGGCGCTA
CTTTTAATCGAGAAACACCAGGAGTTCCCATTGCTTATACAACAAACTTCCTAAAAGACAATGAATTAGCTGTTATTAAAAAC
AACTCAGAATATATTGAAACAACTTCAAAAGCTTATACAGATGGAAAAATTAACATCGATCACTCTGGAGGATACGTTGCTCA
ATTCAACATTTCTTGGGATGAAGTAAATTATGATCCTGAAGGTAACGAAATTGTTCAACATAAAAACTGGAGCGAAAACAATA
AAAGTAAGCTAGCTCATTTCACATCGTCCATCTATTTGCCAGGTAACGCGAGAAATATTAATGTTTACGCTAAAGAATGCACT
GGTTTAGCTTGGGAATGGTGGAGAACGGTAATTGATGACCGGAACTTACCACTTGTGAAAAATAGAAATATCTCCATCTGGGG
CACCACGCTTTATCCGAAATATAGTAATAAAGTAGATAATCCAATCGAATAAGTCGACCTCGAGGACTAGTGAACCTCTTCGA
GGGACCTAATAACTTCGTATAGCATACATTATACGAAGTTATATTAAGGGTTATTGAATATGATCGGCTCGAGATTACTTCGC
ATTGCAATCAGCTACGGACTCAAGCTTACGTCAGGTCATACGATGAAGGCGATAAGTCGCGTAAGTTGCTAGCCCACGCGGCT
CACCTTCACGGGTGGGCCTTTCTTCGGTGTTTCAATCTCGATTAACAGGTAACGCAAAATTGCTCTGTACGTCAGA
SEQ ID NO:2: Pssbp1-dapE-Cm 
TTGGTTTCAGTGAATCCCGTTATCAGCAGTTTTTTGATGAGGTGTAGTCTATTTGTCCTACTCAGGAGAGCGTTCACCGACAA
ACAACAGATAAAACGAAAGGCCCAGTCTTTCGACTGAGCCTTTCGTTTTATTTGATGCACTAGTGCCGATCATATTCAATAAC
CCTTAATATAACTTCGTATAATGTATGCTATACGAAGTTATTAGGTCTGAAGAGGAGTTTACGTCCCATATGGAGTCCCGCTT
ACGCCCCGCCCTGCCACTCATCGCAGTACTGTTGTAATTCATTAAGCATTCTGCCGACATGGAAGCCATCACAGACGGCATGA
TGAACCTGAATCGCCAGCGGCATCAGCACCTTGTCGCCTTGCGTATAATATTTGCCCATGGTGAAAACGGGGGCGAAGAAGTT
GTCCATATTGGCCACGTTTAAATCAAAACTGGTGAAACTCACCCAGGGATTGGCTGAGACGAAAAACATATTCTCAATAAACC
CTTTAGGGAAATAGGCCAGGTTTTCACCGTAACACGCCACATCTTGCGAATATATGTGTAGAAACTGCCGGAAATCGTCGTGG
TATTCACTCCAGAGCGATGAAAACGTTTCAGTTTGCTCATGGAAAACGGTGTAACAAGGGTGAACACTATCCCATATCACCAG
CTCACCGTCTTTCATTGCCATACGAAATTCCGGATGAGCATTCATCAGGCGGGCAAGAATGTGAATAAAGGCCGGATAAAACT
TGTGCTTATTTTTCTTTACGGTCTTTAAAAAGGCCGTAATATCCAGCTGAACGGTCTGGTTATAGGTACATTGAGCAACTGAC
TGAAATGCCTCAAAATGTTCTTTACGATGCCATTGGGATATATCAACGGTGGTATATCCAGTGATTTTTTTCTCCATGCGAAA
CGATCCTCATCCTGTCTCTTGATCAGATCCTGATCCCCTGCGCCATCAGATCCTTGGCGGCAAGAAAGCCATCCAGTTTACTT
TGCAGGGCTTCCCAACCTTACCAGAGGGCGCCCCAGCTGGCAATTCCGACGTCATTGCGTTGCTAGCCAAGCTTAGGATCCGG
AACCCTTAATATAACTTCGTATAATGTATGCTATACGAAGTTATTAGGTCCCTCGAAGAGGTTCACTAGCTATCTAGAGCTTG
GACTCCTGTTGATAGATCCAGTAATGACCTCAGAACTCCATCTGGATTTGTTCAGAACGCTCGGTTGCCGCCGGGCGTTTTTT
ATTGGTGAGAATCCAAGCCCGGGAAACGGGATCCTGATCTTGATCAACCGAGGTCACAACATAGTAAAAGCGCTATTGGTAAT
GGTACAATCGCGCGTTTACACTTATTCAGAACGATTTTTTTCAGGAGGGCCTACTTGTCACACAGGACGGTAATGTCGTGCCC
 
GGTTATTGAGCTGACACAGCAACTTATTCGTCGCCCCTCCCTGAGTCCGGATGACGCAGGCTGTCAGGCATTAATGATTGAAC
GCCTGCGTAAAATCGGTTTTACCATTGAGCATATGGATTTTGGCGACACGCAAAATTTTTGGGCATGGCGTGGGCGTGGCGAA
ACGCTGGCGTTCGCCGGGCATACTGACGTCGTACCGGCTGGCGATGTCGATCGCTGGATCAATCCGCCGTTTGAGCCTACGAT
TCGCGACGGAATGTTGTTCGGTCGCGGCGCGGCGGATATGAAAGGTTCACTGGCGGCCATGGTGGTGGCTGCTGAACGTTTTG
TTGCGCAGCATCCTCATCACCGGGGCCGCCTCGCATTTTTGATTACCTCTGATGAGGAAGCCAGCGCAAAAAATGGCACCGTA
AAGGTGGTTGAAGCGCTGATGGCCCGTAACGAGCGGCTGGATTACTGTCTGGTCGGCGAACCTTCCAGTACTGAAATTGTGGG
CGATGTCGTCAAAAACGGGCGTCGCGGTTCTCTCACCTGTAACCTCACTATCCATGGCGTTCAGGGGCACGTCGCCTATCCGC
ATCTGGCGGATAACCCTGTTCATCGCGCCGCGCCTTTTCTTAATGAACTGGTCGCTATCGAGTGGGATCGGGGCAATGACTTT
TTCCCGGCCACCAGTATGCAGGTAGCCAATATCCAGGCAGGCACCGGCAGCAATAATGTCATTCCCGGCGAACTGTTCGTGCA
GTTTAACTTCCGTTTCAGCACGGAACTGACCGATGAGATGATCAAAGAGCGGGTACATGCGCTGCTTGAAAAGCATCAGCTAC
GCTATACCGTAGACTGGTGGCTTTCCGGGCAGCCGTTCCTGACCGCACGCGGCAAGCTGGTGGACGCGGTGGTAAACGCCATT
GAGCACTATAATGAAATTAAACCGCAATTACTGACGACGGGCGGTACGTCCGACGGACGTTTTATCGCCCGTATGGGAGCGCA
GGTGGTAGAACTGGGGCCGGTGAACGCCACCATTCATAAAATTAATGAATGTGTGAATGCCGCCGATCTCCAGCTACTGGCCC
GTATGTATCAACGCATTATGGAACAGCTCGTCGCCCATCACCATCACCATCACTAAGATTACTTCGCATTGCAATCAGCTACG
GACTCAAGCTTACGTCAGGTCATACGATGAAGGCGATAAGTCGCGTAAGTTGCTAGCCCACGCGGCTCACCTTCACGGGTGGG
CCTTTCTTCGTGAGCGTTCCGGTAAGAGGAAATAAGCATGGACTGGCTGGCAAAATATTG
SEQ ID NO:3: Plasmid PsseA-hlyA-loxp-KnaR-loxp 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCGACTTCCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
CAACCATACCGTCCGTTCTTTCCTTCTAGCGTTTTCAATCGTGGCGTTCACTAGTGCCACACAGCATAAAATTAGCTTGCTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTACTGTCTAGGGCGGCGGATTTGTCCTACTCAGGAGAGCGTTCACCGACAAACAACAGATA
AAACGAAAGGCCCAGTCTTTCGACTGAGCCTTTCGTTTTATTTGATGCACTAGTGCCGATCATATTCAATAACCCTTAATATA
ACTTCGTATAATGTATGCTATACGAAGTTATTAGGTCTGAAGAGGAGTTTACGTCCCATATGGAGTCCCGCTCAGAAGAACTC
GTCAAGAAGGCGATAGAAGGCGATGCGCTGCGAATCGGGAGCGGCGATACCGTAAAGCACGAGGAAGCGGTCAGCCCATTCGC
CGCCAAGCTCTTCAGCAATATCACGGGTAGCCAACGCTATGTCCTGATAGCGGTCCGCCACACCCAGCCGGCCACAGTCGATG
AATCCAGAAAAGCGGCCATTTTCCACCATGATATTCGGCAAGCAGGCATCGCCATGGGTCACGACGAGATCCTCGCCGTCGGG
CATGCGCGCCTTGAGCCTGGCGAACAGTTCGGCCGGCGCGAGCCCCTGATGCTCTTCGTCCAGATCATCCTGATCGACAAGAC
CGGCTTCCATCCGAGTACGTGCTCGCTCGATGCGATGTTTCGCTTGGTGGTCGAATGGGCAGGTAGCCGGATCAAGCGTATGC
AGCCGCCGCATTGCATCAGCCATGATGGATACTTTCTCGGCAGGAGCAAGGTGAGATGACAGGAGATCCTGCCCCGGCACTTC
GCCCAATAGCAGCCAGTCCCTTCCCGCTTCAGTGACAACGTCGAGCACAGCTGCGCAAGGAACGCCCGTCGTGGCCAGCCACG
ATAGCCGCGCTGCCTCGTCCTGCAGTTCATTCAGGGCACCGGACAGGTCGGTCTTGACAAAAAGAACCGGGCGCCCCTGCGCT
GACAGCCGGAACACGGCGGCATCAGAGCAGCCGATTGTCTGTTGTGCCCAGTCATAGCCGAATAGCCTCTCCACCCAAGCGGC
CGGAGAACCTGCGTGCAATCCATCTTGTTCAATCATGCGAAACGATCCTCATCCTGTCTCTTGATCAGATCCTGATCCCCTGC
GCCATCAGATCCTTGGCGGCAAGAAAGCCATCCAGTTTACTTTGCAGGGCTTCCCAACCTTACCAGAGGGCGCCCCAGCTGGC
AATTCCGACGTCATTGCGTTGCTAGCCAAGCTTAGGATCCGGAACCCTTAATATAACTTCGTATAATGTATGCTATACGAAGT
TATTAGGTCCCTCGAAGAGGTTCACTAGCTATCTAGAGCTTGGACTCCTGTTGATAGATCCAGTAATGACCTCAGAACTCCAT
CTGGATTTGTTCAGAACGCTCGGTTGCCGCCGGGCGTTTTTTATTGGTGAGAATCCAAGCCCGGGAAACGGGATCCTGATCTT
GATCAGGCTGTGGGTCAGGCCACTCTTGGCATCGACACCAATGTGGGCCTTCATGCCAAAGTGCCACTGATTGCCTTTCTTGG
TCTGATGCATCTCCGGATCGCGTTGCTGCTCTTTGTTCTTGGTCGAGCTGGGTGCCTCAATGATGGTGGCATCGACCAAGGTG
 
CCTTGAGTCATCATGACGCCTGCTTCGGCCAGCCAGCGATTGATGGTCTTGAACAATTGGCGGGCCAGTTGATGCTGCTCCAG
CAGGTGGCGGAAATTCATGATGGTGGTGCGGTCCGGCAAGGCGCTATCCAGGGATAACCGGGCAAACAGACGCATGGAGGCGA
TTTCGTACAGAGCATCTTCCATCGCGCCATCGCTCAGGTTGTACCAATGCTGCATGCAGTGAATGCGTAGCATGGTTTCCAGC
GGATAAGGTCGCCGGCCATTACCAGCCTTGGGGTAAAACGGCTCGATGACTTCCACCATGTTTTGCCATGGCAGAATCTGCTC
CATGCGGGACAAGAAAATCTCTTTTCTGGTCTGACGGCGCTTACTGCTGAATTCACTGTCGGCGAAGGTAAGTTGATGACTCA
TGATGAACCCTGTTCTATGGCTCCAGATGACAAACATGATCTCATATCAGGGACTTGTTCGCACCTTCCTTAGCACGTTAATT
ATCTATCGTGTATATGGAGGGGATCTAGAATGAAAAAAATAATGCTAGTTTTTATTACACTTATATTAGTTAGTCTACCAATT
GCGCAACAAACTGAAGCAAAGGATGCATCTGCATTCAATAAAGAAAATTCAATTTCATCCATGGCACCACCAGCATCTCCGCC
TGCAAGTCCTAAGACGCCAATCGAAAAGAAACACGCGGATGAAATCGATAAGTATATACAAGGATTGGACTACAATAAAAACA
ATGTATTAGTATACCACGGAGATGCAGTGACAAATGTGCCGCCAAGAAAAGGTTACAAAGATGGAAATGAATATATTGTTGTG
GAGAAAAAGAAGAAATCCATCAATCAAAATAATGCAGACATTCAAGTTGTGAATGCAATTTCGAGCCTAACCTATCCAGGTGC
TCTCGTAAAAGCGAATTCGGAATTAGTAGAAAATCAACCAGATGTTCTCCCTGTAAAACGTGATTCATTAACACTCAGCATTG
ATTTGCCAGGTATGACTAATCAAGACAATAAAATCGTTGTAAAAAATGCCACTAAATCAAACGTTAACAACGCAGTAAATACA
TTAGTGGAAAGATGGAATGAAAAATATGCTCAAGCTTATCCAAATGTAAGTGCAAAAATTGATTATGATGACGAAATGGCTTA
CAGTGAATCACAATTAATTGCGAAATTTGGTACAGCATTTAAAGCTGTAAATAATAGCTTGAATGTAAACTTCGGCGCAATCA
GTGAAGGGAAAATGCAAGAAGAAGTCATTAGTTTTAAACAAATTTACTATAACGTGAATGTTAATGAACCTACAAGACCTTCC
AGATTTTTCGGCAAAGCTGTTACTAAAGAGCAGTTGCAAGCGCTTGGAGTGAATGCAGAAAATCCTCCTGCATATATCTCAAG
TGTGGCGTATGGCCGTCAAGTTTATTTGAAATTATCAACTAATTCCCATAGTACTAAAGTAAAAGCTGCTTTTGATGCTGCCG
TAAGCGGAAAATCTGTCTCAGGTGATGTAGAACTAACAAATATCATCAAAAATTCTTCCTTCAAAGCCGTAATTTACGGAGGT
TCCGCAAAAGATGAAGTTCAAATCATCGACGGCAACCTCGGAGACTTACGAGATATTTTGAAAAAAGGCGCTACTTTTAATCG
AGAAACACCAGGAGTTCCCATTGCTTATACAACAAACTTCCTAAAAGACAATGAATTAGCTGTTATTAAAAACAACTCAGAAT
ATATTGAAACAACTTCAAAAGCTTATACAGATGGAAAAATTAACATCGATCACTCTGGAGGATACGTTGCTCAATTCAACATT
TCTTGGGATGAAGTAAATTATGATCCTGAAGGTAACGAAATTGTTCAACATAAAAACTGGAGCGAAAACAATAAAAGTAAGCT
AGCTCATTTCACATCGTCCATCTATTTGCCAGGTAACGCGAGAAATATTAATGTTTACGCTAAAGAATGCACTGGTTTAGCTT
GGGAATGGTGGAGAACGGTAATTGATGACCGGAACTTACCACTTGTGAAAAATAGAAATATCTCCATCTGGGGCACCACGCTT
TATCCGAAATATAGTAATAAAGTAGATAATCCAATCGAATAAGTCGACCTCGAGGACTAGTGAACCTCTTCGAGGGACCTAAT
AACTTCGTATAGCATACATTATACGAAGTTATATTAAGGGTTATTGAATATGATCGGCTCGAGATTACTTCGCATTGCAATCA
GCTACGGACTCAAGCTTACGTCAGGTCATACGATGAAGGCGATAAGTCGCGTAAGTTGCTAGCCCACGCGGCTCACCTTCACG
GGTGGGCCTTTCTTCGCGATCCACATG
SEQ ID NO:4: Plasmid Pssbp1-dapE-Cm 
CATGGTCATAGCTGTTTCCTTTAATGCCCATGGCGTTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGGGCTTACCATCTGGCCCCAGTGCTGCA
ATGATACCGCGACTTCCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATAGTTTGC
GCAACGTTGTTGCCATTGCTACAGGCATCGTGGTGTCACGCTCGTCGTTTGGTATGGCTTCATTCAGCTCCGGTTCCCAACGA
TCAAGGCGAGTTACATGATCCCCCATGTTGTGCAAAAAAGCGGTTAGCTCCTTCGGTCCTCCGATCGTTGTCAGAAGTAAGTT
GGCCGCAGTGTTATCACTCATGGTTATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTCTTGCCCGGCGTCAATACGGGATAAT
ACCGCGCCACATAGCAGAACTTTAAAAGTGCTCATCATTGGAAAACGTTCTTCGGGGCGAAAACTCTCAAGGATCTTACCGCT
GTTGAGATCCAGTTCGATGTAACCCACTCGTGCACCCAACTGATCTTCAGCATCTTTTACTTTCACCAGCGTTTCTGGGTGAG
CAAAAACAGGAAGGCAAAATGCCGCAAAAAAGGGAATAAGGGCGACACGGAAATGTTGAATACTCATACTCTTCCTTTTTCAA
TATTATTGAAGCATTTATCAGGGTTATTGTCTCATGAGCGGATACATATTTGAATGTATTTAGAAAAATAAACAAATAGGGGT
TCCGCGCACATTTCCCCGAAAAGTGCCACCTGACGTCTAAGAAACCATTATTATCATGACATTAACCTATAAAAATAGGCGTA
TCACGAGGCCCTTAGGCCAAATAGGCCGTTCAGATCCTTCCGTATTTAGCCAGTATGTTCTCTAGTGTGGTTCGTTGTTTTTG
CGTGAGCCATGAGAACGAACCATTGAGATCATGCTTACTTTGCATGTCACTCAAAAATTTTGCCTCAAAACTGGTGAGCTGAA
TTTTTGCAGTTAAAGCATCGTGTAGTGTTTTTCTTAGTCCGTTACGTAGGTAGGAATCTGATGTAATGGTTGTTGGTATTTTG
TCACCATTCATTTTTATCTGGTTGTTCTCAAGTTCGGTTACGAGATCCATTTGTCTATCTAGTTCAACTTGGAAAATCAACGT
ATCAGTCGGGCGGCCTCGCTTATCAACCACCAATTTCATATTGCTGTAAGTGTTTAAATCTTTACTTATTGGTTTCAAAACCC
ATTGGTTAAGCCTTTTAAACTCATGGTAGTTATTTTCAAGCATTAACATGAACTTAAATTCATCAAGGCTAATCTCTATATTT
GCCTTGTGAGTTTTCTTTTGTGTTAGTTCTTTTAATAACCACTCATAAATCCTCATAGAGTATTTGTTTTCAAAAGACTTAAC
ATGTTCCAGATTATATTTTATGAATTTTTTTAACTGGAAAAGATAAGGCAATATCTCTTCACTAAAAACTAATTCTAATTTTT
CGCTTGAGAACTTGGCATAGTTTGTCCACTGGAAAATCTCAAAGCCTTTAACCAAAGGATTCCTGATTTCCACAGTTCTCGTC
ATCAGCTCTCTGGTTGCTTTAGCTAATACACCATAAGCATTTTCCCTACTGATGTTCATCATCTGAGCGTATTGGTTATAAGT
GAACGATACCGTCCGTTCTTTCCTTGTAGGGTTTTCAATCGTGGGGTTGAGTAGTGCCACACAGCATAAAATTAGCTTGGTTT
CATGCTCCGTTAAGTCATAGCGACTAATCGCTAGTTCATTTGCTTTGAAAACAACTAATTCAGACATACATCTCAATTGGTCT
AGGTGATTTTAATCACTATACCAATTGAGATGGGCTAGTCAATGATAATTACTAGTCCTTTTCCTTTGAGTTGTGGGTATCTG
TAAATTCTGCTAGACCTTTGCTGGAAAACTTGTAAATTCTGCTAGACCCTCTGTAAATTCCGCTAGACCTTTGTGTGTTTTTT
TTGTTTATATTCAAGTGGTTATAATTTATAGAATAAAGAAAGAATAAAAAAAGATAAAAAGAATAGATCCCAGCCCTGTGTAT
AACTCACTACTTTAGTCAGTTCCGCAGTATTACAAAAGGATGTCGCAAACGCTGTTTGCTCCTCTACAAAACAGACCTTAAAA
CCCTAAAGGCTTAAGTAGCACCCTCGCAAGCTCGGGCAAATCGCTGAATATTCCTTTTGTCTCCGACCATCAGGCACCTGAGT
CGCTGTCTTTTTCGTGACATTCAGTTCGCTGCGCTCACGGCTCTGGCAGTGAATGGGGGTAAATGGCACTACAGGCGCCTTTT
ATGGATTCATGCAAGGAAACTACCCATAATACAAGAAAAGCCCGTCACGGGCTTCTCAGGGCGTTTTATGGCGGGTCTGCTAT
GTGGTGCTATCTGACTTTTTGCTGTTCAGCAGTTCCTGCCCTCTGATTTTCCAGTCTGACCACTTCGGATTATCCCGTGACAG
GTCATTCAGACTGGCTAATGCACCCAGTAAGGCAGCGGTATCATCAACAGGCTTACCCGTCTTACTGTCAAGAGGACATCCGG
TCAGATAACCGTCATCTTCGGCTACTGTCTAGGGCGGCGGATTTGTCCTACTCAGGAGAGCGTTCACCGACAAACAACAGATA
AAACGAAAGGCCCAGTCTTTCGACTGAGCCTTTCGTTTTATTTGATGCACTAGTGCCGATCATATTCAATAACCCTTAATATA
ACTTCCTATAATCTATCCTATACCAACTTATTACGCTCAAGACCACTTTACCTCCCATATCCAGTCCCGCTTACGCCCCCCC
CTGCCACTCATCGCAGTACTGTTGTAATTCATTAAGCATTCTGCCGACATGGAAGCCATCACAGACGGCATGATGAACCTGAA
TCGCCAGCGGCATCAGCACCTTGTCGCCTTGCGTATAATATTTGCCCATGGTGAAAACGGGGGCGAAGAAGTTGTCCATATTG
GCCACGTTTAAATCAAAACTGGTGAAACTCACCCAGGGATTGGCTGAGACGAAAAACATATTCTCAATAAACCCTTTAGGGAA
 
ATAGGCCAGGTTTTCACCGTAACACGCCACATCTTGCGAATATATGTGTAGAAACTGCCGGAAATCGTCGTGGTATTCACTCC
AGAGCGATGAAAACGTTTCAGTTTGCTCATGGAAAACGGTGTAACAAGGGTGAACACTATCCCATATCACCAGCTCACCGTCT
TTCATTGCCATACGAAATTCCGGATGAGCATTCATCAGGCGGGCAAGAATGTGAATAAAGGCCGGATAAAACTTGTGCTTATT
TTTCTTTACGGTCTTTAAAAAGGCCGTAATATCCAGCTGAACGGTCTGGTTATAGGTACATTGAGCAACTGACTGAAATGCCT
CAAAATGTTCTTTACGATGCCATTGGGATATATCAACGGTGGTATATCCAGTGATTTTTTTCTCCATTTTAGCTTCCTTAGCT
CCTGAAAATCTCGATAACTCAAAAAATACGCCCGGTAGTGATCTTATTTCATTATGGTGAAAGTTGGAACCTCTTACGTGCCG
ATCAGACGTCATTGCGTTGCTAGCCAAGCTTAGGATCCGGAACCCTTAATATAACTTCGTATAATGTATGCTATACGAAGTTA
TTAGGTCCCTCGAAGAGGTTCACTAGCTATCTAGAGCTTGGACTCCTGTTGATAGATCCAGTAATGACCTCAGAACTCCATCT
GGATTTGTTCAGAACGCTCGGTTGCCGCCGGGCGTTTTTTATTGGTGAGAATCCAAGCCCGGGAAACGGGATCCTGATCTTGA
TCAACCGAGGTCACAACATAGTAAAAGCGCTATTGGTAATGGTACAATCGCGCGTTTACACTTATTCAGAACGATTTTTTTCA
GGAGGGCCTACTTGTCACACAGGACGGTAATGTCGTGCCCGGTTATTGAGCTGACACAGCAACTTATTCGTCGCCCCTCCCTG
AGTCCGGATGACGCAGGCTGTCAGGCATTAATGATTGAACGCCTGCGTAAAATCGGTTTTACCATTGAGCATATGGATTTTGG
CGACACGCAAAATTTTTGGGCATGGCGTGGGCGTGGCGAAACGCTGGCGTTCGCCGGGCATACTGACGTCGTACCGGCTGGCG
ATGTCGATCGCTGGATCAATCCGCCGTTTGAGCCTACGATTCGCGACGGAATGTTGTTCGGTCGCGGCGCGGCGGATATGAAA
GGTTCACTGGCGGCCATGGTGGTGGCTGCTGAACGTTTTGTTGCGCAGCATCCTCATCACCGGGGCCGCCTCGCATTTTTGAT
TACCTCTGATGAGGAAGCCAGCGCAAAAAATGGCACCGTAAAGGTGGTTGAAGCGCTGATGGCCCGTAACGAGCGGCTGGATT
ACTGTCTGGTCGGCGAACCTTCCAGTACTGAAATTGTGGGCGATGTCGTCAAAAACGGGCGTCGCGGTTCTCTCACCTGTAAC
CTCACTATCCATGGCGTTCAGGGGCACGTCGCCTATCCGCATCTGGCGGATAACCCTGTTCATCGCGCCGCGCCTTTTCTTAA
TGAACTGGTCGCTATCGAGTGGGATCGGGGCAATGACTTTTTCCCGGCCACCAGTATGCAGGTAGCCAATATCCAGGCAGGCA
CCGGCAGCAATAATGTCATTCCCGGCGAACTGTTCGTGCAGTTTAACTTCCGTTTCAGCACGGAACTGACCGATGAGATGATC
AAAGAGCGGGTACATGCGCTGCTTGAAAAGCATCAGCTACGCTATACCGTAGACTGGTGGCTTTCCGGGCAGCCGTTCCTGAC
CGCACGCGGCAAGCTGGTGGACGCGGTGGTAAACGCCATTGAGCACTATAATGAAATTAAACCGCAATTACTGACGACGGGCG
GTACGTCCGACGGACGTTTTATCGCCCGTATGGGAGCGCAGGTGGTAGAACTGGGGCCGGTGAACGCCACCATTCATAAAATT
AATGAATGTGTGAATGCCGCCGATCTCCAGCTACTGGCCCGTATGTATCAACGCATTATGGAACAGCTCGTCGCCCATCACCA
TCACCATCACTAAGATTACTTCGCATTGCAATCAGCTACGGACTCAAGCTTACGTCAGGTCATACGATGAAGGCGATAAGTCG
CGTAAGTTGCTAGCCCACGCGGCTCACCTTCACGGGTGGGCCTTTCTTCGCGATCCACATG
SEQ ID NO:5: PsseA-hlyA-loxp-KnaR-loxp forward primer
   TTCACAGAAAAGTGTTGCCCCCTTCCATGGCGGAAGGGGGACAAAGGTGAATTTGTCCTACTCAGGAGAGCGTTCA
SEQ ID NO:6: PsseA-hlyA-loxp-KnaR-loxp reverse primer
   TCTGACGTACACAGCAATTTTGCGTTACCTGTTAATCGAGATTGAAACACCGAAGAAAGGCCCACCCG
SEQ ID NO:7: Pssbp1-dapE-Cm forward primer
   TTGGTTTCAGTGAATCCCGTTATCAGCAGTTTTTTGATGAGGTGTAGTCTATTTGTCCTACTCAGGAGAGCG
SEQ ID NO:8: Pssbp1-dapE-Cm reverse primer
   CAATATTTTGCCAGCCAGTCCATGCTTATTTCCTCTTACCGGAACGCTCACGAAGAAAGGCCCACCCG
SEQ ID NO:9: Promoter pepTp 
GCAGGGGTAAAAGTGACCTGACGCAATATTTGTCTTTTCTTGCTTCTTAATAATGTTGTCACAAAAAGTGAGGGTGACTACAT
GG
SEQ ID NO:10: Promoter fnrSp 
CCCCCCAAACTTTCAGCCAAGTCAATAAACTCTCTACCCATTCAGGCCAATATCTCTCTTGCAGCTCAATGCAACGTCAACCC
AT
SEQ ID NO:11: Promoter ysgAp 
TCAGAAGAAGCAAAAAGACACTTTACCGAAGGGTTTAACATTTTTTCGTGATACTCATCACCATGACGCAAATGCGTTGCATA
AA
SEQ ID NO:12: Promoter ssbp1 
AACCCACCTCACAACATAGTAAAACCCCTATTCGTAATCGTACAATCCCCGTTTACACTTATTCACAACCATTTTTTTCACC
AG
SEQ ID NO:13: Promoter Hip1
   GGATAAAAGTGACCTGACGCAATATTTGTCTTTTCTTGCTTAATAATGTTGTCA
SEQ ID NO:14: Promoter BBa_I14018
   TGTAAGTTTATACATAGGCGAGTACTCTGTTATGG
SEQ ID NO:15: Promoter BBa_R1074
   TTAAATTTCCTCTCGTCAGGCCGGAATAACTCCCTATAATGCGCCACCACACTGATAGTGCTAGTGTAGATCAC
SEQ ID NO:16: Promoter Ptet-Fnr
   AAAATTGATCTGAATCAATATTTTGACAAAAATTGATCTGAATCAATATTTACTGAGC
SEQ ID NO:17: Promoter Ptet-arcA
   GTTAATAAAATGTTATTGACAGTTAATAAAATGTTATACTGAGC
SEQ ID NO:18: Promoter PsseA
   TTAGCACGTTAATTATCTATCGTGTATATG
SEQ ID NO: 19: dapE (encoding succinyl-diaminoheptanedioate desuccinylase, Gene ID: 1254005) 
ATGTCGTGCCCGGTTATTGAGCTGACACAGCAACTTATTCGTCGCCCCTCCCTGAGTCCGGATGACGCAGGCTGTCAGGCATT
AATGATTGAACGCCTGCGTAAAATCGGTTTTACCATTGAGCATATGGATTTTGGCGACACGCAAAATTTTTGGGCATGGCGTG
GGCGTGGCGAAACGCTGGCGTTCGCCGGGCATACTGACGTCGTACCGGCTGGCGATGTCGATCGCTGGATCAATCCGCCGTTT
GAGCCTACGATTCGCGACGGAATGTTGTTCGGTCGCGGCGCGGCGGATATGAAAGGTTCACTGGCGGCCATGGTGGTGGCTGC
TGAACGTTTTGTTGCGCAGCATCCTCATCACCGGGGCCGCCTCGCATTTTTGATTACCTCTGATGAGGAAGCCAGCGCAAAAA
ATGGCACCGTAAAGGTGGTTGAAGCGCTGATGGCCCGTAACGAGCGGCTGGATTACTGTCTGGTCGGCGAACCTTCCAGTACT
GAAATTGTGGGCGATGTCGTCAAAAACGGGCGTCGCGGTTCTCTCACCTGTAACCTCACTATCCATGGCGTTCAGGGGCACGT
CGCCTATCCGCATCTGGCGGATAACCCTGTTCATCGCGCCGCGCCTTTTCTTAATGAACTGGTCGCTATCGAGTGGGATCGGG
GCAATGACTTTTTCCCGGCCACCAGTATGCAGGTAGCCAATATCCAGGCAGGCACCGGCAGCAATAATGTCATTCCCGGCGAA
CTGTTCGTGCAGTTTAACTTCCGTTTCAGCACGGAACTGACCGATGAGATGATCAAAGAGCGGGTACATGCGCTGCTTGAAAA
GCATCAGCTACGCTATACCGTAGACTGGTGGCTTTCCGGGCAGCCGTTCCTGACCGCACGCGGCAAGCTGGTGGACGCGGTGG
TAAACGCCATTGAGCACTATAATGAAATTAAACCGCAATTACTGACGACGGGCGGTACGTCCGACGGACGTTTTATCGCCCGT
ATGGGAGCGCAGGTGGTAGAACTGGGGCCGGTGAACGCCACCATTCATAAAATTAATGAATGTGTGAATGCCGCCGATCTCCA
GCTACTGGCCCGTATGTATCAACGCATTATGGAACAGCTCGTCGCCTGA
SEQ ID NO: 20: htrA (encoding serine protease, Gene ID: 1251727) 
ATGAAAAAAACCACATTAGCAATGAGTGCACTGGCTCTGAGTTTAGGTTTGGCATTGTCGCCTCTGTCTGCCACGGCGGCTGA
AACGTCCTCTTCAGCAATGACTGCCCAGCAGATGCCAAGCCTGGCACCGATGCTCGAAAAAGTGATGCCATCGGTGGTCAGTA
TTAATGTTGAAGGTAGCACCACGGTGAATACGCCGCGTATGCCGCGTAATTTCCAGCAGTTCTTTGGCGATGACTCCCCGTTC
TGCCAGGACGGTTCTCCGTTCCAGAATTCTCCGTTCTGCCAGGGCGGCGGTAACGGCGGCAACGGCGGTCAACAACAGAAATT
CATGGCGCTGGGCTCCGGCGTAATTATTGACGCCGCGAAGGGCTACGTCGTCACCAACAACCACGTGGTTGATAACGCCAGCG
TGATTAAAGTACAGCTTAGCGATGGGCGTAAATTCGATGCTAAAGTGGTGGGCAAAGATCCGCGTTCTGATATCGCGCTGATT
CAAATTCAGAATCCGAAGAACCTGACGGCGATTAAGCTGGCGGACTCCGACGCGCTGCGCGTGGGGGATTATACCGTCGCTAT
TGGTAACCCGTTTGGTCTGGGCGAAACGGTGACGTCAGGTATCGTTTCGGCGCTGGGGCGTAGCGGCCTGAACGTAGAAAATT
ACGAGAACTTTATTCAGACCGACGCCGCGATTAACCGTGGTAACTCCGGCGGCGCGCTGGTGAACCTGAACGGTGAGCTGATC
GGTATTAACACCGCGATTCTGGCGCCGGACGGCGGCAACATCGGTATCGGCTTCGCTATCCCCAGTAACATGGTGAAAAACCT
GACGTCGCAGATGGTGGAATACGGCCAGGTGAAACGCGGCGAACTGGGGATCATGGGGACTGAGCTGAATTCCGAATTGGCGA
AAGCGATGAAAGTCGACGCCCAGCGAGGCGCGTTCGTCAGCCAGGTGATGCCGAATTCGTCCGCGGCGAAAGCGGGTATCAAA
GCCGGGGATGTCATTACCTCGCTGAACGGTAAACCGATCAGCAGCTTTGCGGCGCTGCGCGCTCAGGTCGGCACTATGCCGGT
CGGCAGCAAAATCAGCCTCGGTCTGCTGCGTGAAGGTAAAGCGATTACGGTGAATCTGGAACTGCAGCAGAGCAGCCAGAGTC
AGGTTGATTCCAGCACCATCTTCAGCGGGATTGAAGGCGCTGAAATGAGCAATAAAGGCCAGGATAAAGGCGTTGTGGTGAGC
AGCGTGAAAGCGAACTCACCCGCCGCGCAAATTGGCCTCAAAAAAGGCGATGTGATTATCGGCGCTAACCAGCAGCCGGTGAA
AAATATCGCCGAGCTGCGTAAGATTCTCGACAGCAAGCCGTCGGTTCTGGCGCTGAATATTCAGCGTGGTGATAGTTCTATTT
ATTTGCTGATGCAGTAA
SEQ ID NO: 21: hlyA (encoding Listeria hemolysin, Gene ID:987033) 
ATGAAAAAAATAATGCTAGTTTTTATTACACTTATATTAGTTAGTCTACCAATTGCGCAACAAACTGAAGCAAAGGATGCATC
TGCATTCAATAAAGAAAATTCAATTTCATCCATGGCACCACCAGCATCTCCGCCTGCAAGTCCTAAGACGCCAATCGAAAAGA
AACACGCGGATGAAATCGATAAGTATATACAAGGATTGGACTACAATAAAAACAATGTATTAGTATACCACGGAGATGCAGTG
ACAAATGTGCCGCCAAGAAAAGGTTACAAAGATGGAAATGAATATATTGTTGTGGAGAAAAAGAAGAAATCCATCAATCAAAA
TAATGCAGACATTCAAGTTGTGAATGCAATTTCGAGCCTAACCTATCCAGGTGCTCTCGTAAAAGCGAATTCGGAATTAGTAG
AAAATCAACCAGATGTTCTCCCTGTAAAACGTGATTCATTAACACTCAGCATTGATTTGCCAGGTATGACTAATCAAGACAAT
AAAATCGTTGTAAAAAATGCCACTAAATCAAACGTTAACAACGCAGTAAATACATTAGTGGAAAGATGGAATGAAAAATATGC
TCAAGCTTATCCAAATGTAAGTGCAAAAATTGATTATGATGACGAAATGGCTTACAGTGAATCACAATTAATTGCGAAATTTG
GTACAGCATTTAAAGCTGTAAATAATAGCTTGAATGTAAACTTCGGCGCAATCAGTGAAGGGAAAATGCAAGAAGAAGTCATT
AGTTTTAAACAAATTTACTATAACGTGAATGTTAATGAACCTACAAGACCTTCCAGATTTTTCGGCAAAGCTGTTACTAAAGA
GCAGTTGCAAGCGCTTGGAGTGAATGCAGAAAATCCTCCTGCATATATCTCAAGTGTGGCGTATGGCCGTCAAGTTTATTTGA
AATTATCAACTAATTCCCATAGTACTAAAGTAAAAGCTGCTTTTGATGCTGCCGTAAGCGGAAAATCTGTCTCAGGTGATGTA
GAACTAACAAATATCATCAAAAATTCTTCCTTCAAAGCCGTAATTTACGGAGGTTCCGCAAAAGATGAAGTTCAAATCATCGA
CGGCAACCTCGGAGACTTACGAGATATTTTGAAAAAAGGCGCTACTTTTAATCGAGAAACACCAGGAGTTCCCATTGCTTATA
CAACAAACTTCCTAAAAGACAATGAATTAGCTGTTATTAAAAACAACTCAGAATATATTGAAACAACTTCAAAAGCTTATACA
GATGGAAAAATTAACATCGATCACTCTGGAGGATACGTTGCTCAATTCAACATTTCTTGGGATGAAGTAAATTATGATCCTGA
AGGTAACGAAATTGTTCAACATAAAAACTGGAGCGAAAACAATAAAAGTAAGCTAGCTCATTTCACATCGTCCATCTATTTGC
CAGGTAACGCGAGAAATATTAATGTTTACGCTAAAGAATGCACTGGTTTAGCTTGGGAATGGTGGAGAACGGTAATTGATGAC
CGGAACTTACCACTTGTGAAAAATAGAAATATCTCCATCTGGGGCACCACGCTTTATCCGAAATATAGTAATAAAGTAGATAA
TCCAATCGAATAA
SEQ ID NO: 22: Sequence of plasmid pTargetF 
tcgagttcatgtgcagctccatcagcaaaaggggatgataagtttatcaccaccgactatttgcaacagtgccgttgatcgtg
ctatgatcgactgatgtcatcagcggtggagtgcaatgtcatgagggaagcggtgatcgccgaagtatcgactcaactatcag
aggtagttggcgtcatcgagcgccatctcgaaccgacgttgctggccgtacatttgtacggctccgcagtggatggcggcctg
aagccacacagtgatattgatttgctggttacggtgaccgtaaggcttgatgaaacaacgcggcgagctttgatcaacgacct
tttggaaacttcggcttcccctggagagagcgagattctccgcgctgtagaagtcaccattgttgtgcacgacgacatcattc
cgtggcgttatccagctaagcgcgaactgcaatttggagaatggcagcgcaatgacattcttgcaggtatcttcgagccagcc
 
acgatcgacattgatctggctatcttgctgacaaaagcaagagaacatagcgttgccttggtaggtccagcggcggaggaact
ctttgatccggttcctgaacaggatctatttgaggcgctaaatgaaaccttaacgctatggaactcgccgcccgactgggctg
gcgatgagcgaaatgtagtgcttacgttgtcccgcatttggtacagcgcagtaaccggcaaaatcgcgccgaaggatgtcgct
gccgactgggcaatggagcgcctgccggcccagtatcagcccgtcatacttgaagctagacaggcttatcttggacaagaaga
agatcgcttggcctcgcgcgcagatcagttggaagaatttgtccactacgtgaaaggcgagatcaccaaggtagtcggcaaat
aagatgccgctcgccagtcgattggctgagctcatgaagttcctattccgaagttccgcgaacgcgtaaaggatctaggtgaa
gatcctttttgataatctcatgaccaaaatcccttaacgtgagttttcgttccactgagcgtcagaccccgtagaaaagatca
aaggatcttcttgagatcctttttttctgcgcgtaatctgctgcttgcaaacaaaaaaaccaccgctaccagcggtggtttgt
ttgccggatcaagagctaccaactctttttccgaaggtaactggcttcagcagagcgcagataccaaatactgtccttctagt
gtagccgtagttaggccaccacttcaagaactctgtagcaccgcctacatacctcgctctgctaatcctgttaccagtggctg
ctgccagtggcgataagtcgtgtcttaccgggttggactcaagacgatagttaccggataaggcgcagcggtcgggctgaacg
gggggttcgtgcacacagcccagcttggagcgaacgacctacaccgaactgagatacctacagcgtgagctatgagaaagcgc
cacgcttcccgaagggagaaaggcggacaggtatccggtaagcggcagggtcggaacaggagagcgcacgagggagcttccag
ggggaaacgcctggtatctttatagtcctgtcgggtttcgccacctctgacttgagcgtcgatttttgtgatgctcgtcaggg
gggcggagcctatggaaaaacgccagcaacgcggcctttttacggttcctggccttttgctggccttttgctcacatgttctt
tcctgcgttatcccctgattctgtggataaccgtattaccgcctttgagtgagctgataccgctcgccgcagccgaacgaccg
agcgcagcgagtcagtgagcgaggaagcggaagagcgcctgatgcggtattttctccttacgcatctgtgcggtatttcacac
cgcatatgctggatccttgacagctagctcagtcctaggtataatactagtcatcgccgcagcggtttcaggttttagagcta
gaaatagcaagttaaaataaggctagtccgttatcaacttgaaaaagtggcaccgagtcggtgctttttttgaattctctaga
gtcgacctgcagaagcttagatctattaccctgttatccctac
SEQ ID NO: 23: Sequence of plasmid ptet-tetR-aaad 
gacgtctgtgtggaattgtgagcgtcatgtccgagtctgcaatgcttcgatCAAAAAACCCCTCAAGACCCGTTTAGAGGCCC
CAAGGGGTTATGCTAGGCTAGCtagagcGGTACCccTCCCTATCAGTGATAGAGATTGACATCCCTATCAGTGATAGAGATAC
TGAGCACATATGCAGCAGGACGCACTGACCGAATTCATTAAAGAGGAGAAAGGTACCATGTCTAGATTAGATAAAAGTAAAGT
GATTAACAGCGCATTAGAGCTGCTTAATGAGGTCGGAATCGAAGGTTTAACAACCCGTAAACTCGCCCAGAAGCTAGGTGTAG
AGCAGCCTACATTGTATTGGCATGTAAAAAATAAGCGGGCTTTGCTCGACGCCTTAGCCATTGAGATGTTAGATAGGCACCAT
ACTCACTTTTGCCCTTTAGAAGGGGAAAGCTGGCAAGATTTTTTACGTAATAACGCTAAAAGTTTTAGATGTGCTTTACTAAG
TCATCGCGATGGAGCAAAAGTACATTTAGGTACACGGCCTACAGAAAAACAGTATGAAACTCTCGAAAATCAATTAGCCTTTT
TATGCCAACAAGGTTTTTCACTAGAGAATGCATTATATGCACTCAGCGCTGTGGGGCATTTTACTTTAGGTTGCGTATTGGAA
GATCAAGAGCATCAAGTCGCTAAAGAAGAAAGGGAAACACCTACTACTGATAGTATGCCGCCATTATTACGACAAGCTATCGA
ATTATTTGATCACCAAGGTGCAGAGCCAGCCTTCTTATTCGGCCTTGAATTGATCATATGCGGATTAGAAAAACAACTTAAAT
GTGAAAGTGGGTCTTAACTGCAGCCATGGGCTAGCGTCGACCTTAAGTCTAGAGAAAGAAGAGACTCGGCGCGCCatgagcga
agcacttaaaattctgaacaacatccgtactcttcgtgcgcaggcaagagaatgtactctggaaacgcttgaagaaatgctgg
aaaaattagaagttgtcgttaatgagcgtcgtgaagaagaaagcgctgctgctgctgaagtggaagaacgcactcgtaaactg
caacagtatcgtgaaatgttaattgccgacggcattgacccgaatgaactgctgaatagcatggctgccgctaaatccggtac
caaagctaaacgcgcagctcgtccggctaaatatagctatgttgacgaaaacggtgaaactaaaacctggactggccagggtc
gtacaccggctgtaatcaaaaaagcaatggaagaacaaggtaagcaactggaagatttcctgatcaaggaataaGGATCCGGC
TCACCTTCACGGGTGGGCCTTTCTTCGcggtatcagctcactcaaaggcggtaatacggttatccacagaatcaggggataac
gcaggaaagaacatgtgagcaaaaggccagcaaaaggccaggaaccgtaaaaaggccgcgttgctggcgtttttccataggct
ccgcccccctgacgagcatcacaaaaatcgacgctcaagtcagaggtggcgaaacccgacaggactataaagataccaggcgt
ttccccctggaagctccctcgtgcgctctcctgttccgaccctgccgcttaccggatacctgtccgcctttctcccttcggga
agcgtggcgctttctcaatgctcacgctgtaggtatctcagttcggtgtaggtcgttcgctccaagctgggctgtgtgcacga
accccccgttcagcccgaccgctgcgccttatccggtaactatcgtcttgagtccaacccggtaagacacgacttatcgccac
tggcagcagccactggtaacaggattagcagagcgaggtatgtaggcggtgctacagagttcttgaagtggtggcctaactac
ggctacactagaaggacagtatttggtatctgcgctctgctgaagccagttaccttcggaaaaagagttggtagctcttgatc
cggcaaacaaaccaccgctggtagcggtggtttttttgtttgcaagcagcagattacgcgcagaaaaaaaggatctcaagaag
atcctttgatcttttctacggggtctgacgctcagtggaacgaaaactcacgttaagggattttggtcatgactagtgcttgg
attctcaccaataaaaaacgcccggcggcaaccgagcgttctgaacaaatccagatggagttctgaggtcattactggatcta
tcaacaggagtccaagcgagctcgatatcaaacttatttgccgactaccttggtgatctcgcctttcacgtagtggacaaatt
cttccaactgatctgcgcgcgaggccaagcgatcttcttcttgtccaagataagcctgtctagcttcaagtatgacgggctga
tactgggccggcaggcgctccattgcccagtcggcagcgacatccttcggcgcgattttgccggttactgcgctgtaccaaat
gcgggacaacgtaagcactacatttcgctcatcgccagcccagtcgggcggcgagttccatagcgttaaggtttcatttagcg
cctcaaatagatcctgttcaggaaccggatcaaagagttcctccgccgctggacctaccaaggcaacgctatgttctcttgct
tttgtcagcaagatagccagatcaatgtcgatcgtggctggctcgaagatacctgcaagaatgtcattgcgctgccattctcc
aaattgcagttcgcgcttagctggataacgccacggaatgatgtcgtcgtgcacaacaatggtgacttctacagcgcggagaa
tctcgctctctccaggggaagccgaagtttccaaaaggtcgttgatcaaagctcgccgcgttgtttcatcaagccttacggtc
accgtaaccagcaaatcaatatcactgtgtggcttcaggccgccatccactgcggagccgtacaaatgtacggccagcaacgt
cggttcgagatggcgctcgatgacgccaactacctctgatagttgagtcgatacttcggcgatcaccgcttccctcatgacat
tgcactccaccgctgatgacatcagtcgatcatagcacgatcaacggcactgttgcaaatagtcggtggtgataaacttatca
tccccttttgctgatggagctgcacatgaactcgaacgtctcattttcgccagatatc
SEQ ID NO: 24: Sequence of plasmid Psc101-Fnr-SP-msbb-KnaR 
atggtcatagctgtttcctttaatgcccatggcgtttaccaatgcttaatcagtgaggcacctatctcagcgatctgtctatt
tcgttcatccatagttgcctgactccccgtcgtgtagataactacgatacgggagggcttaccatctggccccagtgctgcaa
tgataccgcgacttccacgctcaccggctccagatttatcagcaataaaccagccagccggaagggccgagcgcagaagtggt
cctgcaactttatccgcctccatccagtctattaattgttgccgggaagctagagtaagtagttcgccagttaatagtttgcg
caacgttgttgccattgctacaggcatcgtggtgtcacgctcgtcgtttggtatggcttcattcagctccggttcccaacgat
caaggcgagttacatgatcccccatgttgtgcaaaaaagcggttagctccttcggtcctccgatcgttgtcagaagtaagttg
gccgcagtgttatcactcatggttatggcagcactgcataattctcttactgtcatgccatccgtaagatgcttttctgtgac
tggtgagtactcaaccaagtcattctgagaatagtgtatgcggcgaccgagttgctcttgcccggcgtcaatacgggataata
 
ccgcgccacatagcagaactttaaaagtgctcatcattggaaaacgttcttcggggcgaaaactctcaaggatcttaccgctg
ttgagatccagttcgatgtaacccactcgtgcacccaactgatcttcagcatcttttactttcaccagcgtttctgggtgagc
aaaaacaggaaggcaaaatgccgcaaaaaagggaataagggcgacacggaaatgttgaatactcatactcttcctttttcaat
attattgaagcatttatcagggttattgtctcatgagcggatacatatttgaatgtatttagaaaaataaacaaataggggtt
ccgcgcacatttccccgaaaagtgccacctgacgtctaagaaaccattattatcatgacattaacctataaaaataggcgtat
cacgaggcccttaggccaaataggccgttcagatccttccgtatttagccagtatgttctctagtgtggttcgttgtttttgc
gtgagccatgagaacgaaccattgagatcatgcttactttgcatgtcactcaaaaattttgcctcaaaactggtgagctgaat
ttttgcagttaaagcatcgtgtagtgtttttcttagtccgttacgtaggtaggaatctgatgtaatggttgttggtattttgt
caccattcatttttatctggttgttctcaagttcggttacgagatccatttgtctatctagttcaacttggaaaatcaacgta
tcagtcgggcggcctcgcttatcaaccaccaatttcatattgctgtaagtgtttaaatctttacttattggtttcaaaaccca
ttggttaagccttttaaactcatggtagttattttcaagcattaacatgaacttaaattcatcaaggctaatctctatatttg
ccttgtgagttttcttttgtgttagttcttttaataaccactcataaatcctcatagagtatttgttttcaaaagacttaaca
tgttccagattatattttatgaatttttttaactggaaaagataaggcaatatctcttcactaaaaactaattctaatttttc
gcttgagaacttggcatagtttgtccactggaaaatctcaaagcctttaaccaaaggattcctgatttccacagttctcgtca
tcagctctctggttgctttagctaatacaccataagcattttccctactgatgttcatcatctgagcgtattggttataagtg
aacgataccgtccgttctttccttgtagggttttcaatcgtggggttgagtagtgccacacagcataaaattagcttggtttc
atgctccgttaagtcatagcgactaatcgctagttcatttgctttgaaaacaactaattcagacatacatctcaattggtcta
ggtgattttaatcactataccaattgagatgggctagtcaatgataattactagtccttttcctttgagttgtgggtatctgt
aaattctgctagacctttgctggaaaacttgtaaattctgctagaccctctgtaaattccgctagacctttgtgtgttttttt
tgtttatattcaagtggttataatttatagaataaagaaagaataaaaaaagataaaaagaatagatcccagccctgtgtata
actcactactttagtcagttccgcagtattacaaaaggatgtcgcaaacgctgtttgctcctctacaaaacagaccttaaaac
cctaaaggcttaagtagcaccctcgcaagctcgggcaaatcgctgaatattccttttgtctccgaccatcaggcacctgagtc
gctgtctttttcgtgacattcagttcgctgcgctcacggctctggcagtgaatgggggtaaatggcactacaggcgcctttta
tggattcatgcaaggaaactacccataatacaagaaaagcccgtcacgggcttctcagggcgttttatggcgggtctgctatg
tggtgctatctgactttttgctgttcagcagttcctgccctctgattttccagtctgaccacttcggattatcccgtgacagg
tcattcagactggctaatgcacccagtaaggcagcggtatcatcaacaggcttacccgtcttactgtcaagaggacatccggt
cagataaccgtcatcttcggctactgtctagggcggcggatttgtcctactcaggagagcgttcaccgacaaacaacagataa
aacgaaaggcccagtctttcgactgagcctttcgttttatttgatgcactagtgccgatcatattcaataacccttaatataa
cttcgtataatgtatgctatacgaagttattaggtctgaagaggagtttacgtcccatatggagtcccgctcagaagaactcg
tcaagaaggcgatagaaggcgatgcgctgcgaatcgggagcggcgataccgtaaagcacgaggaagcggtcagcccattcgcc
gccaagctcttcagcaatatcacgggtagccaacgctatgtcctgatagcggtccgccacacccagccggccacagtcgatga
atccagaaaagcggccattttccaccatgatattcggcaagcaggcatcgccatgggtcacgacgagatcctcgccgtcgggc
atgcgcgccttgagcctggcgaacagttcggccggcgcgagcccctgatgctcttcgtccagatcatcctgatcgacaagacc
ggcttccatccgagtacgtgctcgctcgatgcgatgtttcgcttggtggtcgaatgggcaggtagccggatcaagcgtatgca
gccgccgcattgcatcagccatgatggatactttctcggcaggagcaaggtgagatgacaggagatcctgccccggcacttcg
cccaatagcagccagtcccttcccgcttcagtgacaacgtcgagcacagctgcgcaaggaacgcccgtcgtggccagccacga
tagccgcgctgcctcgtcctgcagttcattcagggcaccggacaggtcggtcttgacaaaaagaaccgggcgcccctgcgctg
acagccggaacacggcggcatcagagcagccgattgtctgttgtgcccagtcatagccgaatagcctctccacccaagcggcc
ggagaacctgcgtgcaatccatcttgttcaatcatgcgaaacgatcctcatcctgtctcttgatcagatcctgatcccctgcg
ccatcagatccttggcggcaagaaagccatccagtttactttgcagggcttcccaaccttaccagagggcgccccagctggca
attccgacgtcattgcgttgctagccaagcttaggatccggaacccttaatataacttcgtataatgtatgctatacgaagtt
attaggtccctcgaagaggttcactagctatctagagcttggactcctgttgatagatccagtaatgacctcagaactccatc
tggatttgttcagaacgctcggttgccgccgggcgttttttattggtgagaatccaagcccgggaaacgggatcctgatcttg
atccgccgcaaagtttgagcgaagtcaataaactctctacccattcagggcaatatctctcttgcaggtgaatgcaacgtcaa
gcgatggcctacttgcggccgcaagaagctgtcaccggatgtgctttccggtctgatgagtccgtgaggacgaaacagcctct
acaaataattttgtttaacgcattgcatcacacaggaaagggtacatggaaaccaaaaaaaataatagtgagtatatccctga
attcgaaaaatcctttcgctatccacagtattggggcgcctggttgggcgcggcggcaatggcggggatcgcattaacaccgg
catcattccgcgaccctttgctggcgacgctggggcgttttgccggacggctggggaagagttctcgtcgccgggcgctaatt
aatctgtcgttgtgctttccgcagcgtagcgaagctgagcgcgaagcgattgtcgatgagatgttcgccaccgcgccacaggc
aatggcgatgatggctgagttggcgatgcgcggtccgaaaaaaattcaacagcgtgttgactgggaaggtctggagattatcg
aggagatgcgtcgtaacgacgaaaaagtcatttttctcgtaccgcatggctggggcgtcgacattccagccatgctgatggcc
tctcaggggcaaaaaatggcggcgatgtttcataatcagggtaatccggtttttgactatatctggaacacagtgcgtcggcg
tttcggcggacgtttgcatgcgcgtaatgacgggattaaaccctttattcagtctgttcgtcagggctactggggttactacc
tgccggaccaggatcacggcccggagcatagtgaattcgttgatttctttgcgacatacaaagcgacgctgcctgcaattggt
cggctgatgaaagtgtgccgcgcacgcgtgataccgcttttcccggtgtataatggtaaaacgcatcgcctgactatccagat
tcgcccgccaatggacgatctgctcacggctgacgaccacactatcgccagacggatgaacgaagaggtcgaaatttttgtcg
gcccgcatccggaacagtacacctggatcctgaagctgctcaaaacccgcaagccaggcgagattcagccgtataagcgtaaa
gatctttatcccatcaaagagcagaaactcatctctgaagaggatctgtaactcgagtaagattacttcgcattgcaatcagc
tacggactcaagcttacgtcaggtcatacgatgaaggcgataagtcgcgtaagttgctagcccacgcggctcaccttcacggg
tgggcctttcttcgcgatccacatgc
SEQ ID NO: 25: Sequence of plasmid Psc101-Fnr-SP-lppAB-KnaR 
catggtcatagctgtttcctttaatgcccatggcgtttaccaatgcttaatcagtgaggcacctatctcagcgatctgtctat
ttcgttcatccatagttgcctgactccccgtcgtgtagataactacgatacgggagggcttaccatctggccccagtgctgca
atgataccgcgacttccacgctcaccggctccagatttatcagcaataaaccagccagccggaagggccgagcgcagaagtgg
tcctgcaactttatccgcctccatccagtctattaattgttgccgggaagctagagtaagtagttcgccagttaatagtttgc
gcaacgttgttgccattgctacaggcatcgtggtgtcacgctcgtcgtttggtatggcttcattcagctccggttcccaacga
tcaaggcgagttacatgatcccccatgttgtgcaaaaaagcggttagctccttcggtcctccgatcgttgtcagaagtaagtt
ggccgcagtgttatcactcatggttatggcagcactgcataattctcttactgtcatgccatccgtaagatgcttttctgtga
ctggtgagtactcaaccaagtcattctgagaatagtgtatgcggcgaccgagttgctcttgcccggcgtcaatacgggataat
accgcgccacatagcagaactttaaaagtgctcatcattggaaaacgttcttcggggcgaaaactctcaaggatcttaccgct
 
gttgagatccagttcgatgtaacccactcgtgcacccaactgatcttcagcatcttttactttcaccagcgtttctgggtgag
caaaaacaggaaggcaaaatgccgcaaaaaagggaataagggcgacacggaaatgttgaatactcatactcttcctttttcaa
tattattgaagcatttatcagggttattgtctcatgagcggatacatatttgaatgtatttagaaaaataaacaaataggggt
tccgcgcacatttccccgaaaagtgccacctgacgtctaagaaaccattattatcatgacattaacctataaaaataggcgta
tcacgaggcccttaggccaaataggccgttcagatccttccgtatttagccagtatgttctctagtgtggttcgttgtttttg
cgtgagccatgagaacgaaccattgagatcatgcttactttgcatgtcactcaaaaattttgcctcaaaactggtgagctgaa
tttttgcagttaaagcatcgtgtagtgtttttcttagtccgttacgtaggtaggaatctgatgtaatggttgttggtattttg
tcaccattcatttttatctggttgttctcaagttcggttacgagatccatttgtctatctagttcaacttggaaaatcaacgt
atcagtcgggcggcctcgcttatcaaccaccaatttcatattgctgtaagtgtttaaatctttacttattggtttcaaaaccc
attggttaagccttttaaactcatggtagttattttcaagcattaacatgaacttaaattcatcaaggctaatctctatattt
gccttgtgagttttcttttgtgttagttcttttaataaccactcataaatcctcatagagtatttgttttcaaaagacttaac
atgttccagattatattttatgaatttttttaactggaaaagataaggcaatatctcttcactaaaaactaattctaattttt
cgcttgagaacttggcatagtttgtccactggaaaatctcaaagcctttaaccaaaggattcctgatttccacagttctcgtc
atcagctctctggttgctttagctaatacaccataagcattttccctactgatgttcatcatctgagcgtattggttataagt
gaacgataccgtccgttctttccttgtagggttttcaatcgtggggttgagtagtgccacacagcataaaattagcttggttt
catgctccgttaagtcatagcgactaatcgctagttcatttgctttgaaaacaactaattcagacatacatctcaattggtct
aggtgattttaatcactataccaattgagatgggctagtcaatgataattactagtccttttcctttgagttgtgggtatctg
taaattctgctagacctttgctggaaaacttgtaaattctgctagaccctctgtaaattccgctagacctttgtgtgtttttt
ttgtttatattcaagtggttataatttatagaataaagaaagaataaaaaaagataaaaagaatagatcccagccctgtgtat
aactcactactttagtcagttccgcagtattacaaaaggatgtcgcaaacgctgtttgctcctctacaaaacagaccttaaaa
ccctaaaggcttaagtagcaccctcgcaagctcgggcaaatcgctgaatattccttttgtctccgaccatcaggcacctgagt
cgctgtctttttcgtgacattcagttcgctgcgctcacggctctggcagtgaatgggggtaaatggcactacaggcgcctttt
atggattcatgcaaggaaactacccataatacaagaaaagcccgtcacgggcttctcagggcgttttatggcgggtctgctat
gtggtgctatctgactttttgctgttcagcagttcctgccctctgattttccagtctgaccacttcggattatcccgtgacag
gtcattcagactggctaatgcacccagtaaggcagcggtatcatcaacaggcttacccgtcttactgtcaagaggacatccgg
tcagataaccgtcatcttcggctactgtctagggcggcggatttgtcctactcaggagagcgttcaccgacaaacaacagata
aaacgaaaggcccagtctttcgactgagcctttcgttttatttgatgcactagtgccgatcatattcaataacccttaatata
acttcgtataatgtatgctatacgaagttattaggtctgaagaggagtttacgtcccatatggagtcccgctcagaagaactc
gtcaagaaggcgatagaaggcgatgcgctgcgaatcgggagcggcgataccgtaaagcacgaggaagcggtcagcccattcgc
cgccaagctcttcagcaatatcacgggtagccaacgctatgtcctgatagcggtccgccacacccagccggccacagtcgatg
aatccagaaaagcggccattttccaccatgatattcggcaagcaggcatcgccatgggtcacgacgagatcctcgccgtcggg
catgcgcgccttgagcctggcgaacagttcggccggcgcgagcccctgatgctcttcgtccagatcatcctgatcgacaagac
cggcttccatccgagtacgtgctcgctcgatgcgatgtttcgcttggtggtcgaatgggcaggtagccggatcaagcgtatgc
agccgccgcattgcatcagccatgatggatactttctcggcaggagcaaggtgagatgacaggagatcctgccccggcacttc
gcccaatagcagccagtcccttcccgcttcagtgacaacgtcgagcacagctgcgcaaggaacgcccgtcgtggccagccacg
atagccgcgctgcctcgtcctgcagttcattcagggcaccggacaggtcggtcttgacaaaaagaaccgggcgcccctgcgct
gacagccggaacacggcggcatcagagcagccgattgtctgttgtgcccagtcatagccgaatagcctctccacccaagcggc
cggagaacctgcgtgcaatccatcttgttcaatcatgcgaaacgatcctcatcctgtctcttgatcagatcctgatcccctgc
gccatcagatccttggcggcaagaaagccatccagtttactttgcagggcttcccaaccttaccagagggcgccccagctggc
aattccgacgtcattgcgttgctagccaagcttaggatccggaacccttaatataacttcgtataatgtatgctatacgaagt
tattaggtccctcgaagaggttcactagctatctagagcttggactcctgttgatagatccagtaatgacctcagaactccat
ctggatttgttcagaacgctcggttgccgccgggcgttttttattggtgagaatccaagcccgggaaacgggatcctgatctt
gatccgccgcaaagtttgagcgaagtcaataaactctctacccattcagggcaatatctctcttgcaggtgaatgcaacgtca
agcgatggcctacttgcggccgcaagaagctgtcaccggatgtgctttccggtctgatgagtccgtgaggacgaaacagcctc
tacaaataattttgtttaacgcattgcatcacacaggaaagggtaccatgaatcgtactaaactggtactgggcgcggtaatc
ctgggttctactctgctggctggttgctccagcaacgctaaaatcgatcagctgtcttctgacgttcagactctgaacgctaa
agttgaccagctgagcaacgacgtgaacgcaatgcgttccgacgttcaggctgctaaagacgacgcagctcgcgctaaccagc
gtctggacaaccaggctactaaataccgtaagcatcaccatcaccatcactaaatcagctacggactcaagcttacgtcagga
ttaaagaggagaaactcgagatgaaccgtactaaccagctgatcttaggtgcagtagttctgggttccacgttactggcaggt
tgttcaagcaacgctaaaatcgatcagttgtcttccgatgtacagacgctgagcgctaaagttgagcaactgagtaatgacgt
aaatgcaatgcgttccgacgttcaggctgctaaagacgatgctgctcgcgcgaaccaacgtctggacaacaaagtattccgca
tctgtaaagagcagaaactcatctctgaagaggatctgtaactcgagtaagattacttcgcattgcaatcagctacggactca
agcttacgtcaggtcatacgatgaaggcgataagtcgcgtaagttgctagcccacgcggctcaccttcacgggtgggcctttc
ttcgcgatccacatg
SEQ ID NO: 26 rfbN 
atgaaaataacattaattattcccacatataatgcagggtcgctttggcctaatgttctggatgcgattaagcagcaaactat
atatccggataaattgattgttatagactcaggttctaaagatgaaacggttccgttagcctcagacctgaaaaatatatcaa
tatttaatattgactctaaagattttaatcatggaggaaccagaaatttagcagttgcaaaaactctggacgctgatgttata
atttttctaacgcaagatgcaattctcgcggattcggatgcaattaaaaatttggtttattatttttcagatccattgatagc
agcggtttgtggtagacaacttcctcataaagatgctaatccccttgcagtgcatgccagaaattttaattatagttcaaaat
ctattgttaaaagtaaggcagatatagaaaaattgggtattaaaactgtatttatgtccaattcttttgctgcctatcgccgt
tccgtttttgaagagttaagtgggtttcctgaacatacaattcttgccgaggatatgtttatggcggctaagatgattcaggc
gggttataaggtcgcctactgcgctgaagcggtggtaagacactcccataattataccccgcgagaagagtttcaacgatatt
ttgatactggtgtatttcatgcttgttctccgtggattcagcgtgactttggcggagccggtggtgagggtttccgcttcgta
aaatcagagattcaattcctgcttaaaaatgcaccgttctggattccaagagctttattaacaacctttgctaaattcttggg
ttacaaattaggcaagcattggcaatctttaccgttgtctacatgtcgctattttagcatgtacaagagttattggaataata
tccaatattcttcgtcaaaagagataaaataa
SEQ ID NO: 27 rfbU atgatcgtaaacctatcacgtttaggtaaaagtggtacgggaatgtggcaatactcgattaaatttttaacggcactgcgaga 
aatagctgatgttgacgcaataatctgtagcaaggtacacgctgattattttgaaaagctcggttatgcagtagttactgttc
cgaatattgttagcaacacatcaaaaacatcgcgacttagaccattagtatggtatgtatatagttactggcttgcgctgagg
gttttaattaagtttggtaataaaaaattggtgtgtactacacatcacactatccccttactgagaaaccaaacgataaccgt
acatgatataagacctttttattatccagatagttttattcagaaagtgtattttcgctttttattaaaaatgtccgttaagc
gatgtaagcatgttttaacggtatcttataccgttaaagatagcattgctaaaacttataatgtagatagtgagaaaatatca
gtaatttataatagtgttaataaatctgattttatacaaaaaaaagaaaaagagaattactttttagctgttggtgcaagttg
gccacataaaaatattcattcattcataaaaaataaaaaagtttggtctgactcttataatttaattattgtatgtggtcgta
ctgactatgcaatgtctctccaacaaatggtcgttgatctggaactaaaagataaagtgacttttttacatgaagtctcattt
aatgaattaaagattttatattctaaagcctacgcgcttgtttatccatctattgatgagggttttggtatacctcctattga
agcgatggcatcaaatactccagttatagtgtccgatataccagtatttcatgaagtgttaaccaatggtgcattatatgtga
atccggatgatgaaaaaagctggcagagtgcaattaaaaatatagagcagttgcctgatgcaatttcccgatttaacaactat
gtcgcacggtatgactttgataatatgaagcagatggttggcaattggttggcggaatcaaaataa
SEQ ID NO: 28 rfbV 
atgcttatatcattttgtattccaacttataatagaaaagaatatcttgaagagttgttgaatagtataaataatcaggaaaa
atttaatttagatattgagatatgtatatcagataatgcctctactgatggtacagaggaaatgattgatgtttggaggaaca
attataatttcccaataatatatcggcgtaatagcgttaaccttgggccagataggaattttcttgcttcagtatcccttgcg
aatggggattattgttggatatttggcagtgatgatgctcttgcgaaagactcgttagcgatattacaaacttatctcgattc
tcaagcagatatatatttatgtgacagaaaagagaccgggtgtgatttagttgagattagaaaccctcatcgttcttggctca
gaacagatgatgaactttatgtgtttaataataatttagatagggaaatctatctcagtagatgcttatctattggtggtgta
tttagctatctaagttctttaatagtaaaaaaagaacgatgggatgccattgattttgatgcgtcctatattggcacttccta
tcctcatgtatttatcatgatgagcgtatttaatacgccagggtgccttttgcattatatatcaaaaccactcgtaatatgcc
gaggagataatgatagtttcgagaagaaaggaaaggccagacgaattttaattgattttattgcatatttaaaattagctaat
gatttttacagtaaaaatatatctttaaaacgagcatttgaaaatgttttgctaaaagagagaccatggttatatacaacttt
ggctatggcatgttatggcaatagtgatgaaaaaagagatttatctgaattttatgcaaagctaggttgtaataaaaatatga
tcaacactgtacttcgatttgggaaactagcatatgcagtgaaaaatattaccgtgcttaagaattttactaaacggataatt
aagtag
SEQ ID NO: 29 wzzB 
atgacagtggatagtaatacgtcttccgggcgtgggaacgatccggaacagattgatttgattgagttattgctacagttatg
gcgtgggaagatgaccattattgtagccgttattatcgccattttgctggctgtaggctacctgatgatagccaaagaaaaat
ggacatccacggcgattattacccaacctgatgccgcgcaggttgccacctataccaacgcgctcaacgtcttgtatggtggg
aatgcccccaaaatctctgaagtgcaggcgaattttataagccgctttagctctgcgttttccgcattatcggaagtgctgga
taatcagaaagagcgggaaaagcttaccattgaacagtcggtaaaagggcaggcgctgccactctcggtttcttatgtgagta
ctaccgctgaaggggcgcagcgtcggctggcggaatatatccaacaggtggatgaagaggtcgctaaggaactggaagttgac
ctgaaagataacatcacgctgcaaaccaaaacgttgcaggagtcccttgagacgcaggaagttgtggcgcaggagcaaaaaga
tctgcgtattaagcaaatcgaagaagcgttgcgctatgcggatgaggccaaaatcacgcagccgcagattcagcaaacccagg
atgttacccaggacacgatgttcctgttggggagcgatgcgctaaaatcgatgatacagaacgaagcgacgcgtccactggtc
ttttctccggcctattaccagacgaagcagacactgctggacattaaaaatctgaaagtgactgccgatacggtgcacgtcta
tcgttatgtgatgaagccgacgctgccggtccgtcgcgatagcccgaaaacagccattacccttgtgctggctgtattgctgg
gtgggatgatcggtgccgggattgtgctgggacgcaatgcgctacgtagctataagccaaaagccttgtaa
SEQ ID NO: 30 gtrBb 
atgaaaatttcattagtggttcccgtctttaatgaagaggacgcgatccctattttctataaaacggtcagagaatacagttc
acttaaaccttataacgttgagattatcttcgttaatgatgggagtcacgatgcgactgaatcaatcatcagcgcattagctg
ttgccgatcctcttgttgttccgatctcatttacccgcaattttggtaaagaacctgcactttttgccggattagatcacgcg
accggagatgtggtgatccctatcgatgttgatttacaagatcccatcgaagtaatcccacatttgatcaataaatggcaggc
tggtgcagaaatggtgctggctaagcgtatcgatcgttcaacggatggccacctgaagcgtaaaagcgctgagtggttctaca
ggctgcataacaaaatcagtacgccaaagattgaagagaatgtcggtgattttcgattgatgtcgcgcgagattgtagaaaat
atcaagctattaccagaacgtaaccttttcatgaaaggtatactttcatgggttggaggtcaaacagatgtggtcgaatatgc
ccgtgctgaacgtgtcgcaggtaactcaaaatttaatggctggaaactctggaacctggcgctggaggggattacaagttttt
ctactttccctttgcgtatctggatgtatataggagtgagcgtttctgccctctccctgatatatgccatgtggatgatcatt
gataaattgatgtggggaaaccctgttcctggttatccttcgcttatgaccgcgattctcttcttaggcggcatccagcttat
cggcataggcatcatgggtgaatatatcggacgcgtttacacggaggtgaagcaaagaccccgctatatcgtgaaaaacaaaa
aaacaatgatggaataa
SEQ ID NO: 31 gtrBa 
atgaaaatctcgttagtcgttccggtcttcaatgaagaagccacgatacctattttctataaaacggttcgcgagtttgaaga
gctaaaaccgtatgaagttgagattgttttcatcaacgatggaagcaaagatgcgactgaatcaataattaacaaaatagctg
catctgatccgctcgttattccgctttcgtttacgcgaaacttcggtaaagaacctgctcttttcgcgggtctcgaccatgca
accggggatgcggtcattcctattgatgtcgatttacaggatccgatagaagtaatcccccatctcattgagaagtggcaggc
tggcgcggatatggtgctggctaagcgctcagaccgctcaactgacgggcgcatgaagcgtaagacagctgaatggttttata
agctgcataataaaatcagcaatccgaaaattgaagagaacgttggcgactttcggttaatgagtcgtgaggttgtcgaaaat
attaaacagatgccagaacgtaatctgtttatgaaaggcgttctgtcatgggtgggcggcaagactgatgtagttgaatacgc
ccgcgctgagcgtgttgccggcgattcaaaattcaatggctggaaattatggaatctggcgctggaaggaataacttctttct
caacatttccgctccgcatatggacttacattggattgtttattgcaggtatgtcattcctttacggtgcatggatgattatt
gataaattaatatttggaaataatgttcctggctacccgtctcttcttgtttctgtactttttctgggtggcgttcaattgat
aggaataggtattcttggggaatatattggcaggatttacatagaaacaaaaaaaaggcctaagtacataattaagaatgaga
agaaaaatggttaa
SEQ ID NO: 32 rfbP/wbaP 
atggataatattgataataagtataatccacagctatgtaaaatttttttggctatatcggatttgattttttttaatttagc
cttatggttttcattaggatgtgtctattttatttttgatcaagtacagcgatttattcctcaagaccaattagatacaagag
ttattacgcattttattttgtcagtagtatgtgtcggttggttttggattcgtttgcgacattatacataccgcaagccattt
tggtatgagttaaaagaaatttttcgtacgatcgttatttttgctatatttgatttggctctgatagcgtttacaaaatggca
gttttcacgctatgtctgggtgttttgttggacttttgccctaatcctggtgcctttttttcgcgcacttacaaagcatttat
tgaacaagctaggtatctggaagaaaaaaactatcatcctggggagcggacagaatgctcgtggtgcatattctgcgctgcaa
agtgaggagatgatggggtttgatgttatcgctttttttgatacggatgcgtcagatgctgaaataaatatgttgccggtgat
aaaggatactgagattatttgggatttaaatcgtacaggtgatgtccattatatccttgcttatgaatacaccgagttggaga
aaacacatttttggctacgtgaactttcaaaacatcattgtcgttctgttactgtagtcccctcgtttagaggattgccatta
tataatactgatatgtcttttatctttagccatgaagttatgttattaaggatacaaaataacttggctaaaaggtcgtcccg
ttttctcaaacggacatttgatattgtttgttcaataatgattcttataattgcatcaccacttatgatttatctgtggtata
aagttactcgagatggtggtccggctatttatggtcaccagcgagtaggtcggcatggaaaactttttccatgctacaaattt
cgttctatggttatgaattctcaagaggtactaaaagaacttttggctaacgatcctattgccagggctgaatgggagaaaga
ttttaaactgaaaaatgatcctcgaatcacagctgtaggtcgatttatacgtaaaactagccttgatgagttgccacaacttt
ttaatgtactaaaaggtgatatgagcctggttggaccacgacctatcgtttcggatgaactggagcgttattgtgatgatgtt
gattattatttgatggcaaagccgggcatgacaggtctatggcaagtgagtgggcgtaatgatgttgattatgacactcgtgt
ttattttgattcctggtatgttaaaaactggacgctttggaatgatattgccattctgtttaaaacagcgaaagttgttttgc
ggcgagatggtgcgtattaa
SEQ ID NO: 33 rfe 
ttacttggttaagtttggccggttctcacgttgcctgcgcaggcgacgcttcacacgcttaataaatctcgccaccttccagg
cccgcttaatgcagtagccatagaggaagaatgctagcaaaaagagcaccaacataacccattcaggaacaaaatgagagtat
tctgccgttacgcctactcccgccagaatcgccgctgcgagcgtaataagaacaaatgcctggcgggaagtaaaccctgctcg
cataaccaaatgatgaatatgttggcgatcaggggaaaaggggctcatccctttacgcagacggcgatacataatcgccacca
tatccatcaatggaatagcgataatccatagcgcggtaaccgggctgatagagtgagttttcccctgcgtcgtttccagtaag
agccagatgacggtaaagccaatcagtgtactaccggcatcgcccataaagactttgtaacgccgccccaaaataccgagatt
aagcataatgtaaggcagaatcgcggcgatcatggcaaagcaccacattgccagactagtctgaccatcaaaccataaaatca
gccccatcgccgcaaatgagacgctggataacccacccagcaggccgtcaataccgtccaccatattgaaagcgttgatcgcc
gcccataccgcaaacagagtaaggaaatagccaaaggggccaagcactaactcccaaggcccaaagatgtaaccaaggctacc
taagtgaagttttgcgataaccatcattacaaccgcaataacagcctgaaccacagcacgaatttttacactgatatcaaaac
ggtcatccatcgcaccaacaaaaaccaatacaccagcgcaaataagatatagcgataagtgcggtatgtagtaatccgagagg
ccaaacataaagcagattcctgcaaacacagaaatcccgccgactaatgggataacgccctgatgtcgtttacgaaagtttgg
tttatccactaaaccaatttttatggctacttttcgcgcaagaaaaataaaaatggtcgtgaataaaaaaatactgattagct
cagataaagcggtgagtaacttcac
SEQ ID NO: 34 rfaB 
Atgaaaatagcttttattggcgaagcggtatccggttttggagggatggaaaccgttattagcaacgttatccatacgtttga
aaatagctctccgaagatcaactgtgaaatgtttttcttttgtcgtaatgacaaaatggataaagcatggctaaaagagatta
aatatgcgcaatcattctcaaatataaaattaagttttcttcgtcgagctaagcatgtttataattttagtcagtggctgaaa
gaaacgtcaccggatattgtgatatgtattgatgtcatctcatgtctttatgccaataaagcacgaaagaaaagtggtaaaca
ttttactattttctcgtggccgcatttttcgctcgaccataaaaaacatgcagaatgtataacgtacgcggattatcatctgg
ccatcagtagtggtattaaagaacagatcatggcacggggtatctcagcgcaagatattagcgtcgtttataatcctgtctct
attaaaacagtcattgtgccgccgcctgagcgcgataaaccagctgtatttctctatgtagggcgtcttaaatttgaagggca
gaaaagagttaaagatttatttgatggcttagctcgtacgacaggggaatggcagctacatattattggtgatggctcagatt
ttgaaaagtgccaggcctatagtcgggagcttggtattgagcagcgtgtgatctggtatggttggcaaagcgcgccgtggcaa
gtcgtacagcaaaaaataaaaaatgtcaccgcgttactacttacctctgcatttgagggatttcctatgaccctgctggaagc
aatgtcatatggaattccgtgtattagttctgattgtatgtctggtccgcgcgatatgattaaacccggtttgaatggtgagc
tttacactccgggtgctatagatgattttgtaggacaccttaatcgcgttatttctggtgaagtaaaatatcagcatgatatt
attcccggcacgattgagagattttacgatgtgctatattttaaaaattttaataatgcaatattctcgaaattacaaaagtg
a
SEQ ID NO: 35rfaC/waaC 
ttaatgaatctttccaaatacgcttgtggcatctaaattcgccaggttctgttctggtgagcagcatgccatttggttcttcc
cataacctccaattaacccagggtccgttgggccatatagcgtaatattcggtctgtcgagcgcggcggtgagatggctcagg
ccggtatcaaccgatacgacaaattttgcgccagccagcactctggcgacctcctccaggctcatgcgcggtaacacatccac
atagtcaaagccttcggccagtcgtttagcccgggcctcctcatgaggcgcgccccagggaagctttatccgtaatccggtgt
tgcccaccaggccgataagctcgcgccagtttgcttccggccagtgtttatcatcgcgggtcgtggcatgtaaaaacaccgca
tacggatcgctaaccgcctgctggcaatgcagaaaatgttttgcgatggcataatcgccctgcgactgcggtttatcgtatcc
caggcttttggcgaacagctcgcgcgtccgttcaaccgcatgttgttgctttgcgatatggtgtttacggttatagaacaggc
tggccagcggttcgcgggcggtactccagtccataccgtgctttatcccatgcgccagacgcgtcaccagcgccgcgcttttt
accagcccctgcgcatcaatcacagcgtcgtattggtttgcgcataccgcccgacgaaaggctgtgcgttccgctttgatggg
cgcggaaaaccaggccttgcgccaacggcgaatagcgacgggaatcacgcgatcgacagcactgtgccaggacggaatttgtg
caaacccttcttcgacagcccaatcaaattgaatccccggaatcgcctgttgcgcgtcggtaagcgcaggcagggtatgtaat
acgtcgcccatcgatgatgttttaacgatcaaaacccgcat
SEQ ID NO: 36 rfaF 
ttaaacgccctcttccgacaacagcgaatgaagctcttccagaacccgctgcggcgtgatatcgatcaggctctggtgatagc
cttgcgccgtatcacctttacgcactttgtgataacccgtaatgagacgaatcacccgggccttatgagacagcggcggcgtg
aaatccgggctacttggaccatacaaggcgaccagcgggcggtcgagcgccgccgcgacgtgcatcagcccggaatcgttagt
gacgatggctttacaggcggctatcagaatgacggcctgttccagctgggtttcccccgccaggttgcgacaccatgcctgct
 
gctcgctattcagcgccgccaggatctcatttccggcttcatggtcttttgccgagccaaacagcacgacctgatacccttcg
ttaatgagctgctttgccagttcggcgtagtgatagtgcggccaacgttttgccgggccaaattctgcgccggggcaaaagcc
gatcagaggacgttcagaagatagtgaaaagtcgctgcacatcagcgacttttcaccctcgctaacctggagctgtggccaga
gtagcggctggggcagatctttcgccgtgcgcatcacgcccttgtcataagccagcgccacgtagcgctccaccatcagtggc
caggcgtctttatcaaggacgcgcgcatcgttcagcaggccatagcgcatttcgccgcgccagccggtacggtgcgggatatt
ggcaaagaaaggaatcagcgccgatttaaacgaatttggcaacacccaggcgcgatcgtagcgcttctctcgcaggctatggc
ccaatctgcggcgctcgccgatttccagcgcgccgtggcccaacggcatgggtatcgcctcgttaacttccggcatacgcgat
aataacggacgacaccaggctggcgccatcacgtcgattatcgcctggggatagcgtgctttaagcgtgcgatagagactttg
cgacatcatcatgtcgcccacccaggacgggccaatgaccaaaattttcat
SEQ ID NO: 37 rfaG 
atgagagttgccttttgcttatataaatattttccttttggcggtctgcagcgtgattttatgcgcattgcccaaaccgtggc
ggcgcgagggcatcaggttcgtgtttatactcagtcatgggaaggggaatgcccggataactttgaattaatccgcgtgccgg
ttaaatcccggacgaaccacggtcgtaacgcagaatattatgcctgggtgcaacaccatttgcgcgaccaccctgtcgatcgg
gtggttggattcaataagatgccgggccttgatgtgtattacgccgcagacgtgtgttacgccgaaaaagtcgcacaggaaaa
aggatttttctatcgcctgacatcacgctatcggcattatgctgcttttgaacgcgccacgtttgaacacggcaagccgacgc
aactattaatgctgacgaataagcagattgctgacttccaaaaacattatcagactgaagcggagcgtttccatattcttcct
ccggggatttacccggacagaaaatatagccaacagatcccaaacagtcgtcaaatttatcgtcagaaaaatggtatctcaga
acagcaaaaattactgttgcaagtagggtctgactttacccgtaaaggtgtggatcgctctattgaagcgctggcatcgctac
ccgaatctttacggcaaaatacggtgctctatgttgtcgggcaggataagccgaagaagtttgcagcactggctgaaagaagc
ggcgtcggcacgaatgtgcattttttctccggacgtaatgatatcgcggagttaatggcggcagccgaccttttactgcatcc
agcctatcaggaagctgctggtattgttttgctggaagccattactgctggtttgccggtgctgacaaccgcggtgtgcggtt
atgcacattatattgtggatgcaaactgtggcgaagcgatgactgaaccattccgccaggatgcgctaaatgaggttttactc
aaagcgctgacacagccttccttacgcaacgcctgggctgaaaatgcgcggtattatgctgatacccaggatttatacagctt
accggagaaggccgcggatattattacaggtgatttagatggttga
SEQ ID NO: 38 yibD 
atgaaaaatagtaaaaccaaagtgagtatcattgtcccgttatataatgcgggagcggattttaatgcttgcatggcgtcgtt
aatcgcgcaaacgtggtcggcgctggaaattattattgtgaatgatggatcgacggatcattccgttgagatagcaaaacatt
acgcggaacattacccacatgttcgactgcttcatcaggccaatgctggcgcatctgtcgcccgtaatcttggcctgcaagcg
gcgaccggcgattatgtcgcctttgtcgatgcggatgaccaggtctacccgaagatgtatgaaacgctgatgactatggcgct
taacgatgatctggacgttgcgcagtgtaatgcggactggtgcgtccgaaaaaccgggcacgcctggcaatctattccgaccg
atcgtctgcgttccaccggggtattaagcggaccggattggttgcgtatggcgttggcctcgcggcgctggacgcatgttgtc
tggatgggcgtttatcgacgtgcgttaattaccgataacaatattactttcgttcccggactacatcatcaggacatattatg
gtcgacggaagttatgtttaatgccacgcgcgtacgttataccgaacaatcattatataaatatttcctgcatgataattcgg
taagccgtttgcaaagacaaggcaataaaaatcttaattatcagcggcattatattaaaattacgcgattattagaaaagctc
aatcgtgattatgcccgtcgtattccgatttacccggaatttcgccagcaaattacctgggaagcgttacgcgtttgtcatgc
ggtacgtaaagagcctgatattttgacccgccagcgtatgattgccgaaatttttacttctggcatgtatagacggatgatgg
ctaacgtccgcagcgcgaaagcagcttatcagacgctgctctggtccttccggctgtggcaatggcgcgacaaaaccttgtcg
caccgtcgtatggcccgtaaggcgctcaatctgtcttag
SEQ ID NO: 39 rfaI 
atgagcagaaaatattttgaagaagaagtcattcaacagactttagattataactatgcacaacatagtgatgctgataaatt
taatatagcttatgggattgataaaaactttctttttggctgtggtgtctctattgcatcggttctcctcgctaatccagaga
aggcgttagctttccatgtttttaccgatttctttgactctgaagaccagcagcgatttgaggcattagcaaaacagtacgct
acgcagattgttgtttacctaatcgactgtgagcgcttaaaatcgctacccagtaccaaaaactggacctatgcaacatactt
tagattcattatcgccgattatttttcagataaaacagatagagtactttatctggatgcagatattgcatgtaaggggagta
ttcaggaacttattgatcttaattttgctgaaaatgagattgcggctgtcgttgctgaaggcgagttggaatggtggactaag
cgctcggttagcctggcaacgcctgggctggtttctggctattttaatgccggttttattttaattaacatacctctttggac
tgcagaaaatatctctaagaaagcgattgaaatgctaaaagatccagaggtagtacagcgcataacgcaccttgatcaggatg
tattaaatatatttttagtgaataaagcgcgttttgttgataaaaaatttaatacacaatttagtcttaactatgaattaaaa
gattcagttattaatccagttgatgctgagactgtatttgttcattatatcggaccaacgaagccctggcatagttggggggc
ttaccccgtgtcacaatattttttacaggctaagagcaactcaccatggtctcattgtgcacttttaaatccagtgactagcc
atcagttacgttatgcggcaaagcatatgtttaatcagaagcattatacttcgggtataaattattatatagcctactttaaa
cgtaaacttcttgaataa
SEQ ID NO: 40 rfaJ 
atggattcatttcctgagatagaaatagctgaatataaagtttttgatgaaagtaataatgatgataacgtattaaacat
ttcttatggcgttgatgaaaactatcttgatggtgtgggggtatcaatcgcttcagttgtattaaacaataatatcccgctcg
cttttcacattatttgtgactcatactccccgtgttttgtaaaatatatagagcgtttagccgtacagcatcacataaaaatt
tctctttatcttattaaagtagaaagccttgaggtattgcctcaaactaaagtatggtcgagagcaatgtattttcgtttatt
tgctttcgattatctcagcaagaaggtaaataccttactttatttggatgccgatgttgtatgcaaaggatctttgcaagatc
ttttacagcttgatctgacagagaagatcgctgcggtcgtaaaagatgttgattccatccagaataaggtaaatgagagatta
agcgcttttaatttacaaggtggttattttaactccggcgtggtttttgttaacctgaaattatggaaagagaatgccttaac
caaaaaggcatttttacttttggcaggtaaagaggctgactcttttaaatatcccgatcaggatgttttgaatattctcctac
aggataaagtcatttttctaccgcgaccgtataatactatttatactattaaaagtgagttgaaagataagtcacataaaaaa
tatagcaatataattaatgataatactattttaattcattatacgggcgctacaaaaccatggcatgcctgggcaaattatcc
ttcagttatctattataaaaatgcacgactgaactcgccctggaaagattttcccgcaaaagatgcgcgtaccatagtcgaat
ttaagaagcgatataaacatcttctcgtgcaaggtcattattttaaaggccttctggctggaagcgcatatctttatcgtaaa
cttttccacaaataa
SEQ ID NO: 41 rfaK 
atgattaaaaaaatcatatttactgttactcctatattttcaattcctcctcgtggtgcggctgcggtagaaacctggattta
ccaggttgcaaaacgactatcaataccgaatgctattgcttgtataaagaatgctggctatcctgaatataataaaataaacg
ataactgtgatattcattacatcgggtttagtaaagtttataagcgtctttttcagaaatggactcgtctcgacccactaccc
tattcccagcgcatccttaatattagagataaagtgactacccaggaagatagcgtcattgttattcataatagtatgaaact
gtatcggcagatcagagagcgcaatccgaatgcaaaactggttatgcacatgcataatgcatttgaaccagaacttcctgata
atgatgcaaaaattatcgtgcccagtcagtttcttaaagcgttttatgaagaaagattgcccgccgctgctgttagtattgtg
cctaatggtttttgtgctgagacttataaaagaaacccacaagataatcttcgtcagcaattaaatattgcggaagatgccac
cgttctcttgtatgccgggagaatttcgcctgataaaggcatcctgttgcttttgcaggcgttcaaacaattacgtaccttaa
gaagtaatattaaacttgtcgttgttggcgacccttatgcaagccgcaagggtgaaaaagcagagtatcaaaagaaagtactg
gacgccgcaaaagagattggaacggattgtattatggctggggggcaatctcctgaccagatgcataacttctatcatatagc
cgatctggttattgtgccatctcaggttgaagaagcattttgcatggtggctgtagaagcgatggcagcaggaaaagcggttc
ttgccagcaaaaaaggggggattagcgaatttgtgttagatggcataacgggctatcacctcgcagaacctatgtcgagcgac
agtataattaatgatattaaccgtgcgcttgctgataaggaacgccaccagattgccgaaaaagcaaaatccctggtgttttc
aaaatacagttgggaaaatgtagcgcagcgtttcgaggaacaaatgaaaaactggtttgataagtga
SEQ ID NO: 42 rfaL 
ttatctatttcttagcgccagcagaaaaccggtaatgataccaatttgagcaatatcgacctgttcaaaattgccacgaacga
tataaaaaccgacgaaagataaaaatagcaagagatgagcattgtaggggcttatctctactttcctgagggtagagctggct
gtttccctgatgatagcgccatataaatagaccaggctcgccagacccaatatgcctgcactaaaccagatgtacagaatggt
attatgcggaccgatagattctttaaaggtccacgttggataatcgacaacgcgtttattataaacaccatcatacacatcat
taccatagccgtagcccttgatcgggttttcctgaatcagtatccacgcggtgccctgggttccgttagtataacgatatgag
ctatctgtctgctgtaatttatacagtaaatgttctgggtctggtttgttattatgttgagtgataaccaaagcgccgataat
ggctaataaaatggcaccaactcctattaacttccattggcggttcagtattgcccacagaacacctactataagcaccgcca
accatgcccctcgcgatagggttcccaggataaagaaaaggtagatggcgctaagcaccaaaaaaaccaacttaattgcattt
tttctaaacagccaaatattcaataatgctggaaataagaaaaccatggaatcggacatatgtcgatgcgcatagcttatgaa
tggcataatccctttattatagtcctcgatatacagaatactctctgcaaggcagcgaagtcctaaacttgttaaaaaggaga
aaagtactattttcgcaaccgtttcttttgtttcatcttttaatagtacgggaattaataaagtatataataagaagccctcc
agtaccgtattttcaaattccttgaaactttctttcatatctggcgatatgagtatggaataaacaaggattaatgataatac
tgctacgctataaaatacgctatttttgaaaagaggggggaaacttttcggtgagcgtgagacctgataaatcgcggtgataa
ccataagtatgattatcaaatgtttataacgcgtaataccatccagaaaatacgtggcaacaaaaagaaaaaccagcgcttta
ttccagatcggcttccatttctctttatttaacgttaatgatgtggttagcat
SEQ ID NO: 43 rfaP 
atggttgagctgaaagcgccgttaaccacactatggcgcggtaaagatgcttttgaggaagtaaaaacgttacagggcgaagt
gttcagagagctggagacgcgtcgaacattgcggtttgagctggacggtaaaagctacttcctgaagtggcataaaggtactt
cgctgaaagaaattgtgaagaacctgatttcgttacgtatgcctgttctgggcgctgacagagaatggcacgccattcatcgt
ctacatgagctgggcgttgatacgatgcacggcgttggttttggtgaaaaaggcgtaaacccgctaaccagaacatcatttat
tatcaccgaagatttaacgcccaccattagccttgaagactactgtgctgactgggctgttaatccaccggacgcgcaggtga
agtggatgatcattaagcgtgttgcgactatggtacgtaaaatgcacgccgggggaattaaccatcgcgactgttatatttgc
cactttcttctgcatttacctttcactggtcgcgaagaggatttaaaaatctccgtaattgacctgcatcgcgcgcagatacg
ccagcacgttccccttcgctggcgtgacaaagatttaattgggctttatttttcttcaatgaatattggcctgactcagcgag
atatattccggtttatgcgtgagtatttctctctccccctgcgagagattttgcaaaaagaatcggggttgattcatcaggca
gatgttaaagccgctcgaattaaagaaaggacaataagaaaaaatctgtaa
SEQ ID NO: 44 rfaQ 
gtggaaaagccatttcgaagaattctgattataaaaatgcgttttcatggagacatgttacttaccacccctgtcatcagcac
gctgaagcagaattatcccgatgcaaaaatcgatgtgcttctttaccagaacaccataccgatattgtcggaaaatccggaga
ttaatgcactctacggcatcagtaacaaaggcgcaggaacaaaagagaagatcaaaaacgcgctatcgctaatcaaaaaattg
cgtgctaactcgtatgatctggtcgtcaatctcaccgatcagtggagcgtggcgcttattgtgcgttttttaaacgcaaaaat
aaaaatctcgcaggatttcggtaatcgtcagtctgctctatggaaaaagagctttacgcatttagtaccttatgcgggagaac
atgctgttgagcgcacattatccgcgctaaagccgttagcgctaaaacagtatgtcacggaaaccaccatgagctatcgcccg
gaacattgggaaaacatgcgacaacagctcaaacaacttggtgtgacccgacaatatgttgttattcagcctacggcacggca
gctatttaagtgctgggataatgataaattctcgcaggttattgatgccgtgcagcgccgcggttatcaagttgtactgacgt
caggcccggccgcagacgagatggcctgcgtggatgctattgcacgcggctgtgagacaaaaccggttactggtctggcgggt
aaaactcgctttcctgaattgggtgcgttgattgaccatgcggatctctttatcggcgttgattcggcccccggtcatattgc
cgcagcagtcaaaacgccggtcatttgtctttttggcgcgacggatcatgtattctggcgtccctggaccgacgacatcattc
agttttgggcaggaaattatcagccgatgcctgaacggcatgagcttgatcgtaataaaaaatacctttccgttattccggct
gaagatgttatcgccgcgacggaaaaaatgctgccgcatgaggcacgcaccatagatttggacagcctgctatga
SEQ ID NO: 45 rfaY 
gtggaaaagccatttcgaagaattctgattataaaaatgcgttttcatggagacatgttacttaccacccctgtcatcagcac
gctgaagcagaattatcccgatgcaaaaatcgatgtgcttctttaccagaacaccataccgatattgtcggaaaatccggaga
ttaatgcactctacggcatcagtaacaaaggcgcaggaacaaaagagaagatcaaaaacgcgctatcgctaatcaaaaaattg
cgtgctaactcgtatgatctggtcgtcaatctcaccgatcagtggagcgtggcgcttattgtgcgttttttaaacgcaaaaat
aaaaatctcgcaggatttcggtaatcgtcagtctgctctatggaaaaagagctttacgcatttagtaccttatgcgggagaac
atgctgttgagcgcacattatccgcgctaaagccgttagcgctaaaacagtatgtcacggaaaccaccatgagctatcgcccg
gaacattgggaaaacatgcgacaacagctcaaacaacttggtgtgacccgacaatatgttgttattcagcctacggcacggca
gctatttaagtgctgggataatgataaattctcgcaggttattgatgccgtgcagcgccgcggttatcaagttgtactgacgt
caggcccggccgcagacgagatggcctgcgtggatgctattgcacgcggctgtgagacaaaaccggttactggtctggcgggt
aaaactcgctttcctgaattgggtgcgttgattgaccatgcggatctctttatcggcgttgattcggcccccggtcatattgc
 
cgcagcagtcaaaacgccggtcatttgtctttttggcgcgacggatcatgtattctggcgtccctggaccgacgacatcattc
agttttgggcaggaaattatcagccgatgcctgaacggcatgagcttgatcgtaataaaaaatacctttccgttattccggct
gaagatgttatcgccgcgacggaaaaaatgctgccgcatgaggcacgcaccatagatttggacagcctgctatga
SEQ ID NO: 46 rfaZ 
atgggcagcgttaacttcataactcacgccgatgttctgcaacttattgcgaaaagaacggctgaagattgtattatttttct
ttctggtcctacgtccagaaagacgccgctgtccttgttacgaatgaaagatgtcattgcagtcaatggttctgttcaatatt
tgctaaacaataacgtcaagccatttttgtaccttctgacggatattcgcttcctccatcgtcggcgcgaagatttttataac
tttagccgtaacagtcagtttactatcgtcaatcttgatgtgtacgaacaggcttcagtggatgatcaaaaatatattgaaga
aaactgtttaattattcgctccttttatcgccgagaaaaagggggctttttaaagaaaattaaatttaatatcctcaagcggg
ttcataaagcattacttatttcagttcctctttcgaaaagagggcgactggctggattttgcaaggatatcagcatcgggtat
tgctcttgccatactattgcttataccgctattcaggttgcctattccctcaagtatggccgtattatatgttctgggcttga
cttaacagggagttgcccacgtttctatgacgaatctaccagccctatgccatctgagcttagtaaagatctgtttaaaatac
tgccattttttacttttatgagaaaaaatgtgagtgatttaaatatctttaatttatcagatgatactgcaattcattacgat
attattccatatattacagcctcggagcttgaggatgaaatatattacgataaaattgtctag
SEQ ID NO: 47 yijP 
atgcaatccacattactccagactaaacccgcgtttagctggaaagccctgggctgggcactgctctacttttggttcttttc
caccctcctgcaggccattatttatctcaccggatacagcgggacaaacggtctgcgggattcgctactgtacagctcactgt
ggctgatcccggtctttctgtttccaggccgcatccgggttattgctgccgttattggcgtcgtactctgggccgcctcgctg
gcggcgctgagctattacgtaatctacgggcaggagttctcgcaaagcgtgctgtttgtgatgtttgaaaccaacgccaatga
agccagcgaatatttaagccaatatttcagtctgaaaattgttcttgtcgcgctggcttatacggtggcggcgattttgctgt
ggacacgtctgcgcccggtttatattccatccccctggcgttatctggtgtcgtttgccctgctgtacgggctgatcctccat
cccatcgcgatgaatacctttatcaagcataagtcgatggagaaaacgctggatagcctggcatcacgaatggaacccgccgc
gccgtggcaatttattactggctactatcagtaccgtttacagctcgcctcgctgaataaactgctgaatgaaaacgatgccc
tgccgccgctggctaacttccaggaccactccggcgatgcgccgcgcacgctggtactggtgattggcgaatcgacccagcgt
ggccgtatgagtctgtacggttatccgcgcgaaaccacgccggaactggacgcgctgcataaaaccgaccccgggctgaccgt
gtttaataacgtggtaacttcgcgaccgtacaccattgagatcctgcaacaggcgctaacgttcgcggacgaaaagaacccgg
actggtatctgacgaagccatcgctgatgaatatgatgaaacaggccgggtataaaaccttctggatcactaaccagcagacg
atgacggcgcgtaataccatgctgaccgtgttctccaagcagactgataagcagttctatatgaaccagcagcgcactcaaag
cgcgcgcgaatatgacagcaacgtgctggcgccgtttaaagcggtgttagccgacccggcgccgaaaaaattcattattgtgc
atctgctgggaacgcatattaaatacaagttccgctatccagaaaaccagggcaagtttgatggtaaaaccgaccatgttccg
ccgggattaagcagtgacgagctggaatcgtataatgattacgacaacgcgaacctgtataacgactatgttgtcgccagcct
gattaaggactataaagcgaccgatccgaacggtttcctgctctacttctccgatcacggtgaagaagtgtatgacacgccgc
cgcataagacgcagggacgaaacgaagattcgccaacccgccatatgtatacggtgccgttcctgctgtggacctcagagaaa
tggcaggcagcgcatccgcgcgacttctcacaggatgtcgatcgtaagtacagcagttccgagcttatccatacctggtcgga
cttagcgggcctgacctatgatggctacgacccgacgcgttccatcaccaacccgcagttcaaagagaccacgcgctggattg
gcaacccgtataagaaaaacgcgctgattgattatgatacgttgccgtatggcgatcaggtcggtaatcaatag
SEQ ID NO: 48 gmhA 
ttatttaaccatctctttttcgatcaactgaatcaaaatatgaattactttgatatgaatttcctgaatacgatcggcataac
cgaagtgcggtacgcgaatttcaatatccgccgttcccgccattttgccgccgtctttgccggtcaacgtaatcactttcatg
cctttttcacgggccgcggcaatcgctttaatcacgttaccagagttacccgacgtcgagatccctaacaggacatcgccttc
gcgccctaccgcctcaacgtaacgagagaaaatatagtcatagccaaaatcattactgacgcaggatatatggctaacatcag
aaatcgcaattgccgggtagcccggacgattttcacgataacgacctgtcagctcttcagcaaagtgcatcgcatcgcaatgg
gagccgccgttaccgcaggaaagtaccttaccacccgctttaaaactgtcagccaacaggaccgccgcgcgctgaatggcgtg
aatattggcgtcatcttttaaaaaattagccagcgtttccgccgcttcgttcagttcgttacgaataagatcctggtacat
SEQ ID NO: 49 yaeD 
ttatttttgctgcttttttatcgctgatggcaggtcagcaaggctgtttaacacccagtccgccgcgttttctgcttctgccg
tgaccggtttgcccgtacgcaccagcacttttgtcccgacatttgccgccgctgccgcctgcatatcttccagcttgtctccc
accatataagaagccgccatatcgatatgcaggaaatcacgcgccgagataagcattcctggatgcggcttgcggcaatcaca
tacctgacggaactcttctatactgccctgcggatggtgaggacaataatagatgccgtccaaatcaacatcgcgatccgcca
acgaccagtccatccattcggttaacgtttcaaactgcgcctcagtgaatttgccgcgcgcgattccggactggttcgtcacc
accaccaacgcatagcccattttttttagctcgcgcatagcgtcaataacgccgtctataaactcaaatgcatcgatttcatg
tacgtagccgtgatccacattaatggtgccgtcacggtcgagaaaaattgcgggtacaaactttgccac
SEQ ID NO: 50 rfaE 
atgaaagtaaatctgccagcgtttgaacgtgcaggcgtcatggttgtgggtgatgtaatgctggatcgctattggtatggccc
cacttgccgtatttcaccggaagcgccggtgcccgtggttaaggtaaataccgttgaggaacgcccgggcggcgcggcgaacg
tggcgatgaacattgcgtctctgggagcgaacgcccgtctggtcggcctgacgggtattgatgacgccgcgcgcgcgctgagc
aaaacgctggcggaggtcaatgtgaagtgcgacttcgtttctgtgccgacgcatccgacgattaccaaactgcgcgtactatc
acgtaatcagcagctcattcgtcttgattttgaagaaggctttgagggcgtggacccgcagccgttgcatgagcgtatcaacc
aggcgctgggatcgatcggcgcgctggtattgtccgattatgccaaaggcgctctgaccagcgtgcagactatgatttccctg
gcgcgccaggcgggcgtgccggtgctcatcgatccgaaaggaacggattttgaacgttaccgcggcgccacgctgctgacgcc
aaacctttctgaatttgaggcggtcgcggggaaatgtaaaagcgaagacgaactggttgaacgcggcatgaaactcattgctg
attacgacctttccgcgctgttggtcacgcgttccgaacagggaatgacgctgctgcaaccgaataaagcgccgctacatatg
ccgacgcaggcgcaggaagtttatgatgttaccggtgcgggcgatacggtgatcggcgtgctggcggcgacgctggcggcggg
aaataccctggaagaggcgtgttatttcgccaatgcggcggcgggcgtagtggtaggtaaactcgggacgtcaacggtttccc
ctattgagctggaaaacgcagtgcgcggacgcgcggataccggcttcggcgttatgaccgaagaggagttgagacaggccgtc
 
gccagcgcgcgtaagcgtggcgagaaagtggtcatgaccaacggcgttttcgatattctgcacgcgggccatgtctcttatct
ggcgaacgcgcgcaaactgggcgaccgcctgattgttgcggtcaatagtgacgcctcgactaaacgcctgaagggcgaaagcc
gtccggttaatccgctcgaacagcgtatgatcgtgctaggcgcgctggagtcggtcgactgggttgtctcttttgaagaggat
acgccgcaacgactgattgccggtattctgccggatctgctggtaaaaggcggcgactataagccggaagagatcgcgggcag
cgaagaggtctgggccaacggcggtgaagtcatggtgctgaacttcgaagatggttgttccacgaccaatatcatcaaaaaga
tccagaccgagagcgagaagtaa
SEQ ID NO: 51 rfaD 
ttacgcgtcgcggttcagccaggccatatactccgtgacgccttcggcgacggtcttaaagggtttgtcgtagcccgcgttgc
gcagattggttaaatccgcctgcgtaaacgcctgatagcgacctttcagcttatccgggaacggaatgtattcaatgctacct
tttttatggtatgccagcgtcgcgtcggcgacggcctggaaagattccgcacggcctgtgcccaggttaaagatgccggactt
gccgctttccaggaaccacaggttaacggcggccacatcgcccacgtaaacgaagtcgcgcttgaagttttcgctgccttcaa
acagtttcgggctttcgccgttgtttaactgtgtattcagatgaaatgccacgcttgccatgctgcctttatggccttcacgt
ggtccatagacgttgaaatagcggaaaccgacaatctgcgagttcgcttctggcaggatctggcgcacatattcatcaaacag
gaatttagaatagccataaacgttaagcggtttttcgtattcgcgcgattcgatgaaatcagacgtgcgaccgccataggtgg
cggcagaagaggcgtagaggaacgggatttcgcgctcaagacaatagtgcagcagctctttggagtattgatagttattatcc
atcatatacttgccgtcccactcggtggtggaagagcaggcgccttcatggaaaatagcttcgatatcgccgagctcttctcc
ggacataatctggatcaggaaatcttccttatccatatagtcagcaatgttcagatccaccaggtttacaaacttggtgccgt
ctttcaggttatccaccaccagaatatcggtgatacctttatcattcagggccttaacgatattgctgccgataaagcccgcg
ccgccggtaacgatgatcat
SEQ ID NO: 52 lpxA 
ttaacgaatcggaccgcgcgttgaacgctcaaagaactcggtaaacgccttcacttccggatgcttttcagcaagttcagcga
tttccagcttcgcttcgtcgagcgttttaccactgcggtacaacagtttgtaggcattacgaatcgccaccagcccttcacgg
ctaaagccgcgacgcttcagcccttcgatatttaccccgaacggcgtcgcatggttaccctgcgcaatcacatacggagggac
atcctgcgccacgccggagcagccgccgaccatcacatgcgcaccgataatgcagaattgatgaactgccgtcataccgccga
tgatcgcaaaatcatcgactgatacgtgccccgccagcgtggcgttgttggcgaggatacagcgattacctaccgtacaatca
tgtgcgacatgcgcattgatcatcagcaggttatcgctacccaccttcgtcaacccaccgccctgcactgtaccacgatgaat
ggtgacgctttcgcggatgcggttacgatcgccaatttccacacgggtcggctcacccgcatatttcagatcctggttcactt
caccgatggaggcaaactgataaatctcgttgtcgcggccgattttggtttgaccattcacgaccacatgagacttcagtacg
gttccctcaccaatttcgacctgcggcccaacaatacaaaagggaccaatgtgggcattagcgccaattacagcgccgtcttc
cacaatggcggtaggatgaataaaggcggatttatcaatcac
SEQ ID NO: 53 lpxB 
tcattgtgctaactccaggacggcatctgccgcctgctcatcggcattacagcggatctgctgatgcaattcacggaaggtgt
cgtgcatcgcatggctggttttaccgttcgccagtagcggcaacagcgcttcggcgagtttttgcggttcgcactcttcctgc
aacagctctttgactaattctcttcccgccagcaggttaggcaaagacacatactccgttttcaccagacgtttcgccagcca
gaaagtgaagggcttcatacgatatcccactaccatcgggcattttgccagcatacactccagtgccgccgtacccgaggcca
gcagagccgcgtcgctggcgatcatcgcctcgcgcgccatgccgtccagcaaatgaacggcgagatccggcgcaacctccgct
ttaattttttcaaactgctcgcggcgtttcgcgttcaccagcggcactaccacttccagatcgggatagcgctgtcgcagcaa
ttgtgcggttttcaggaaatcggcgctgagcatttccacttccgcgccacggctgcctggcagtagcgccagacaatgggcgt
catgcgggatacccagcacgtcgcgcgccgcatttttatccgggtccagcggcatggcatccgccatggtgtgaccgataaag
cggcacgggacgttaaatttatcgtaaaacgctttttcgaaaggcagaaaagccaacaccatatgggtggatctgcctatttt
gaaaacgcgtttctgtcgccaggcccaaacggacgggctgacataatgaatggtcttaatccctttttttttcagattacctt
cgagggtaatattgaaatcaggggcatcaatcccgacaaagacatcgggcttgagttcggtaaaacggcgggtcagatcggcg
cggatatgcaacaaacggcgtaaacgtccgagcacttcaacaatgcccatgaccgccagctcttccatctcgtaccaggcttc
acacccttcggcctgcatacgcggccccgcaacgcccacaaaacgggcgttgggtacgcgcgctttcagcgcacgaattaagc
ctgcgccaagaatatcgccggacgtttcaccggcgaccagggcaatcgttaaaggacgctgtgccgccat
SEQ ID NO: 54 lpxC 
ttatgccagtaccgtcgaaggcgctttgaaagccaacggcagttctgcgtcgtcctggaaggtcacaaattcccaggcttcct
gttttgccaggactgcctgtaacaatttattattcaatgcgtgaccggatttatacgcggtaaacgcaccaataatattgtga
ccacacatgaacaagtcaccgatcgcgtcaagcattttgtgacgaacgaattcatcttcaaaacgcaggccgtcttcgttcaa
tacgcgataatcgtcaacaacgatggcacaatcgaagctgccgcccaggcacaggccgcgggactgcagatattcgatatcac
gcataaaaccgaaggtacgcgcacggctgatctggcgcatgaacgcatccgcagagaagttcatcgcgtagcgctggctgctg
gaatcgatcgccggatggttaaagtcgatggtgaaatccaacgtaaaaccattgtacggtctgaattcagcccatttatcgcc
atcttcaacccgaacagtctctttgatgcgtacgaattttttggcgcagttcagttcatcaataccggcatcaagcagcaggt
agacaaacggagcggcactgccatccatgatcgggatttccggggcattaacctcaataacgatgttatcgatacctaagccc
gccagagcagcattcaggtgctcaacggttgaaatccgtacatcatgctcattcaccagacacgtacagagcatggtatcacg
cacagatttggcatcggccgggaaatctaccggtggattcaagtcggtgcgacgatagatgaccccggtattggccggcgcag
ggcgtaatgtcagggtgacttttttgccggtgtgtaaaccgacgccagtcgcctgaacgatacgtttaagtgtcctttgtttg
atcat
SEQ ID NO: 55 lpxD 
ttagtcttgttgattaaccttgcgctcaatcgctttgagacgcttgctcatatcatcaatgttcatcaccagtgcagcagttt
tacgccatactttgttgggttgcagcggaatgcctgaggagtagacgcccggttccgtgatgggacgcatcaccatacccatg
ccagttaccgtgactttgtcgcatatttccatatgcccattgatcacgctggcgccgccaatcatgcagtaacggccaatctt
caggctacccgccataatgacgccaccggcaactgccgtattgtcgccaatcacgacgttatgtgcaatctggcactgattat
caataatcacgccattgccaataacagtatcatccaacgcgccacggtcaatggtggtacaagcgccgatctcgacacgatcg
ccaataatgacccggcccagttgtgggatcttcacccagttgccacgatcgttagcgtagccaaaaccgtccgcgccgatcac
cgtactggactggatcaggcaattctcaccgatctgaatgtcgtggtaaatcgttacgttcgcccacaagcgtgaacccgccc
 
cgattttgctattttttccgacgaaacagcctgcgccgataaccacgttatcgcccagttgtacgccagattcaatcaccgca
ttcgcgccgactgaaacattgctacccagcgttgccgtcgcatcaatcacggcgcttggcgcgatattctgcgcgggctgcgg
cgtagtatctaaaatttgcgccatgcgcgcgtaggtcaggtagggatttttaactaccagcgccgcactcttagcaaaaggaa
gatcgtcctgcgtcatgacaaccgcagaagcctggcataaacctaagtgttcacggtacttaggattcaccataaacgtaatg
tggcctgttgttgcagattgcatggacgcaacgccggtgatgacgatatcgccatcaccgtgtaattctgcatccaactgttc
tgctaagtcagccagtcgaattgaaggcat
SEQ ID NO: 56 lpxH 
gtggcgacactgtttattgcagacttgcatctgcaaacggaagaaccggcgatcgtcgccggttttctgcgttttttagccgt
agaagcccgtcaggccgacgcgctgtatattcttggcgatctcttcgaggcctggattggcgacgacgaccccaacccgctac
atcgtgaaatggccgttgccattaaatcgctggttgattccggcgtcccctgcttctttattcatggtaatcgcgacttcctg
attggcaaacgctttgcccgcgaaagcggcatgattctactgccgcaggaaaaagtgctcgacctgtatggccgcaatgtgtt
gattatgcacggcgatacgctctgtaccgatgatgccggatatcaggcgtttcgcgccaaagtccataatccgtgggtgcaac
gactgttccttaccctgccgctgtttatccgccgccgtatcgccgccagaatgcgcgccggcagtaaagccgccaacagcagc
aaatcgctggatattatggacgtcaatgcgcagaccgtcgtcgccgaaatggaaaaacaccgcgtacagtggctgattcacgg
tcacacccaccgcccggcggtgcatgaactttccgccaacgaccagcccgcattccgcgtggtgttaggcgcatggcatcatg
aaggttcaatggtaaaagtcacgccggacaacgttgagttaatcgccttccccctgtaa
SEQ ID NO: 57 lpxK 
ctagcgaaccagcgaggtgatatgttgaagcaacttatcaggctgctcaccggacaaacgcgcatcaaccggtaagaaccacc
agttatcttcggcaaaagcacggcatttcaccgcgtctttttccgtcattaccagggtctgcccttcaccgaccaacgcctga
acgtcagcaggggcgagcgtttgatgatcggccagcgggacgcacttttgcggatgcgcgccacaggcttccagcgtggcgaa
aaagcgcggcggatgaccgatgcccgccatcgccacaatattgctcagttgcgccacatcgcaacgcgctcccgtacgtaagt
tcaccgccagtccaggcgctagctgcatggggatttcgcccgcccgggcgacgccgccattcacgatagcggcgtctacggtt
ttcaggcgactggcgcgttcgcgcataggaccggcaggaagccaccagccgttgccaaaacggcgcacgccgtcaatcaccac
aatttcaatatctcgcgcgagtcggtaatgctgcaacccatcgtcggtgataataatctgcacattatgcgctgccagaatcg
ctttcaccgccgcggcgcgttcaggcgctaccgcgacaggcgcgccggtccgttgataaatcaataccggttcatctcccgct
tctgcggttgtcgtctccggcgtcaggagcagcggataagcggcagctttcccgccgtagccgcgggaaacgacccccacgcg
cacgccacgctgttgcagcttttccaccagccaaatgactacgggggttttaccgttaccgccagccgtaagattccctacca
caaccaccggtaccggcgctcgccaggcgcgtttaaaaccaagcttatagctcagacgaatcgccccgctcaccaagccatac
agccaggagagcggcagcagtaaccgccatagcggggattcacccgaccagatacgcgcaatcat
SEQ ID NO: 58 htrB 
atgacgaagttgcctaagttctccgtggccttactgcacccgcgctattggttaacctggttgggtataggcgcgctttggtt
ggtcgtgcaactcccctacccggtcatctataaattgggttgcgcattgggccacctggcgcgacgcgtaatgaaacgccgcg
caaaaatcgcctatcgcaaccttgaattatgctttccggaaatgagcgcgcaggaacgccacaccatggtggtcaaaaacttt
gaatccgtcggtatgggcgtgatggaaaccggcatggcctggttctggcccgatcggcgagtgaaccgctggatggaagcgag
cggtctggagcatatccgtgaagttaaggcgcagggactggggttcattctggtaggaatacatttccttacccttgagtttg
gcgcccgcatgtttggtatgcataacccgggtatcggagtttatcgcccgaatgataatccgttgctcgactggttacagacc
tggggacgcttacgctccaataaatcaatgttggatcgcaaagatctgaaaggcatggttaaggcattgaaaagcggcgaatt
gatctggtatgcgccggatcacgattatggcccgcgcgccagcgtttttgtaccattattcgctgtcgaccaggctgccacga
cctccggtacctggatgctcgcgcgcatgtctaaagcctgtattatccccttcgtgcctcgccgtaaacccgatgggaaaggc
tacgaactgatcattcttcctgcggaatattcgcctccgctggagagcgcagaagcgactgccgcatggatgaataagattgt
tgagcagtgcattatgatggcgccagagcagtatatgtggctgcatcgccgctttaaaactcgccctgaaggcgtaccgtcgc
gctattga
SEQ ID NO: 59 msbB/lpxM 
atggaaaccaaaaaaaataatagtgagtatatccctgaattcgaaaaatcctttcgctatccacagtattggggcgcctggtt
gggcgcggcggcaatggcggggatcgcattaacaccggcatcattccgcgaccctttgctggcgacgctggggcgttttgccg
gacggctggggaagagttctcgtcgccgggcgctaattaatctgtcgttgtgctttccgcagcgtagcgaagctgagcgcgaa
gcgattgtcgatgagatgttcgccaccgcgccacaggcaatggcgatgatggctgagttggcgatgcgcggtccgaaaaaaat
tcaacagcgtgttgactgggaaggtctggagattatcgaggagatgcgtcgtaacgacgaaaaagtcatttttctcgtaccgc
atggctggggcgtcgacattccagccatgctgatggcctctcaggggcaaaaaatggcggcgatgtttcataatcagggtaat
ccggtttttgactatatctggaacacagtgcgtcggcgtttcggcggacgtttgcatgcgcgtaatgacgggattaaaccctt
tattcagtctgttcgtcagggctactggggttactacctgccggaccaggatcacggcccggagcatagtgaattcgttgatt
tctttgcgacatacaaagcgacgctgcctgcaattggtcggctgatgaaagtgtgccgcgcacgcgtgataccgcttttcccg
gtgtataatggtaaaacgcatcgcctgactatccagattcgcccgccaatggacgatctgctcacggctgacgaccacactat
cgccagacggatgaacgaagaggtcgaaatttttgtcggcccgcatccggaacagtacacctggatcctgaagctgctcaaaa
cccgcaagccaggcgagattcagccgtataagcgtaaagatctttatcccatcaaataa
SEQ ID NO: 60 lpxO 
atgttcgcagcaatcattatcggtatttttattatcagcgtcatttatgcgcattcacgcggggtggaaaaacaaaaactttc
ccgtcagctctttgaccactcgacgtttatggcgccgatcaatatgttcatgactcgcttctcaacgttaccggcaaagcagc
cgtactttgataccacggcatttccagagctgcagaagctgacggaaaactggcaggtgattcgtgaagaggcactacagctt
cagcatcacataaaagccgcgcaggccaataacgacgccggattcaatacctttttcaagcgcggctggaaacggttttacct
taaatggtattccgatgcccacccgtcggcggaaacgttatgcccgataacgacaaagctggtcaacagcatcccctctatta
aagcggcgatgttcgccgagttgcctcccggggcctatctgggaaaacatcgcgacccctatgccggttcagtacgttaccat
cttggattaagcacaccgaatgacgaccgctgttttattgaagttgatcgacagcgccatagctggcgcgacggcaaagccgt
catcttcgacgaaacctacgttcattgggcggaaaataaaactgagcagacgcgtattatcctcttttgcgatattgagcgcc
caatgaaatggcgctgggcgcagagcgtaaaccactgggtcggcgcatcgttgatgtccgccgccagttcgccaaacgacgag
aacgatcgcaccggtgcgattaatcgcatctttaaatacgttcacgcagcacgcgatgccgggcaacgtctgaaaaagaaaaa
 ccgcactctctattacgcgctgaaatatctcgttattgctgctatttttgccgcgataatccttttcagcctcctctga
SEQ ID NO: 61 lpxP 
tcagatgtagagtgacgcttcccccaacgggcgagtcttgaaacggcgatggatccagagatattgctccggggcgcgcatga
tctctttctcaataattttattcatataggcagcggcttgattttcatctgccgggtaaccttccatttcaggggtaatatac
agacgataacccgagttatcggatttccttaccattgtaacggtcaacatggcggcgccagacagcctggacagtacataggt
gccgttggtggtggcgacgttttcaaccgcgaaaaatggcgcaaaggagctgcctttagggccgtagtcctggtcgggagcaa
accaaacggcttcccctttttttagcgcgcccacaatgccgcgcagattgtttctgccgatcatcgctttatttgagcgcata
cgaccgcgcgtctgcacccactccatgagcggattgttatgcggacgataggtcgccatcatcggctggcagagacccattac
ccggcctcccaattccagcgacataaaatgcacgccaacgaccattacgccgcgattttgcgcctgcgcacgggtaaggttgt
ccagcccgtccacatcaaaccatttgcgaacccggctatcaggccagaaccaggccatgcccgtttcaagcagcgccatgcca
agagagtggaaattctcggcgatcagtttttccctttcttcccgggagagggtcggaaaacagagttcaatatttttctgggc
gattgactcacgtcgcttcaggaaggggcgcgccagtttaccggtgcgggtgcccagaaaacgcaaaaccgggtagggaagct
gcaccagtaaccagaggatgccaacgccaaaccacgtcagccagtagcgcgggtgcaaaaacgcgcgtgaaaatttgctttga
ggaaacat
SEQ ID NO: 62 yeiU 
ttatttgtttaaaatttgtttatttccgcccgggagataattctcaaaaagcgctatcgcccggtcgctgagcggggtcatca
gccaccaggggaggccaatcaaaataaccgtgagcgacccgaccacaatatcggtgaaccagtgagcgccaatcataacgcgc
ggaaacgcgaaaaccacaaatataataagggcgataatccccgcagttttgccgaaataacgcagcataaaggcggaaaatat
cagcagcatcataccgtgatcgcctgggaaactgtcttttgacgcgtcttttgtcgggatgtgcagcaactcgctgacgcgat
aaatatgctcaaaagagagcgttgggctggcgcgcttgacggggatcagcgcctgaccgagttggttaagcaccaccgctgtt
agtagcatgaccaatccgattatcacaatgcgccgccggcccgatgcgttttcttttagccagaaactgagcatcagtccgcc
catcgccagcagcgaacagccgtcaaacgcccgattattggtaatagcgacccaccagaggaacgcgtggctttccaccagtt
tctggttaaagaagtggaatatgccggaatcaatggtgaaccagaagccgtgattgacgggaagataccatgacaaaaacagc
gccagaccggcggcattaagcagaattatcaaggaatagcgggttttcatcgtcat
SEQ ID NO: 63 kdtA 
tcaatgcgttttcggtggcagataaggttccagcagttgcagcagacgctggagcgcgccctgattttgatacagcacttcaa
ctgcgtgacgtccgtagaaattacgataatcagcgtcggtcagtaaagaggaaacttcttttgccagcgtagccgcatcggta
atcgtgataagtccgctcgcctgatccagacgggcgcaaatatctttaaagttaaaggtatgcggacccatcagtaccggaat
cgcatgagcggcggcctccagcgggttatgaccgccgcgttcaaccagcgaaccaccaacaaaggcgagatcggcaatgccat
agagcaacattaattcgcccatggtatcgcctaccacgacctgggtgctggcggacggtacttcgcccgaagaacgagtgatg
tagcttaaccctgcctgacgcacaagattgatagcatccgggaaacgctccggatggcggggcaccagaatcagtaataaatt
cgggaattgatgtaataacgcctggtgagcggcgataacgatactctcttcgccatcgtgggtgctggtggcaatccagaccg
gacggtgcggcgcccactggcggcgtagcgtaacggctttagccgccagctgcggcgtaactgaaatatcaaatttcagactg
ccggtgaccgtgacctgattgttcttcgcgcccaatgccacaaagcgttcgccatcttcttcgttttgcgcggcaatcagggt
gatacggcgcaagagcgtacggacaaacttgccaagcttcgcataacccgcggcggagcgggcggaaagccgcgcattagcga
taaccaggggaatatgacgtttgtgcagcgcagcaatcagatttggccagagctcagtctccatgatcaataccagcttagga
tcaatcttattgaggaaacgattgagcgcatcgggcaaatcataaggcaagtaaacgtgctgaacatcgttgccaaaggcgga
ctggacgcgctccgagccggtcggcgtcatggtcgttacggtaataggcagatcgggatagcgatgacgtagagcgcggacca
atgggatggccgccagagtttcgcccaccgagacggaatgcagcatgattccgcccggtttcaacggacggcggtagaatccg
tagcgttcaccccagcgcttacgataggccggcgctttacgtccacgcacccaaagccgtatccagatcagaggctgaataag
gtagagaagagcggtgtaaagcaattcgagcat
SEQ ID NO: 64 arnT 
tcatttaggccgatactgaattaacaccagacgcccctgataatcaatattgtccgcagggggaagggaaagagcgctgatgt
cttcatctttggctattgaaagtactagcgtaataatgccctcctgacgatgctggttgagccaggcgttaaaatcgtcggct
ttaacgaatttatcctgcacatcaggatagctaagaccatacctgagttcgcccgcgtgtccgtagagcataatatcgtctcg
tttcagactccaggccagcccggcggcgacgcccacgttgtcggcaagaatatagcggcttgatgccaaaggcgcctgggtca
tttcgacaaaaaactgtggctgtttggactccatcaccctgtcggggatggaaaagccgaacagcagcgccagtcccagcgga
caaaacgcgggtaataggtatttttggctatgacagaggctataccagcccacaaacgcccaaacagtaaagacgccccagac
gcaaaaaactttataggtttcgatatgcgtccacaccggcgatttcagcgggccccatgaggagacgacaaacgctgcgacga
tcccgactaacccgaacaccaggttgatcccgccgttgacacgtagcgcggcgacgccttcttttacgttatgcaggacgaag
cgcgccatgagtatcgctatcggtgcgaaacaggaaagaacataggtgggcagtttccctttcgcaatgctgaaaaagagaag
cggcatgatcgtccatccgagcaaatagaatgcgccgtttcgctcgcgccagcccagtttaagcgcgccaggcagtaacccca
gccacggtaaacttcccgcgagcagtaccggcagatagtaccagaaaggggccttatgctgggcatcgctcatggcgaatcgc
tggatatgctccacccagaaaaagtaatgccagaaatcggcttcgcgccgcgcaatggcgatcgcccagggaaggacaaccac
aaagcagctcaagaccgccagccagccgtaaagcaaaaagtccttccagcgtttctggacaatgacccacggcagcacgctca
ccacaggtacggccagggcgaggaagcctttagttaatacacccagaccgcaggttgcgcccagtagcaaaaacatcccaatc
ttgcccattcgggtcgtggcctgcatcccctgccagaaacaacacatcccggccgtaagccaaagggcgatcatcgggtcgag
taccgcatacgtcccgatactgtaaacggcaaagagggagagaaagataacggaggcgagcaacgcggtacgtttatcccgcc
ataagcggaacgtcagccatgccaccagcgctgcggccagcaatgtcgttaaaatagcgccagcccgcacgccaaaattggta
gcgccaaaaagccactgacccagactgtttatccagtaaccggctatcggtttttcaaaataacgtaatcccagaaaatgcgg
cacgatccagtcgccagacgccagcatctcccgacttatttcggcatagcgcgtttcatccggctgccagagcaggcggctgt
tgacaggaataacatagtagagcgcgataaacgcggcgaaagccagataatagcgtatcgatttcatcat
SEQ ID NO: 65 pagP 
tcaaaactggaaacgcatccaggcgaaatagacgtttccgttgttatacgaacccggaatgtaggtcatctgaaaggtagcgg
gaccatagcctatcgacgctaaaggcaacagcacagggatgggaatataattccagttgtcgcgcgcggtaacgcctgcggta
aagcccagtccaaggcgaaaattatcgtcttccagcggtcgccaggttttttcccagccataaccgcctatgggttcccattt
gttaaatgaatctttaaaggccatcatatagaggccatgccagttgcctttgtcgtcccagcgggactgaccaaaaccaacgc
cccacggccgctcgttatagcgatccgttttctctttatcgtaggcaaagcgcgcatgccaggtaatggcggggacatacaaa
tcataatgctcaggctgtcgccacgtttctgcgacgttatcggtaaacgtgttaaacccccctttatccgccgcagagactga
aggaatagccagccatggcatcaaaaggagagcaataataagaaaatactttctgatgatcatcgcaacatacat
SEQ ID NO: 66 yjdB 
atgttaaagcgctttcttaaaagacctgttcttgggcaaatcgcctggcttctgcttttttccttttatattgccgtctgcct
gaacattgcgttctacaagcaggtactacaagacctaccgttaaactcgctgcgcaatgtgctggtgtttatttccatgccgg
tcgtcgcgtttagcgtggtcaatagtgtgctgacgctggcctcatttatttggctaaaccgactgctggcctgcgtttttatt
ctggtcggcgctgccgcgcagtattttattttgacttacggcatcatcatcgatcgctccatgatcgccaatatgatggatac
cacgcccgcggaaacctttgcgctgatgacgccgcaaatggtgctgacgctgggattaagcggcgttctggcagccgtgattg
ccttctgggtcaaaatccgtccggcgacgccgcgcttacgtagcgggctttaccgcctggccagcgtcctgatctctatttta
ttagttattctggtcgccgcctttttctataaagattacgcctcgttatttcgaaataataaacagttgatcaaagcattaag
cccatcgaacagtattgtcgccagttggtcatggtattcacatcaacggctggcgaatttgccgctggtacgcattggcgagg
atgcccatcgcaatccattaatgctgaaaggcgatcgcaaaaacctgacgattctcatcgttggcgaaacctcgcgcggcgat
gatttctctcttggcggctatccgcgcgacaccaatccgcggctggcgaaagacgatgtgatctacttcccgcataccacctc
ttgcggtacggcgaccgcgatctccgttccctgcatgttttctgatatgccgcgcaaacattatgacgaagagctggcgcatc
accaggaagggctgctggatattatccagcgcgcggggattaacgtactgtggaatgataatgacggcggctgtaaaggcgca
tgcgatcgcgttccgcatcaaaatgtcaccgagctaaacctgcccggacaatgtatcgacggcgagtgctacgatgaagtgtt
atttcacggtctggaagactatatcgaccatttgaaaggcgatggcgttattgtattacataccatcggcagccacggcccaa
cctattacaaccgttatccaccgcagtttaaaaaatttaccccaacctgtgacactaacgaaattcagaactgttcccaagag
cagttgatcaacacctatgataataccgtgctgtacgtggactatattgtagataaagccattaacctgctgaaatctcatca
ggataaattcaccaccagcctggtctatctttccgatcacggcgaatcattgggtgaaaatggcgtttatttacacggtttgc
cgtattccattgcgccggatacgcaaaaacatgtcccgatgctcatttggctttctaaagattatcaacagcgttatcaggtg
gatcaggcctgcctgcaaaaacgggcgagcacgctggattattcacaggacaatcttttctcaaccatgctgggattaaccgg
cgtacaaacgacgtattaccaggccgcagatgatattctgcaaccgtgcaggagactaagcgaatga
SEQ ID NO: 67 yhjw 
atgagatacattaaatcgatgacgcaacagaaacttagtttcttgcttgcgctctatatcggtctgtttatgaattgcgccgt
gttttaccgccgtttcggcagttatgcacaagaatttaccatttggaaaggcctctccgcagttgtcgaactgggcgccacgg
tgctggtcactttcttcttacttcgtcttctttcactgtttggccgacgcgtctggcgtgtgctggccacgctggtggtgctg
ttttccgctggcgccagttattacatgaccttcctgaacgtggtgattggctacggcattattgcgtctgttatgaccaccga
tatcgatctctcgaaagaggtggtggggctgcactttgtattgtggctgattgccgtgagcgtgcttccgctcatctttatct
ggagtaaccactgtcgctacacgttgttgcgccagctacgtacgccggggcagcgttttcgcagcgccgctgtagtggtactc
gcaggcgtaatggtgtgggcgcctattcgcctgctggatatacagcaaaaaaaggttgaacgggcgacaggcatcgacttacc
cagctatggcggcgtggtggcgaactcctacctgccctcaaactggttatctgcgttagggctgtatgcctgggcgcaggtag
atgagtcgtcggacaataattcgttaataaacccggccaggaaatttacctatgttgcgccgaaagatggggatgacacctac
gtcgttttcattatcggtgagacgacccgttgggatcacatggggattttcggctacgagcgtaataccacgccgaagctggc
gcaggaaaaaaatctggcggcgttccgcggctattcctgcgataccgcgacgaagctttctttacgctgtatgtttgtacggg
aaggcggggcggataataacccgcagcgtaccttaaaagagcagaatgtttttgccgtactcaaacagctcggattcagttcc
gatctgtacgccatgcagagcgagatgtggttctatagcaataccatggcggataatatctcttaccgcgagcagattggcgc
cgagccgcgtaaccgcggtaaaacggtggatgacatgctgttgattgatgagatgcagaactcgcttgcccagaatccggagg
gtaagcatctgattatcctgcataccaagggatcgcattttaactatacgcagcgttatccgcgcagctacgctcagtggaag
cccgaatgtattggggtcgatagcggctgcacgaaagcgcagatgatcaactcttacgataactccgtgacctatgtggatca
ctttattaccagtgtgttcgatcagctacgtgataaaaaagcgattgtgttctacgcggctgaccacggcgagtcgattaacg
aacgcgaacatttgcacggtacgccgcgcaatatggcgccgccggagcaattccgtgttccgatgctggtatggatgtcggat
aaatatcttgccagtccgcaacatgcgcagatgttcgctcacctgaaacagcaggcggagatcaaagtgccgcgtcgtcatgt
ggagctatacgatacgataatgggctgcctggggtatacatcgccgaatggcggcattaaccagaacaacaactggtgccata
ttcccgatgtgcagaaagtcgccgcgaagtag
SEQ ID NO: 68 kdsA 
atgaaacaaaaagtggttaacattggcgacattaaggtggcaaatgacctgccgtttgtgctgtttggcggtatgaacgtgct
ggagtcacgcgatctggcaatgcgcatttgtgagcactacgtaaccgttacccagaagctgggtattccttacgtgttcaagg
cctcttttgataaagccaaccgttcctctattcactcttaccgtggcccgggtctggaagaagggatgaaaatcttccaggaa
ctcaagcagacatttggcgtaaaagtgatcactgacgtccatgaagccagccaggcgcagcctgttgctgatgtggttgacgt
gattcagctcccggcgtttctggcgcgccagaccgacctggtggaagcgatggcgaaaaccggcgctgtgattaacgtgaaaa
agccgcagttcgtaagcccgggtcagatgggcaatattgtggataagttccatgagggcggtaacgataaggtgattctgtgc
gaccgcggcgcgaacttcggctatgacaacctggtggtggatatgctgggctttagcgtgatgaaaaaggtctccggcaactc
gccagtgattttcgacgtcacccacgcgctgcaatgccgcgacccatttggcgccgcctctggcggtcgtcgtggtcaggtga
ccgagctggcgcgcgccggtatggctgtcggtctggcgggcctgttcctggagtcgcatccggacccggctaacgcgaagtgc
gacggtccttccgcgctgccgctggcgaaactggagcagttcctgacccagataaaagcgattgatgatttggtgaaaagctt
cgacgagctggataccgagaactga
SEQ ID NO: 69 kdsB 
ttaacgcatttcagcgcgcacgcgctccaggtcgtcggcggtgtctacgcccgtgccgggcaccgctttggcgacggcgacat
gaattttttcgccgtaccacagcacacggagttgctccagcatttcaatatgttccagcgggctgggttgccagctcacgtaa
 
cggcggataaaccccgcgcgatagccataaatgcccaggtgacgaaggcaagtatcccctaccgtttccagactttttgcaaa
gcgatcgcgatcccagggaatcgtcgcccgggaaaagtaaagcgcatagccttcagcatccagcacgacttttaccgcattcg
ggttaaacgcttcttcggcgctgtgaatcggcaccgccagcgtcgccatgccaacctggcgctgcgccagattttccgcgacc
tggcgaataataaccgccgggatcatcggctcatcgccctgaacgttcacaataacggtatcgtcgctgaatccgcacttctc
taccacttccgccaaccgttcggtgccggactggtgatcggcgcgcgtcatgcacacttcgccgccagccgcttctaccgcac
gagccacgtcttcatgatcggtcgcgacaataatgcgttccgcgccagattcgcgcgcccgctccaggacatgaacaatcatg
ggcttaccattaatatccactaatggtttgcctggcagtcgggtagatgaaaaacgggccggaatgatgacgacgaaactcat
SEQ ID NO: 70 yrbI 
tcatatcgattgccctttggcctcatccagcttgccctgcgccagcagcagcaaatcgcacacctcacgtaccgcgccgcgcc
cgccggcaatgtgcgtcacatagtcagcgcgggggatcagcagtggatgcgcatccgcaacggcaacgcttaaccccactttt
tccatcactggccagtcaatcagatcatcgccgacataggcaacattttccggcgcaatggccagtttttccagcagatcgct
aaaggcgatcaacttattcgactgtccctgataaagatggacaatccccagcgtggcgcagcgatcttctacaagtttagcct
ttcgtccggtaataatggcaacctcgatattggaggtcaaagcacagcgaataccatagccgtcacgaacgttgaaagccttc
agctcttcgccgttattgcccatataaatcaggccgtcagacaatacgccatccacgtccaggatgagcaggcggatattctc
cgctttggtcatgacatgggggctgacggggccataacaggtcggaagcgacgcacctgctttactcat
SEQ ID NO: 71 yrbH 
ctacacgacgcctgcacgcaggagatcatgcatatgtaacacaccgagtaactggtcgccatcagcaaccagtacggaggtga
tatggcgggactgcattaaattcagcgcatcgacagcgagaataccgggacgcacgcgaatgccccctggcgtcatcacctcg
gcgatccccaactggcgcatatcgccgcccatatcgaatacgcgacgtaaatcgccgtcggtgaagataccgtcgatcttcat
ggattcatcgcaaatgacggtcataccgagatttttacgcgtaatttctaatagcgcatcacgcagggtggcatgtttattaa
catgcgggatctcatcgccagtatgcataatatcgcttacgcgcagcagtagcttacgtcccagcgcgccgccaggatgcgac
agagcgaaatcctcggcggtaaagccgcgcgcttttaacaacgccacagcgagcgcgtcgcccatcaccagcgttgcggtggt
actactggtaggcgccagtcctaacggacacgcttctttgggcactttaacacacagatgcacatctgccgcacgcgccatgc
tgctttccggtcgcccggtaatgcaaattaacggcacgtgcagacgtttaagtaccggtattagcgccgcgatctcgctggat
tcgccggagttggagatagcgataaccacatcctgcggcgtaaccatccccaaatcgccgtgcgcggcttcgcccggatgtac
aaaaaaggaagaggtgccggtactggcgaacgtggcggccattttccgtccgatatggccggatttccccatccccatgacca
ccacttttccggtgcagttgaacattttttcacaggccagcgtgaaatgctggttgatatattgatcaagctccgccaggcct
tcacgttcaatttccaggacctctttgcctgcctgctgaaagtcaaaccccggttgcaacgctaaatgcgacat
SEQ ID NO: 72 msbA 
ctacacgacgcctgcacgcaggagatcatgcatatgtaacacaccgagtaactggtcgccatcagcaaccagtacggaggtga
tatggcgggactgcattaaattcagcgcatcgacagcgagaataccgggacgcacgcgaatgccccctggcgtcatcacctcg
gcgatccccaactggcgcatatcgccgcccatatcgaatacgcgacgtaaatcgccgtcggtgaagataccgtcgatcttcat
ggattcatcgcaaatgacggtcataccgagatttttacgcgtaatttctaatagcgcatcacgcagggtggcatgtttattaa
catgcgggatctcatcgccagtatgcataatatcgcttacgcgcagcagtagcttacgtcccagcgcgccgccaggatgcgac
agagcgaaatcctcggcggtaaagccgcgcgcttttaacaacgccacagcgagcgcgtcgcccatcaccagcgttgcggtggt
actactggtaggcgccagtcctaacggacacgcttctttgggcactttaacacacagatgcacatctgccgcacgcgccatgc
tgctttccggtcgcccggtaatgcaaattaacggcacgtgcagacgtttaagtaccggtattagcgccgcgatctcgctggat
tcgccggagttggagatagcgataaccacatcctgcggcgtaaccatccccaaatcgccgtgcgcggcttcgcccggatgtac
aaaaaaggaagaggtgccggtactggcgaacgtggcggccattttccgtccgatatggccggatttccccatccccatgacca
ccacttttccggtgcagttgaacattttttcacaggccagcgtgaaatgctggttgatatattgatcaagctccgccaggcct
tcacgttcaatttccaggacctctttgcctgcctgctgaaagtcaaaccccggttgcaacgctaaatgcgacat
SEQ ID NO: 73 wzy 
atgcttataatttcatacattgcattatgcttattatttatagtttatctctatactctttccgtaagaattgaggggaaaat
aataaatgtaatggtcccatacctgataataacagtccctacactgtacgtgtttgaaggtatatttgtatacctctcagagg
tgcggaattatacagtggaatatttgtttttctatacttgctatataacatacatagcatcatttgttatttcttatctttac
acacaaagaaaacccatatacaacaaatcaaacacgaaaaataaaccaaggtatgtgtttacttcattgttattcaccttcct
tgcttttatcatttaccttccagtgttgatggagttcagagagtatatacttagcccaagaagaatatacgaattaaccagaa
cagggtatggtatatacttctatccttcattaatgttttctcttgtcgcttctatttgcgcgttctttacatacaaaaaatca
aagttattttgtatttccatagttttatttaactgtatacttattttcttgcatggtaacaaaggaccaatatttagtatatt
tatagcattcatcctttacctttcatatattgaaaataaaaaaattaaatttatgttcctggtaaaatcgtttgctgttatag
cagtcattgtaacggcattctttgcatatacgtttactgatgggaatccgatagaaaatatggcgaattactcggattatacc
cgtaatgctgttcttgttgcttcctcaaactttgactttatgtacggaaaattactaatggaaagcgaggtttactcgaggat
tccgagggctatttggcctgataagcctgaagattttggcgcattgtatctggcaaaagtatttttccctgatgcattctaca
gaaatcagggcgctcctgctttcgggtatggtgaactatacgcagatttcgggctttttacaccagtttggttagttatatct
ggagtatttaaaggcgtcctagctaagtatttctccaataaaactcaggaaacaaagtcagcgcattatttcataatgttcct
attttgcattggaataagtgtgattcctgttagcatgggatggttgttccctgagcatttgatgattgcttttatggtataca
ttgcatcttcctttgttttttcagagcatataagattcgttttactaagaaacaataaataa
SEQ ID NO: 74 wzxE 
tcatgctcgcctacgccagagtaaaaatacgccgcaacaaagtgaaaaatagacaatataggtggccatataagcctgtgccg
cacccagcgcgccgtgagcgggaatcagccagtgagcgaacgccgttaacagaatgaactggctaatttcagccagaatgtaa
aatcgcaacgacgctttggcaatcaccagataaccgaaaacataggcgccgactttcagcacgtcgcccactaactgccaggc
gaacagatcgcgcatcgcggtgaatttagcggaaaaaagcagccagatagcaaaatcgcgcagtagccagacggtaaaactcg
ccgccgcaaccgccggtaaaacaaatttcagcgcttttaccacttcacgcgtaatgtcctgtttttccgtcaggcgtgagagc
gtgggtaacaggtaaacgctgaaggaggcggtgataaattgcagataggcgtcagaaatactgcttacgccctgccagatccc
gacatcgctccagctatagtgcgcggccagttggtttcgcatcatcacataggcaaccggcatcgtaacggaggtaatcagcg
ccatcagggtaaatttactgagctgccctgccagaccgttgtcccaggagggcttgagatagcgtagcgggacggtgccgcgc
 
ttaatcagtattataccggctggcactaccaccagcgccggcaccagcgccaggccaagcagagcgccttcatagccgccgac
acggtagcagagataccaggcggcgacgcccaaaaagctgccgacaattaacgataaggcattacccgacgcgtcacgaaagc
ccttcattagcgccagcagcaggttcgcccaggcgatccccatttgtaccagcgcaaccagacgcaccagcccttgatagtcc
gtatgaccgaacaatccctggctaatcggcgccgccgccagcaagaagaccagcgccagcagcgtagagaaccccaataccat
cgcggaggaggttcccaccacggtacgtaactgcgccgggttatcatgatgttgtgcgacaagtttggtaacgccgttaaata
tcccggcgccagccaatacgcccagcacggtaatcatctggcggaagttacccgcttgtccgacgcccgccggaccaaaagag
acggccagtagtttcaccaccagcagtccggcgccaatttttaccagcgtactgaccgccgtccacagggaggctttagcaag
cgacat
SEQ ID NO: 75 lptA 
ttagttactcttcttttgcgccggagtctggcctttgtttttgtcttgcaactgcgacggcaccagaacggtggtgacgcgtt
tgcctttctcgctgaaggcctgcattttctgctctttcaccaggtaggtgattttgtcgccggtaatgttgctgtcaagctgc
tccagataagcgttgccggtcagcaccacaaaatctttcgccagctcgtagtgcatatgggaagcgtggcctttcaccggctt
accgttgtcctgcatctggtagaaggtggccggattgccgtaaccgtcgatcacctctttcccctgttcgccgccagggcggg
taaccacgactttatcggcgttgatcttaatcgtgccctgggtcatcaccacgttgcccgtaaaggtcacgacgttgccttgc
atatccagcgactgttgatccgagtcaatatgaataggctgttcggtatcgcccgtgacggcgaaagccggaatgctggcggc
cagcagcgagccggcaagcataagattaaggctgagtttgtttgttttgaatttcat
SEQ ID NO: 76 lptB 
tcagagtctgaagtcttccccaagatatacacgcttaacgtgttcgtcttgcaggatttctgtcggcgtaccgtgggcaataa
ggtggccctggctcacgatataggcgcgttcacagactgccagcgtctcacggacgttatgatcggtgatcagcacgccgagc
ccgctatcgcgcagatgctcgataatgcgtttgatgtcgataacggaaatcgggtcaacgccggcaaagggttcatccagcag
gataaatttgggattagcggccaacgcgcgggctatttctacacgacggcgttcaccaccggacagcgcctgtcccatgctgt
cgcgcagatgctcgatatgaaactcttccatcagctcgttggcgcggtcttctcgctgttctgcggtcaaatcgtcacgaatt
tgcagtaccgccatcaggttatcaaatacgctgagacggcggaaaatagaggcttcctgcggcaaatagccgataccgcggcg
cgcgcgcgcgtgcagcggcagcagactgatatcttcatcatcgatgatgatattgcctgcgtcacgcggcacgataccgacga
ccatataaaaggtggtggttttacccgcgccgtttggaccgagcagcccgacaatctccccggagttaacggtcagactgaca
tcttccactacgcgacggcctttataggccttcgccagattctttgcagttaatgttgccat
SEQ ID NO: 77 lptC/yrbK 
ttaaggctgagtttgtttgttttgaatttcataagaggttctaaccttttcaatcagctcggcgttcttgctgcgtaagttac
cgcgcattttcagtccgctggagttaaatgttgtgccgtataacgtcaccagatcgttcgaggtaacatcctgcgtcaccaga
ttaatctgcgcgttatcggtggtaattctgcgtaattgagcgtcaggcaccagggcgttgacttcaacatggccatacagata
gagcatccgatcgttagtcaatttggctttatcggccttgatcgaccatgtcggcactttatccttatcaaaggtggttaata
ccggttgcgtaaaccatgagaccgcctgatcggaaaaatattcaacgtgctgcgcgatcagacgatagctcaacgcgccttcc
ggactgtacacgacggtatccgtatgctcgcttttatacgtgggatcgttgctgttgaccatcacggcagcaggatcgtcttt
atccgccagattgataccaatcagtaccagaatggccagcgacagtagaatgataacccaacgtctggttttactcat
SEQ ID NO: 78 lptD 
atgaaaaaacgtattcccactcttctggccaccatgatcgccagcgccctttatagtcatcaggggctggcagccgatctcgc
ctcacagtgtatgttgggcgtgccgagctacgatcgtcctctggtaaaaggcgataccaacgatctgccggttactatcaatg
ccgataacgctaaaggtaactacccggacgatgccgtttttaccggcaacgtggacattatgcaggggaatagccgcctgcaa
gcggatgaagtgcagcttcatcagaagcaggcggaaggtcagccggaacctgtacgcaccgtcgatgcgctgggtaatgtgca
ttatgatgacaatcaggtcatccttaaagggccgaagggctgggcgaacctgaacaccaaagacacgaacgtctgggaaggcg
attaccagatggtgggccgtcaggggcgcggtaaagccgatctcatgaagcagcgcggcgaaaaccgctataccattctggaa
aacggcagctttacctcctgtctgcctggctccgatacctggagcgtggtggggagtgaagtcatccatgaccgcgaagaaca
ggttgcggagatctggaacgcccggtttaaagtaggtccggttccgatcttttatagcccctatttacagctacccgtcggtg
acaagcgtcgctcaggtttcctgatcccgaacgcgaaatacacgaccaagaactatttcgagttctacttaccgtattactgg
aacatcgcgcccaatatggacgccaccatcaccccgcactatatgcaccgccgcggcaatattatgtgggagaacgaattccg
ttatctcacgcaggcaggcgagggagtgatggaattagattatctgccttctgataaagtctacgaggacgatcaccccaaag
agggcgataagcaccgctggttattcaactggcagcactcaggcgtgatggatcaggtgtggcgttttaacgtcgattacacc
aaagtcagcgactccagctactttaacgatttcgacagtaagtacggttccagtaccgacggctacgcaacgcagaaattcag
cgtcggctacgccgtacaaaactttgacgctacggtgtcgaccaaacaattccaggtctttaacgatcaaaacaccagcagct
attcagcggagccgcagttagacgttaactactaccataacgatctcggcccgtttgatacccggatttacggccaggcggtg
cactttgttaacaccaaagacaatatgccggaagcgacccgcgtccacctggagccaaccatcaatttgccgctctccaaccg
ctggggcagcctgaacaccgaagcgaagctgatggcgacgcactatcagcaaacgaatctggacagctataacagcgatccaa
acaataaaaataagctggaagattcggttaaccgcgtcatgccgcagtttaaagtcgacggtaagctcatcttcgaacgcgat
atggcgatgctggcgccggggtatacccagacgctggaaccacgcgtgcagtacctgtatgtgccgtaccgcgaccagagcgg
catctataactacgattcttctttgctgcaatccgactataacggcctgttccgcgaccgcacttatggcggtctcgaccgta
ttgcttccgccaaccaggtcacaaccggcgtcacaacacgcatttatgatgatgccgccgttgaacgttttaacgtttctgtt
ggtcaaatctactatttcacggagtctcgcaccggcgatgacaacattaaatgggagaatgacgacaaaaccggttcgctggt
ttgggcaggcgacacttactggcgtatttcagaacgctgggggctgcgtagcggagtgcagtacgatacccgtctggatagcg
tcgctaccagcagcagcagcctcgaataccgtcgggatcaggatcgtctggtacagttgaactaccgctatgccagcccggaa
tatattcaggctacgttgccttcgtattattccacggcagagcagtataaaaacggcatcaatcaggtgggcgcggtggcaag
ttggccgattgccgatcgctggtcgattgtcggcgcgtactacttcgataccaattcgagcaaacctgcagaccagatgctcg
gcttgcagtacaactcttgctgctatgcgatccgcgtcggatacgaacgtaagctgaacggttgggataacgataaacaacac
gcgatttatgataacgcgattggcttcaacattgagctgcgcggtttgagctctaactacggcctcggcacgcaagaaatgtt
gcgttcgaacattctgccgtaccaaagctctatgtaa
SEQ ID NO: 79 lptG

ttacgacttacgcagcattagccagaggctaatcaggaagaaagacgcgctcggcagcagcgcgccgacaatcggcggaatgc

cgtataccagcgtcagcgggccaaaaatctggtcgagaacgtaaaagacgaagccgaagctaatccccgtcaccacgcgcacg
cccatcggcacactacgcagcggtccaaagataaacgacagcgccatcagcatcatcaccgccacggagagcggctggaagat
tttactccacatattgagctgataacgtccggcgtcctgtccgctcgacttcagatatttaacgtagttatgcagaccgctga
tggaaagcgcatccgggtccagcgccaccacgcctaacttatccggcgtcagattcgttttccaggtgccgctcaccgtctgc
gaaccggtgatctgtttcgggttttgcagatcggactcatccacctgcgacaaacgccagaccttatgctccgggtcgaattt
tgccgaagcggcgtaacgcactgactgcaaacgacgctgatcgttgaaagcgtaaatactaatgccggccagctcctcgtcgc
ccttcacccgttcaatatagacaaagttattcccgtctttcgcccacagaccttgctgggtggaaagcagcgaaccgccatac
atcgcctgcgcacgatagttacgcgccatctgctcgccctgcggggcgacccattcgccgatcgccatggttaacagcaccag
ggggatagccgttttcatcaccgacagcgccacctgcatacgggtgaaacccgatgcctgcatcaccaccagttcgctacgct
gcgccagcatccccagtcccagcagcgcgccaagcagcgccgccataggaaagaagatctggatatccttaggtacgctgagc
agggtgtacatacccgcgcccagcgcgtcgtagttaccctgccccgcttttttcagctgatcgacaaacttgatgatcccgga
gagcgacaccagcatgaacaacgtcatcatgatggtggtaaaaatggttttaccgatatagcggtcaagtacaccaaatggct
gcat

## Claims

1. A modified bacterium, wherein the bacterium comprises a hypoxia regulated lipopolysaccharide (LPS) gene expression cassette comprising a gene involved in the biosynthesis or transport of LPS or a gene encoding a murein-lipoprotein under the control of a first strictly hypoxia inducible promoter, as compared to an unmodified starting strain.

2. The modified bacterium of claim 1, wherein the gene involved in the biosynthesis of LPS is selected from the group consisting of a gene involved in the synthesis of O-antigen, a gene involved in the synthesis of core region, and a gene involved in the synthesis of lipid A.

3. The modified bacterium of claim 2, wherein the gene involved in the synthesis of O-antigen is selected from the group consisting of genes rfbN, rfbV, wzzB, gtrBb, gtrBa, and rfbP/wbaP.

4. The modified bacterium of claim 2, wherein the gene involved in the synthesis of core region is selected from the group consisting of genes rfaB, rfaC/waaC, rfaF, rfaG, yibD, rfaI, rfaJ, rfaK, rfaL, rfaP, rfaQ, rfaY, rfaZ, yijP, gmhA, yaeD, rfaE, and rfaD.

5. The modified bacterium of claim 2, wherein the gene involved in the synthesis of lipid A is selected from the group consisting of genes lpxA, lpxB, lpxC, lpxD, lpxH, lpxK, htrB, msbB/lpxM, lpxO, lpxP, yeU, kdtA, arnT, pagP, yjdB, yhjw, kdsA, kdsB, yrbI, and yrbH.

6. The modified bacterium of claim 1, wherein the gene involved in the transport of LPS is selected from the group consisting of genes msbA, Wzy, wzxE, lptA, lptB, lptC/yrbK, lptD, and lptG.

7. The modified bacterium of any one of claims 1-6, wherein the gene involved in the biosynthesis or transport of LPS or the gene encoding murein-lipoprotein is a naturally occurring gene in the chromosome of the bacterium, wherein the native promoter of the gene involved in the biosynthesis or transport of LPS or the gene encoding murein-lipoprotein is functionally replaced by the first strictly hypoxia inducible promoter, whereby the expression of the gene involved in the biosynthesis or transport of LPS or the gene encoding murein-lipoprotein in the bacterium is completely under the control of the first strictly hypoxia inducible promoter.

8. The modified bacterium of any one of claims 1-6, wherein the LPS gene expression cassette is exogenous, and the gene involved in the biosynthesis or transport of LPS or the gene encoding murein-lipoprotein naturally occurring in the chromosome of the bacterium is deleted or functionally inactivated, whereby the expression of the gene involved in the biosynthesis or transport of LPS or the gene encoding murein-lipoprotein in the bacterium is completely under the control of the first strictly hypoxia inducible promoter.

9. The modified bacterium of claim 8, wherein the exogenous LPS gene expression cassette is integrated into the bacterial chromosome.

10. The modified bacterium of claim 8, wherein the exogenous LPS gene expression cassette is outside of the bacterial chromosome.

11. The modified bacterium of claim 10, wherein the exogenous LPS gene expression cassette is present in the plasmid carried by the bacterium.

12. The modified bacterium of any one of claims 1-11, wherein the bacterium further comprises a hypoxia regulated essential gene expression cassette comprising an essential gene of the bacterium under the control of a second strictly hypoxia inducible promoter, and the bacterium is deficient in at least one gene involved in or regulating the endogenous anti-oxidative stress response pathway or a functional expression product thereof.

13. The modified bacterium of any one of claims 1-12, wherein the first and second strictly hypoxia inducible promoters are each independently selected from the group consisting of pepTp, fnrSp, ysgAp, ssbp1, Hip1, BBa_I14018, BBa_R1074, Ptet-arcA, and Ptet-Fnr.

14. The modified bacterium of any one of claims 1-12, wherein the first and second strictly hypoxia inducible promoters are each independently selected from the group consisting of fnrSp and ssbp1 promoters.

15. The modified bacterium of any one of claims 12-14, wherein the expression product of the essential gene is responsible for the synthesis of 2,6-diaminopimelic acid (DAP) within the bacterium, and when cultured under aerobic conditions, the bacterial growth is dependent on the additional DAP or an analog thereof added to the medium.

16. The modified bacterium of claim 15, wherein the essential gene is selected from *dapA, dapB, dapD, dapE, argD, dapF,* and any combination thereof.

17. The modified bacterium of claim 15, wherein the essential gene is selected from *dapA* and *dapE.*

18. The modified bacterium of any one of the claims 12-17, wherein the gene involved in or regulating the endogenous anti-oxidative stress response pathway is an HtrA serine protease family gene.

19. The modified bacterium of any one of claims 12-18, wherein the functional expression product of the gene involved in or regulating the endogenous anti-oxidative stress response pathway is an HtrA serine protease family related protein.

20. The modified bacterium of any one of claims 12-19, wherein the bacterium is deficient in the activity of HtrA serine protease.

21. The modified bacterium of any one of claims 12-20, wherein the gene involved in or regulating the endogenous anti-oxidative stress response pathway is *htrA.*

22. The modified bacterium of any one of claims 12-21, wherein the bacterium is deficient in *htrA.*

23. The modified bacterium of any one of claims 12-22, wherein the essential gene is a gene naturally occurring in the bacterial chromosome, and the native promoter of the essential gene is functionally replaced by the second strictly hypoxia inducible promoter, whereby the expression of the essential gene in the bacterium is completely under the control of the second strictly hypoxia inducible promoter.

24. The modified bacterium of any one of claims 12-22, wherein the essential gene expression cassette is exogenous, and the essential gene naturally occurring in the bacterial chromosome is deleted or functionally inactivated, whereby the expression of the essential gene in the bacterium is completely under the control of the second strictly hypoxia inducible promoter.

25. The modified bacterium of claim 24, wherein the exogenous essential gene expression cassette is integrated into the bacterial chromosome.

26. The modified bacterium of claim 24, wherein the exogenous essential gene expression cassette is outside of the bacterial chromosome.

27. The modified bacterium of claim 26, wherein the exogenous essential gene expression cassette is present in the plasmid carried by the bacterium.

28. The modified bacterium of any one of the preceding claims, which further comprises a pH regulated expression cassette, wherein the pH regulated expression cassette comprises a gene encoding a bacteria-derived hemolysin protein under the control of a promoter that is active under acid pH condition.

29. The modified bacterium of claim 28, wherein the bacteria-derived hemolysin protein is a gram-negative bacterial hemolysin protein.

30. The modified bacterium of claim 29, wherein the gene encoding the gram-negative bacterial hemolysin protein is *hlyA* or *hlyE.*

31. The modified bacterium of claim 30, wherein the *hlyA* or *hlyE* is derived from *Listeria monocytogenes, Vibrio cholerae,* or *Escherichia coli.*

32. The modified bacterium of any one of claims 28-31, wherein the promoter that is active under acid pH condition is active at a pH less than 7.0, 6.9, 6.8, 6.7, 6.6, 6.5, 6.4, 6.3, 6.2, 6.1, 6.0, 5.9, 5.8, 5.7, 5.6, or 5.5.

33. The modified bacterium of any one of claims 28-32, wherein the promoter that is active under acid pH condition is selected from the group consisting of sseA, ssrA, ssaB, ssaG, ssaM, and ssaR.

34. The modified bacterium of any one of claims 28-32, wherein the promoter that is active under acid pH condition is sseA.

35. The modified bacterium of any one of the preceding claims, wherein the unmodified starting strain is a facultative anaerobic bacterium.

36. The modified bacterium of any one of the preceding claims, wherein the bacterium is a bacteirum of *Enterobacteriaceae.*

37. The modified bacterium of any one of the preceding claims, wherein the bacterium is a bacteirum of *Escherichia sp., Salmonella sp., Shigella sp., Klebsiella sp., Yersinia sp., Citrobacter sp., Enterobacter sp., Serratia sp., Proteus sp., Morganella sp., Providencia sp., Hafnia sp.,* and *Pantoea sp.*

38. The modified bacterium of any one of the preceding claims, wherein the bacterium is selected from the group consisting of *Escherichia coli, Enterobacter blattae (E. blattae), Enterobacter fergusonii (E. fergusonii), Enterobacter hermannii (E. hermannii), Enterobacter vulneris (E. vulneris), Salmonella enterica (S. enterica), Salmonella bongori (S. bongori), Salmonella typhi (S. typhi), Salmonella choleraesuis (S. choleraesuis), Salmonella typhimurium (S. typhimurium), Shigella dysenteriae (S. dysenteriae), Shigella flexneri (S. flexneri), Shigella boydii (S. boydii), Shigella sonnei (S. sonnei), Klebsiella pneumoniae (K. peumoniae), Klebsiella oxytoca (K. oxytoca), Yersinia pestis (Y. pestos), Yersinia enterocolitica (Y. enterocolitica), Yersinia pseudotuberculosis (Y. pseudotuberculosis), Yersinia aldouae (Y. aldouae), Yersinia bercovieri (Y. bercovieri), Yersinia frederiksenii (Y. frederiksenii), Yersinia intermedia (Y. intermedia), Yersinia kristersenii (Y. kristersenii), Yersinia mollaretti (Y. mollaretti), Yersinia rohdei (Y. rohdei), Yersinia ruckeri (Y. ruckeri), Citrobacter freundii (C. freundii), Citrobacter koseri (C. kaseri), Citrobacter braakii (C. braakii), Enterobacter aerogenes (E. aerogenes), Enterobacter cloacae (E. cloacae), Enterobacter gergoviae (E. gergoviac), Enterobacter sakazakii (E. sakazakii), Enterobacter taylorae (E. tavlorae), Enterobacter amnigenus (E. aminigenus), Enterobacter intermedius (E. intermedius), Enterobacter asburiae (E. asburiac), Enterobacter cancerogenus (E. cancerogenus), Enterobacter dissolvens (E. dissolvens), Enterobacter nimipressuralis (E. nimipressuralis), Serratia marcescens (S. marcescens), Serratia entomophila (S. entomophila), Serratia ficaria (S. ficaria), Serratia fonticola (S. fonticola), Serratia grimesii (S. grimesii), Serratia liquefaciens (S. liquefaciens), Serratia odorifera (S. odorifera), Serratia plymuthica (S. plymuthica), Serratia proteamaculans (S. proteamaculans), Serratia rubidaea (S. rubidaea), Serratia ureilytica (S. ureilytica), Proteus mirabilis (P. mirabilis), Proteus vulgaris (P. vulgaris), Proteus myxofaciens (P. myxofaciens), Proteus penneri (P. penneri), Proteus hauseri (P. hauseri)), Morganella morganii (M. morganii), Providencia alcalifaciens (P. alcalifaciens), Providencia rustigianii (P. rustigianii), Providencia stuartii (P. stuartii), Providencia rettgeri (P. rettgeri), Providencia heimbachae (P. heimbochae), Hafnia alvei (H. alvei),* and *Pantoea agglomerans (P. agglomerans).*

39. The modified bacterium of any one of the preceding claims, wherein the bacterium is *Salmonella typhimurium.*

40. The modified bacterium of claim 34, wherein the starting strain is *Salmonella typhimurium* SL7207.

41. The modified bacterium of any one of the preceding claims, wherein the bacterium expresses a wild-type lipopolysaccharide (LPS).

42. The modified bacterium of any one of the preceding claims, wherein, when administrated to a subject with a tumor, the bacterium is capable of surviving and proliferating in the tumor tissue but is rapidly cleared in normal tissue.
